# FASCICULE DE BREVET EUROPEEN

(11) **EP 2 443 152 B1**
(45) Date de publication et mention de la délivrance du brevet: **25.07.2018**
(21) Numéro de dépôt: 10723176.3
(22) Date de dépôt: 01.04.2010
(51) Int. Cl.: C07K 16/44, G01N 33/574, G01N 33/577

(54) **PROCÉDÉ DE DOSAGE DE LA PRODÉFENSINE-A6 POUR LE DIAGNOSTIC IN VITRO DU CANCER COLORECTAL**
TESTVERFAHREN FÜR PRODEFENSIN-A6 ZUR IN-VITRO-DIAGNOSE VON KOLOREKTALKARZINOM
METHOD FOR ASSAYING PRODEFENSIN-A6 FOR THE IN VITRO DIAGNOSIS OF COLORECTAL CANCER

(30) Priorité: 03.04.2009 FR 0952192
(43) Date de publication de la demande: 25.04.2012
(73) Titulaire: Biomérieux, 69280 Marcy L'etoile (FR)
(72) Inventeur: ATAMAN-ÖNAL, Yasemin, 01600 Reyrieux (FR); BEAULIEU, Corinne, 69140 Rillieux La Pape (FR); BUSSERET, Sandrine, 69001 Lyon (FR); CHARRIER, Jean-Philippe, 69160 Tassin La Demi-lune (FR); CHOQUET-KASTYLEVSKY, Geneviève, 69340 Francheville (FR); ROLLAND, Dominique, 69290 Saint Genis Les Ollieres (FR)
(74) Mandataire: Bitaud, Valérie Marie-Odile
(86) Numéro de dépôt international: PCT/FR2010/050620
(87) Numéro de publication internationale: WO 2010/112777

(56) Documents cités:
- NAM MYEONG J ET AL: "Identification of defensin alpha 6 as a potential biomarker in colon adenocarcinoma" JOURNAL OF BIOLOGICAL CHEMISTRY, vol. 280, no. 9, mars 2005 (2005-03), pages 8260-8265, XP002554542 ISSN: 0021-9258
- JONES D E ET AL: "Defensin-6 mRNA in human Paneth cells: implications for antimicrobia peptides in host defense of the human bowel" FEBS LETTERS, ELSEVIER, AMSTERDAM, NL, vol. 315, no. 2, 4 janvier 1993 (1993-01-04), pages 187-192, XP025576660 ISSN: 0014-5793 [extrait le 1993-01-04]
- SZYK AGNIESZKA ET AL: "Crystal structures of human alpha-defensins HNP4, HD5, and HD6" PROTEIN SCIENCE, vol. 15, no. 12, décembre 2006 (2006-12), pages 2749-2760, XP002554543 ISSN: 0961-8368
- ABARZUA P ET AL: "MICROINJECTION OF MONOCLONAL ANTIBODY PAB421 INTO HUMAN SW480 COLORECTAL CARCINOMA CELLS RESTORES THE TRANSCRIPTION ACTIVATION FUNCTION TO MUTANT P53" CANCER RESEARCH, AMERICAN ASSOCIATION FOR CANCER RESEARCH, BALTIMORE, MD., US, vol. 55, 15 août 1995 (1995-08-15), pages 3490-3494, XP002035503 ISSN: 0008-5472
- KIM JIN-AH ET AL: "Troglitazone activates p21Cip/WAF1 through the ERK pathway in HCT15 human colorectal cancer cells" CANCER LETTERS, vol. 179, no. 2, 28 mai 2002 (2002-05-28), pages 185-195, XP002554544 ISSN: 0304-3835
- ROMANI ROSSANA ET AL: "Does dependent in vivo inhibition of human colorectal cancer (LoVo) by the gastrin receptor antagonist, CI-988" CLINICAL AND EXPERIMENTAL PHARMACOLOGY AND PHYSIOLOGY, vol. 23, no. 5, 1996, pages 438-440, XP002554545 ISSN: 0305-1870
- GOI TAKANORI ET AL: "Angiogenesis and tumor proliferation/metastasis of human colorectal cancer cell line SW620 transfected with endocrine glands-derived-vascular endothelial growth factor, as a new angiogenic factor." CANCER RESEARCH, vol. 64, no. 6, 15 mars 2004 (2004-03-15), pages 1906-1910, XP002554546 ISSN: 0008-5472

### SEQUENCE LISTING

<110> bioMérieux
<120> Procédé de dosage de la ProDéfensine-A6 pour le diagnostic in
   vitro du cancer colorectal
<130> PDEFA6
<150> FR09 52192
   <151> 2009-04-03
<160> 31
<170> Patent In version 3.3
<210> 1
   <211> 81
   <212> PRT
   <213> human
<400> 1
   Glu Pro Leu Gln Ala Glu Asp Asp Pro Leu Gln Ala Lys Ala Tyr Glu
   1               5                   10                  15
   Ala Asp Ala Gln Glu Gln Arg Gly Ala Asn Asp Gln Asp Phe Ala Val
               20                  25                  30
   Ser Phe Ala Glu Asp Ala Ser Ser Ser Leu Arg Ala Leu Gly Ser Thr
           35                  40                  45
   Arg Ala Phe Thr Cys His Cys Arg Arg Ser Cys Tyr Ser Thr Glu Tyr
       50                  55                  60
   Ser Tyr Gly Thr Cys Thr Val Met Gly Ile Asn His Arg Phe Cys Cys
   65                  70                  75                  80
   Leu
<210> 2
   <211> 46
   <212> PRT
   <213> human
<400> 2
   Glu Pro Leu Gln Ala Glu Asp Asp Pro Leu Gln Ala Lys Ala Tyr Glu
   1               5                   10                  15
   Ala Asp Ala Gln Glu Gln Arg Gly Ala Asn Asp Gln Asp Phe Ala Val
               20                  25                  30
   Ser Phe Ala Glu Asp Ala Ser Ser Ser Leu Arg Ala Leu Gly
           35                  40                  45
<210> 3
   <211> 43
   <212> PRT
   <213> human
<400> 3
   Ala Glu Asp Asp Pro Leu Gln Ala Lys Ala Tyr Glu Ala Asp Ala Gln
   1 5 10 15
   Glu Gln Arg Gly Ala Asn Asp Gln Asp Phe Ala Val Ser Phe Ala Glu
   20 25 30
   Asp Ala Ser Ser Ser Leu Arg Ala Leu Gly Ser
   35 40
<210> 4
   <211> 2
   <212> PRT
   <213> human
<400> 4
   Asp Pro
   1
<210> 5
   <211> 3
   <212> PRT
   <213> human
<400> 5

   Asp Pro Leu
   1
<210> 6
   <211> 6
   <212> PRT
   <213> human
<400> 6
   Glu Asp Asp Pro Leu Gln
   1 5
<210> 7
   <211> 9
   <212> PRT
   <213> artificial sequence
<220>
   <223> epitope
<220>
   <221> MSC_FEATURE
   <222> (1)..(1)
   <223> V ou L
<220>
   <221> MSC_FEATURE
   <222> (2)..(2)
   <223> T ou L
<220>
   <221> MSC_FEATURE
   <222> (3)..(3)
   <223> P, S ou C
<220>
   <221> MSC_FEATURE
   <222> (4)..(4)
   <223> P ou S
<220>
   <221> MSC_FEATURE
   <222> (5)..(5)
   <223> W ou T
<220>
   <221> MSC_FEATURE
   <222> (6)..(6)
   <223> A, Q, M, C ou E
<220>
   <221> MSC_FEATURE
   <222> (7)..(7)
   <223> I, E ou D
<220>
   <221> MSC_FEATURE
   <222> (8)..(8)
   <223> F, Y, S ou L
<400> 7
   Xaa Xaa Xaa Xaa Xaa Xaa Xaa Xaa Arg
   1               5
<210> 8
   <211> 12
   <212> PRT
   <213> artificial sequence
<220>
   <223> epitope
<400> 8
   Asn Tyr Val Thr Pro Pro Trp Ala Ile Phe Arg His
   1               5                   10
<210> 9
   <211> 12
   <212> PRT
   <213> artificial sequence
<220>
   <223> epitope
<400> 9
   Trp Thr Gly Val Leu Ser Pro Thr Gln Glu Tyr Arg
   1 5 10
<210> 10
   <211> 12
   <212> PRT
   <213> artificial sequence
<220>
   <223> epitope
<400> 10
   Ser His Leu Thr Pro Pro Trp Met Asp Tyr Arg Val
   1 5 10
<210> 11
   <211> 12
   <212> PRT
   <213> artificial sequence
<220>
   <223> epitope
<400> 11
   Val Met Ala Val Thr Cys Ser Thr Cys Asp Ser Arg
   1 5 10
<210> 12
   <211> 12
   <212> PRT
   <213> artificial sequence
<220>
   <223> epitope
<400> 12
   Leu Thr Pro Pro Thr Glu Asp Leu Arg Pro Pro Asp
   1 5 10
<210> 13
   <211> 8
   <212> PRT
   <213> artificial sequence
<220>
   <223> epitope
<220>
   <221> MSC_FEATURE
   <222> (1)..( 1)
   <223> S ou T
<220>
   <221> MSC_FEATURE
   <222> (2)..(2)
   <223> C ou absent
<220>
   <221> MSC_FEATURE
   <222> (3)..(3)
   <223> T, L ou E
<220>
   <221> MSC_FEATURE
   <222> (4)..(4)
   <223> H ou R
<220>
   <221> MSC_FEATURE
   <222> (5)..(5)
   <223> I, F ou E
<220>
   <221> MSC_FEATURE
   <222> (6)..(6)
   <223> V ou G
<220>
   <221> MSC_FEATURE
   <222> (8)..(8)
   <223> C ou N
<400> 13
   Xaa Xaa Xaa Xaa Xaa Xaa His Xaa
   1               5
<210> 14
   <211> 12
   <212> PRT
   <213> artificial sequence
<220>
   <223> epitope
<400> 14
   Tyr Gly Asn His Ser Cys Thr His Ile Gly His Cys
   1               5                   10
<210> 15
   <211> 12
   <212> PRT
   <213> artificial sequence
<220>
   <223> epitope
<400> 15
   Gly Pro Ser Tyr Thr Cys Leu His Phe Gly His Cys
   1               5                   10
<210> 16
   <211> 12
   <212> PRT
   <213> artificial sequence
<220>
   <223> epitope
<400> 16
   Thr Glu Arg Glu Val His Asn Trp Phe Pro Phe His
   1               5                   10
<210> 17
   <211> 9
   <212> PRT
   <213> artificial sequence
<220>
   <223> epitope
<220>
   <221> MSC_FEATURE
   <222> (1)..(1)
   <223> P ou W
<220>
   <221> MSC_FEATURE
   <222> (4)..(4)
   <223> W ou E
<220>
   <221> MSC_FEATURE
   <222> (5)..(5)
   <223> S, A, Q ou W
<220>
   <221> MSC_FEATURE
   <222> (6)..(6)
   <223> M, L, R ou P
<220>
   <221> MSC_FEATURE
   <222> (7)..(7)
   <223> H, F, W ou G
<220>
   <221> MSC_FEATURE
   <222> (8)..(8)
   <223> V ou A
<220>
   <221> MSC_FEATURE
   <222> (9)..(9)
   <223> V ou I
<400> 17
   Xaa His Pro Xaa Xaa Xaa Xaa Xaa Xaa
   1 5
<210> 18
   <211> 12
   <212> PRT
   <213> artificial sequence
<220>
   <223> epitope
<400> 18
   Tyr Pro His Pro Trp Ser Met His Val Ile Arg Ala
   1 5 10
<210> 19
   <211> 12
   <212> PRT
   <213> artificial sequence
<220>
   <223> epitope
<400> 19
   Thr Thr Thr Pro His Pro Trp Ala Leu Phe Ala Val
   1 5 10
<210> 20
   <211> 12
   <212> PRT
   <213> artificial sequence
<220>
   <223> epitope
<400> 20
   Thr Pro His Pro Trp Gln Arg Trp Val Val Tyr Ser
   1 5 10
<210> 21
   <211> 12
   <212> PRT
   <213> artificial sequence
<220>
   <223> epitope
<400> 21
   Glu Asp Val Leu Arg Trp His Pro Glu Trp Pro Gly
   1 5 10
<210> 22
   <211> 6
   <212> PRT
   <213> artificial sequence
<220>
   <223> epitope
<220>
   <221> MSC_FEATURE
   <222> (1)..(1)
   <223> Y ou N
<220>
   <221> MSC_FEATURE
   <222> (3)..(3)
   <223> E, D ou Q
<220>
   <221> MSC_FEATURE
   <222> (4)..(4)
   <223> T, N, R, M ou K
<220>
   <221> MSC_FEATURE
   <222> (5)..(5)
   <223> W, H ou F
<220>
   <221> MSC_FEATURE
   <222> (6)..(6)
   <223> P ou G
<400> 22
   Xaa His Xaa Xaa Xaa Xaa
   1 5
<210> 23
   <211> 12
   <212> PRT
   <213> artificial sequence
<220>
   <223> epitope
<400> 23
   Tyr His Glu Thr Trp Pro Pro Lys Ser Ala Gln Leu
   1 5 10
<210> 24
   <211> 12
   <212> PRT
   <213> artificial sequence
<220>
   <223> epitope
<400> 24
   Tyr His Asp Asn Trp Pro Gln Pro Ser Arg Ser Trp
   1 5 10
<210> 25
   <211> 12
   <212> PRT
   <213> artificial sequence
<220>
   <223> epitope
<400> 25
   Gln His Asn His Gln Arg His Gly Ala Met Gly Ala
   1 5 10
<210> 26
   <211> 12
   <212> PRT
   <213> artificial sequence
<220>
   <223> epitope
<400> 26
   Tyr His Asp Met Trp Pro Met Ser Gly Arg Met Ala
   1 5 10
<210> 27
   <211> 12
   <212> PRT
   <213> artificial sequence
<220>
   <223> epitope
<400> 27
   Tyr His Asp Asn Trp Pro Pro Leu Asn Gly Ala Arg
   1 5 10
<210> 28
   <211> 12
   <212> PRT
   <213> artificial sequence
<220>
   <223> epitope
<400> 28
   Tyr His Asp Met Trp Pro Ala Ile Gln Leu Ser Pro
   1 5 10
<210> 29
   <211> 12
   <212> PRT
   <213> artificial sequence
<220>
   <223> epitope
<400> 29
   Tyr His Glu Lys Phe Pro Gly Pro Val Val Leu Pro
   1 5 10
<210> 30
   <211> 13
   <212> PRT
   <213> human
<400> 30
   Glu Pro Leu Gln Ala Glu Asp Asp Pro Leu Gln Ala Lys
   1 5 10
<210> 31
   <211> 10
   <212> PRT
   <213> human
<400> 31
   Ala Tyr Glu Ala Asp Ala Gln Glu Gln Arg
   1 5 10

## Description

La présente invention concerne le domaine de la cancérologie. Plus particulièrement, la présente invention a pour objet un procédé de diagnostic *in vitro* du cancer colorectal chez un patient humain par détermination de la présence de la ProDéfensine-A6 dans un échantillon biologique issu de ce patient, ledit procédé pouvant être utilisé tant dans le diagnostic précoce, le dépistage, le suivi thérapeutique, le pronostic, que dans le diagnostic des rechutes dans le cadre du cancer colorectal.

Le cancer colorectal (CCR) est un problème majeur de santé publique. Son incidence mondiale a été estimée à 875000 nouveaux cas en 1996¹. Tous sexes confondus, c'est le cancer qui survient le plus fréquemment dans les pays occidentaux où il est généralement classé parmi les 3 premières causes de décès par cancer. Le taux de survie à 5 ans tout stade confondu est voisin de 60%.

Seul un diagnostic précoce offre l'espoir d'un traitement curatif. Or, à l'heure actuelle, il n'existe aucun test sérologique de dépistage, ni de diagnostic spécifique qui soit précoce.

Le dépistage du cancer colorectal est réalisé actuellement en Europe avec deux approches distinctes : premièrement à l'aide d'un test paraclinique qui consiste à rechercher la présence de sang dans les selles (Faecal Occult Blood test, FOBT, commercialisé par exemple sous le nom d'Hémoccult®). Cette technique a démontré son utilité clinique. Lorsqu'elle est utilisée tous les 2 ans chez les personnes âgées de 50 à 74 ans, elle peut réduire de 15 à 20% la mortalité par cancer colorectal². Pour cela, il faut que plus de la moitié de la population concernée participe régulièrement au dépistage et qu'une colonoscopie soit faite en cas de test positif, suivie éventuellement d'un traitement adapté.

Néanmoins, cette technique de dépistage souffre d'un certain nombre de handicaps :
- L'inconvénient majeur de ce test est sa sensibilité médiocre, tout spécialement pour les adénomes (lésion dysplasique pré-cancéreuse) qui, s'ils sont de grande taille ou en dysplasie sévère, conduiront dans 1 cas sur 10 au développement d'un cancer.
- Le test est également peu spécifique. L'apparition de sang dans les selles peut être liée à une affection non tumorale : hémorragies recto-coliques, hémorroïdes, fistules, ... Dans ce cas, une investigation par colonoscopie doit être réalisée avec les inconvénients décrits ci-après.
- Enfin les Hémoccult® sont délicats à interpréter, ils doivent donc être lus dans des centres spécialisés, par un personnel qualifié et compétent.

Des tests immunologiques spécifiques de l'hémoglobine humaine (Feca EIA®, Heme Select®, ...) ont également été décrits. Ils constituent probablement un progrès par rapport à l'Hémoccult® mais ils présentent par essence les mêmes problèmes. C'est ainsi que InSure™, commercialisé par Enterix Inc., permet de détecter 87% des patients atteints de CCR et 47% de ceux ayant des polypes précancéreux. Il s'agit d'un test de détection de l'hémoglobine humaine dans les selles, et plus particulièrement de la portion de globine de cette molécule.

Une deuxième stratégie de dépistage est la réalisation systémique d'une colonoscopie après 50 ans, qui permet en théorie de réduire la mortalité par cancer colorectal. Mais l'acceptabilité de cet examen chez des sujets en bonne santé est trop faible pour qu'une politique de dépistage utilisant l'endoscopie diminue la mortalité (il y a une compliance aux alentours de 2% pour la colonoscopie dans les pays d'Europe ayant mis en place cette stratégie de dépistage). Il existe un risque non négligeable (1 ‰ de perforation et hémorragie du côlon et de décès (1/10 000), ainsi qu'un coût élevé pour la santé publique. De plus, la colonoscopie nécessite une préparation colique préalable très contraignante, qui explique en grande partie la mauvaise compliance.

Des marqueurs tumoraux dosables par immunoessais ont été décrits de longue date dans le cadre du cancer colorectal. Il s'agit notamment de l'antigène carcinoembryonnaire (ACE) et du CA19-9.

L'ACE est utilisé pour le suivi. Il ne peut pas être utilisé pour le dépistage ni pour le diagnostic précoce du cancer colorectal car sa sensibilité et sa spécificité sont insuffisantes. En effet, ce marqueur est exprimé par d'autres types de cancers, et dans des pathologies bénignes. Malgré tout, il est possible de gagner en sensibilité sans perdre en spécificité en associant à l'ACE un autre marqueur tumoral tel que le CA19-9 ou le CA72-4.

Les causes de variations physiologiques du CA19-9 sont rares mais d'autres affections bénignes (hépatobiliaires, pancréatiques), ou malignes peuvent induire une élévation du CA19-9. Ce marqueur pris isolément ne présente donc pas non plus d'intérêt pour le diagnostic. Néanmoins, sa concentration sérique étant corrélée à la taille de la tumeur et à la présence de métastases, il peut permettre également un suivi thérapeutique ou la mise en évidence précoce de récidives.

Des tests commerciaux ont par ailleurs été proposés tels que :
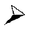 Colopath®/ColorectAlert^{MD}, commercialisé par Ambrilia, est un test de dépistage rapide et peu invasif pour le CCR. Colopath® détecte un plasmalogène (classe de lipides complexes faisant partie des phospholipides) dans le mucus rectal des individus avec une pathologie colorectale, tandis que le ColorectAlert^{MD} détecte l'antigène-T, un sucre complexe dans le mucus rectal. Le test Colopath®/ColorectAlert^{MD} implique l'application de mucus rectal sur une bande de test et le résultat positif ou négatif est basé sur une réaction de Schiff. Ambrilia a étudié 1 787 sujets et démontré que le Colopath®/ColorectAlert^{MD} détecte 54% des cas de cancer colorectal de stade précoce et 49% de tous stades confondus.
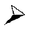 *COLARIS,* commercialisé par Myriad Genetics, est un test de détection dans le sang de mutations dans les gènes MLH1 et MSH2 pour le dépistage des cancers héréditaires du côlon non polyposiques (syndrome HNPCC). Le résultat du test est disponible en 3 semaines. Myriad utilise les techniques de séquençage les plus sensibles et les plus spécifiques existantes à l'heure actuelle. Le coût du test est élevé.
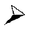 *DR-70*®*,* commercialisé par AMDL, est un test de dépistage de différents types de cancers (poumon, côlon, sein, foie, estomac, ...). Il n'est donc pas spécifique du CCR. Son principe est basé sur la technique ELISA double sandwich (dosage de l'antigène DR-70). La révélation se fait par réaction enzymatique (anticorps couplés à la biotine et à la streptavidine). Une réaction colorée indique la présence de cancer.

La Demanderesse a maintenant mis en évidence de façon surprenante un nouveau marqueur d'adénocarcinome, lequel est relargué par les tumeurs coliques malignes hors des tissus cancéreux et est caractéristique de ces tumeurs, de sorte qu'il peut être détecté tant dans les échantillons biologiques distants des tumeurs malignes, que dans les tumeurs elles-mêmes.

Ainsi, la présence invention a pour premier objet un procédé de diagnostic *in vitro* du cancer colorectal par détermination de l'augmentation de la concentration, par rapport aux valeurs de référence déterminées pour les sujets sains, du marqueur protéique ProDéfensine-A6 dans des échantillons biologiques issus de patients suspectés d'être atteints du cancer colorectal, et de préférence distants des tumeurs, mettant en oeuvre un anticorps monoclonal spécifique de la ProDéfensine-A6 reconnaissant l'épitope 1 de SEQ ID N°6.

Elle concerne également l'utilisation de ce procédé tant dans le diagnostic précoce, le dépistage, le suivi thérapeutique, le pronostic, que dans le diagnostic des rechutes dans le cadre du cancer colorectal.

Le procédé de l'invention permet donc de diagnostiquer de façon spécifique et précoce le cancer colorectal par un test simple consistant à rechercher la présence de la ProDéfensine-A6 dans un échantillon biologique prélevé chez un patient, de préférence distant de la tumeur potentielle. En effet, la Demanderesse a montré de façon inattendue que les tumeurs coliques non seulement sécrétaient spécifiquement de la Prodéfensine-A6, mais surtout le relarguait hors du tissu cancéreux, comme cela sera mis en évidence de façon plus détaillée ci-après, et que sa concentration dans l'échantillon biologique dans lequel on met en oeuvre le procédé de l'invention était augmentée par rapport aux valeurs de référence déterminées pour les patients sains.

La détermination de la présence de la ProDéfensine-A6 dans un échantillon biologique distant ou non de la tumeur permet alors de conclure à la pathologie recherchée. Un des avantages du procédé de l'invention réside donc en la possibilité d'utiliser un échantillon distant de la tumeur potentielle à titre d'échantillon de diagnostic, ce qui permet un diagnostic simple et non invasif alors qu'un diagnostic tissulaire nécessite une biopsie prélevée de façon invasive. En effet, l'étude de marqueurs tissulaires, par exemple sur coupe de tissu (immunohistochimie), peut présenter un intérêt pronostique mais n'a aucun intérêt pour le dépistage ou le diagnostic du cancer colorectal.

Les Défensines sont une famille de peptides antimicrobiens impliqués dans la défense de l'hôte contre les attaques microbiennes. Elles sont constituées de 30 à 40 acides aminés et ont la propriété de désagréger sélectivement les membranes. Comme d'autres protéines eucaryotes, les Défensines peuvent être présentes sous forme de protéine mature ou sous forme de précurseur.

Un précurseur, également appelé protéine précurseur, est constitué d'un propeptide et d'une partie mature. Ainsi, la ProDéfensine A6 est la protéine précurseur de la protéine mature Défensine A6, est constituée de 100 acides aminés et comprend un peptide signal (acides aminés 1-19), le propeptide (acides aminés 20-65) et la protéine mature Défensine A6 (acides aminés 66-100).

De façon générale, les protéines précurseurs ont longtemps été considérées comme étant uniquement des molécules métaboliques. Toutefois, un certain nombre d'exemples récents, notamment dans le domaine des neuropeptides, indiquent que dans certaines situations, les protéines précurseurs ont une activité biologique propre et dissociée de celle du peptide mature qu'ils peuvent générer. Certes, la séquence des protéines précurseurs inclut celle des protéines matures, par exemple la séquence des ProDéfensines inclut celle des Défensines, mais leurs points isoélectriques et poids moléculaires sont différents. Les Défensines et ProDéfensines sont donc à considérer comme deux protéines différentes.

Les Défensines alpha 5³ et alpha 6⁴ sont essentiellement produites par les cellules de Paneth de l'intestin grêle. Les ARNm des Défensines alpha 5 et 6 sont surexprimés dans le tissu colique en cas de maladie de Crohn⁵. La Défensine alpha 6 a été identifiée comme marqueur potentiel du cancer du côlon par Nam et al.⁶. Nam et al. ont développé un dosage ELISA par compétition dosant spécifiquement la Défensine alpha 6. Ils ont défini un seuil (30 ng/ml) au-delà duquel les patients étaient diagnostiqués comme ayant un cancer colorectal. Lors d'une analyse de 18 sérums de donneurs sains et de 49 sérums de cancer, ils ont obtenu une sensibilité diagnostique de 69,4% pour une spécificité diagnostique de 83,3%. Aucune mention n'a été faite dans ce document sur le précurseur de la Défensine alpha 6, la ProDéfensine alpha 6.

Ainsi, le précurseur de la Défensine alpha 6, la ProDéfensine-A6 (N° Swiss Prot Q01524), n'a jamais été décrit comme pouvant être utile comme marqueur dans le cadre du cancer et notamment du cancer colorectal et comme pouvant être dosé dans un échantillon biologique distant ou non de la tumeur maligne.

Par détermination de la présence de la protéine précurseur, on entend la détermination du précurseur au-delà des valeurs de référence déterminées pour les patients sains. Le précurseur recherché peut-être le précurseur intact de 100 acides aminés, la protéine précurseur sans le peptide signal (acides aminés 20 à 100), ou le propeptide seul (acides aminés 20 à 65). Il peut être également des fragments de ces derniers, tels que des fragments du propeptide, à l'exclusion de la protéine mature (acides aminés 66-100) et des fragments de celle-ci.

Selon un mode de réalisation particulier de l'invention, on détermine la présence du précurseur de la Prodéfensine-A6, sans le peptide signal. La séquence décrite pour ce précurseur dans la base de donnée Swiss-Prot est la SEQ ID N°1 (EPLQAEDDPLQAKAYEADAQEQRGANDQDFAVSFAEDASSSLRALGSTR AFTCHCRRSCYSTEYSYGTCTVMGINHRFCCL ; correspondant aux acides aminés 20-100). De préférence, on détermine la présence du propeptide lui-même ayant au moins la séquence SEQ ID N°2 (EPLQAEDDPLQAKAYEADAQEQRG ANDQDFAVSFAEDASSSLRALG) et au plus la séquence SEQ ID N°1.

Comme il est bien connu de l'homme du métier, il existe des polymorphismes protéiques, et les séquences données ci-dessus ne sont qu'indicatives. Ce sont les séquences consensus indiquées dans la base de données Swiss-Prot, mais il peut exister des substitutions d'acides aminés dans un pourcentage qui sera évalué par l'homme du métier pour considérer qu'il s'agit de la même protéine. De même, le site de clivage du propeptide à l'acide aminé 65 est théorique et n'est donné qu'à titre indicatif.

Par relarguage par les tumeurs coliques, on entend la sécrétion active ou passive ou la libération quel qu'en soit le mécanisme du marqueur tumoral par les cellules tumorales elles-mêmes ou par les cellules non tumorales voisines suite à des lésions ou des modifications de phénotype cellulaire résultant du développement tumoral.

Par échantillon biologique dans lequel on met en oeuvre le procédé de l'invention, on entend tout échantillon biologique susceptible de contenir le marqueur tumoral d'intérêt. A titre d'exemple d'échantillon biologique non distant de la tumeur, on peut citer les échantillons solides tels que le tissu provenant de la tumeur, de biopsies de cette tumeur, de ganglions lymphatiques, des métastases du patient, et les cellules purifiées à partir de ces échantillons solides. A titre d'exemple d'échantillon biologique distant de la tumeur, on peut citer les fluides biologiques tels que le sang total ou ses dérivés, par exemple sérum ou plasma, les urines, la salive et les épanchements, la moelle osseuse et les selles, et les cellules purifiées à partir de ces échantillons liquides. On préfère le sang ou ses dérivés ainsi que les selles, les épanchements et les cellules purifiées à partir de ces échantillons liquides.

Le procédé de l'invention peut être amélioré en détectant, outre la Prodéfensine-A6, au moins un autre marqueur tumoral, le cas échéant également relargué par les tumeurs coliques hors des tissus cancéreux. Ainsi, la combinaison d'au moins deux marqueurs permet d'améliorer la spécificité et la sensibilité du test de diagnostic du cancer colorectal.

Ainsi, un autre objet de l'invention consiste également à déterminer la présence d'au moins un autre marqueur tumoral choisi dans le groupe de marqueurs suivants : Leucocyte Elastase Inhibitor, Ezrine, Aminoacylase 1, Liver Fatty Acid-Binding Protein, Intestinal Fatty Acid-Binding Protein, Apolipoprotéine AI, Apolipoprotéine AII, Plastine-I, Beta 2 Microglobuline, Protéasome 20S, Galectine-3, L-Lactate Deshydrogénase Chaîne B, Calréticuline, Regenerating Islet-Derived Protein 3 Alpha, Tumor-Associated Calcium Signal Transducer 1, Kératine type II Cytoskeletal 8, Kératine type I Cytoskeletal 18, Kératine type I Cytoskeletal 19, Epithelial-Cadhérine, ACE, Villine, CA19-9, CA 242, CA 50, CA 72-2, Testostérone, TIMP-1, Cripto-1, la Protéine Disulfide Isomérase, Intélectine-1, Cytokératine 20, Translationally-Controlled Tumor Protein, (Pro)défensine-A5, MIF, Pyruvate kinase M2-PK, Calgranuline C, CD24, CCSA-3 (colon cancer specific antigen) et CCSA-4, la détection de fragments d'ADN dans le sang ayant des altérations spécifiques de leur profil de méthylation, comme par exemple l'ADN méthylé du gène AXL4 (Aristaless-like Homeobox-4 Gene Methylation) ou l'ADN méthylé du gène Septin-9, la détection d'altérations spécifiques de fragments d'ADN dans les selles comme des mutations spécifiques de l'ADN dans les selles ou des altérations spécifiques du profil de méthylation de l'ADN dans les selles, la détection d'hémoglobine humaine dans les selles.

Par marqueur tumoral autre que la ProDéfensine-A6, on entend la protéine, l'ARN messager ou des modifications spécifiques du gène correspondant, comme des mutations ou des méthylations. En d'autres termes, seule la Prodéfensine-A6 est uniquement recherchée sous forme protéique, complète ou sous forme de fragment.

Le marqueur tumoral Leucocyte Elastase Inhibitor (N° Swiss Prot P30740, également appelé LEI, Serpin B1, Monocyte/neutrophil elastase inhibitor, M/NEI ou EI) a été séquencé en 1992⁷. Le LEI inhibe spécifiquement les protéases ayant des propriétés de type Elastase ou Chymotrypsine par formation de complexe non dissociable sous l'action du SDS⁸. C'est ainsi que le LEI inhibe trois des protéases majeures produites par les neutrophiles : la Leucocyte Elastase, la proteinase-3 et la Cathepsine G. Ces protéases permettent au système immunitaire de défendre l'organisme par protéolyse de substrats extracellulaires ou phagocytés. Mais lorsque ces protéases sont en excès, elles sont responsables de réactions inflammatoires. Le LEI pourrait donc avoir un rôle de régulation et de limitation de l'action inflammatoire induite par les protéases cellulaires. La Demanderesse a montré quant à elle de façon surprenante, dans la demande de brevet WO2009/024691, que la concentration de cette protéine était augmentée par rapport aux valeurs de référence déterminées pour les patients sains, de sorte que cette protéine est un bon marqueur dans les échantillons biologiques issus d'un patient atteint d'un cancer colorectal, lesdits échantillons étant distants ou non de la tumeur.

Le marqueur Ezrine (N° Swiss Prot P15311, également appelé p81, Cytovillin ou Villin-2) est une protéine assurant la liaison entre la membrane cellulaire et les filaments d'Actine du cytosquelette de la cellule, notamment dans les microvillosités des cellules épithéliales intestinales⁹. W.G. Jiang et S. Hiscox¹⁰ ont montré que les Interleukines IL-2, IL-8, IL-10, ... pouvaient inhiber l'expression d'Ezrine dans la lignée cellulaire de cancer colorectal humaine, HT29. Les mêmes auteurs¹¹ ont montré que l'inhibition de l'expression d'Ezrine dans les lignées cellulaires de cancer colorectal, HT115 et HRT18, réduisait l'adhésion entre cellules et augmentait la mobilité et le comportement invasif des cellules. Ils ont conclu que l'Ezrine régulait les adhésions cellule/cellule et cellule/matrice, en interagissant avec les molécules d'adhésion cellulaire, E-Cadhérine et beta-Caténine. Ils ont suggéré que l'Ezrine pouvait jouer un rôle important dans le contrôle du potentiel invasif des cellules cancéreuses. Par ailleurs, T. Xiao et al.¹² ont utilisé un dosage ELISA pour quantifier l'Ezrine plasmatique de patients atteints d'un cancer du poumon. Toutefois, ils n'ont pas observé de différences par rapport à des sujets contrôles. La Demanderesse a montré quant à elle de façon surprenante, dans la demande de brevet WO2009/019365, que la concentration de cette protéine était augmentée par rapport aux valeurs de référence déterminées pour les patients sains, de sorte que cette protéine est un bon marqueur dans les échantillons biologiques issus d'un patient atteint d'un cancer colorectal, lesdits échantillons étant distants ou non de la tumeur.

Le marqueur Aminoacylase 1 (N° Swiss Prot Q03154, également appelé EC 3.5.1.14, N-Acyl-L-Amino-Acid Amidohydrolase ou ACY-1) fait partie de la famille des Aminoacylases. Ce sont des enzymes qui catalysent l'hydrolyse des acides aminés acylés pour donner des acides gras et des acides aminés¹³. Un dosage immunochimique de l'activité enzymatique Aminoacylase a été développé dès 1975 par K. Lorentz et al.¹⁴ et a été utilisé pour doser différents tissus et sérums¹⁵. L'étude a montré une augmentation de l'activité Aminoacylase en cas de pathologies hépatiques mais non en cas de cancer du côlon. Par ailleurs, le gène de l'Aminoacylase 1 a été identifié sur le chromosome 3p21.1¹⁶. La région 3p21.1 est réduite à l'homozygotie lors d'un cancer du poumon à petite cellule, et dans ce cas, l'expression de l'Aminoacylase est réprimée ou indétectable¹⁷. De même S. Balabanov et al.¹⁸ ont montré que l'expression de l'Aminoacylase était réprimée en cas de cancer du rein. La Demanderesse a montré quant à elle de façon surprenante, dans la demande de brevet WO2009/019366, que la concentration de cette protéine était augmentée par rapport aux valeurs de référence déterminées pour les patients sains, de sorte que cette protéine est un bon marqueur dans les échantillons biologiques issus d'un patient atteint d'un cancer colorectal, lesdits échantillons étant distants ou non de la tumeur.

Le marqueur Liver Fatty Acid-Binding Protein (N° Swiss Prot P07148, également appelé L-FABP, FABP1, FABPL, protéine Z ou protéine transporteur de stérol) appartient à la famille des FABP qui comprend neuf isoformes. Chaque isoforme est dénommée d'après le tissu dans lequel elle a été détectée la première fois. Ces isoformes possèdent une communauté de fonction, des structures tridimensionnelles ressemblantes mais leur homologie de séquence n'est pas élevée. La L-FABP a été séquencée en 1985¹⁹. C'est une petite protéine de 15 kDa, abondante dans le cytosol, possédant la capacité de se fixer aux acides gras libres ainsi qu'à la bilirubine. Quelques études récentes semblent indiquer que les altérations de l'expression de la protéine L-FABP pourraient induire un processus de tumorigenèse. Pour le cancer de la prostate, le niveau d'expression des ARNm du L-FABP dans les biopsies de tissu tumoral était 10 fois plus élevé que dans le tissu normal²⁰. Pour le cancer du côlon, plusieurs équipes ont identifié une diminution de l'expression de la protéine L-FABP au niveau du tissu tumoral comparée à la muqueuse colique normale, en utilisant des techniques d'électrophorèse en 2 dimensions²¹. Ce résultat a aussi été confirmé par des techniques d'immunohistochimie. De plus, la protéine L-FABP est un marqueur pronostic de résection hépatique chez les patients atteints de cancer colorectal ayant métastasé dans le foie²². Dans la demande de brevet WO00/33083, il a été suggéré que ce marqueur pouvait être détecté dans des fluides biologiques de patients atteints du cancer du côlon. La Demanderesse a quant à elle confirmé, dans la demande de brevet WO2009/019368, que la concentration de cette protéine était diminuée par rapport aux valeurs de référence déterminées pour les patients sains, de sorte que cette protéine est un bon marqueur dans les échantillons biologiques issus d'un patient atteint d'un cancer colorectal, lesdits échantillons étant distants de la tumeur.

Le marqueur Intestinal Fatty Acid-Binding Protein (N° Swiss Prot P12104, également appelé I-FABP, FABP-2 ou FABPI) a été séquencé en 1987²³. C'est une petite protéine de 15 kDa, abondante dans le cytosol, possédant la capacité de se fixer aux acides gras libres ainsi qu'à la bilirubine. La protéine I-FABP est exprimée au niveau des entérocytes de l'intestin grêle et peut constituer environ 2% du contenu protéique de ce type cellulaire. Au niveau tissulaire, le duodenum et le jejunum contiennent des quantités significativement plus élevées de I-FABP que le côlon (jejunum : 4,8 µg/g, côlon : 0,25 µg/g)²⁴. La I-FABP n'a pas pu être détectée dans les échantillons de plasma de sujets sains. Par contre, dans certains contextes pathologiques comme l'ischémie intestinale, la maladie de Crohn ou la cirrhose biliaire primitive, il est possible de mettre en évidence une augmentation de la concentration de I-FABP plasmatique chez certains sujets²⁴. Pour le cancer de la prostate, il a été montré que le niveau d'expression des ARNm du I-FABP dans les biopsies de tissu tumoral était 7 fois plus élevé que dans le tissu normal²⁰. Dans le modèle d'induction de tumeur colorectal par l'azoxyméthane chez le rat, le niveau d'expression des ARNm de la I-FABP est diminué de 2,92 à 3,97 fois lorsque les animaux ont une alimentation qui réduit l'incidence de cancer (protéines du soja ou hydrolysat de petit lait)²⁵. La Demanderesse a confirmé, par exemple dans la demande de brevet WO2009/0l9366, que la concentration de cette protéine était augmentée par rapport aux valeurs de référence déterminées pour les patients sains, de sorte que cette protéine est un bon marqueur dans les échantillons biologiques issus d'un patient atteint d'un cancer colorectal, lesdits échantillons étant distants de la tumeur.

Les Apolipoprotéines sont une famille de protéines constituées d'acides aminés polaires permettant le transport des lipides dans le sang par formation d'un complexe macromoléculaire hydrophile appelé lipoprotéine. Pour chacune des Apolipoprotéines plasmatiques humaines existent des isoformes issues de polymorphisme génétique et/ou de modifications post-traductionnelles dont la présence dans le sang peut être associée à certaines pathologies²⁶. La concentration plasmatique des Apolipoprotéines est non négligeable, de l'ordre du mg/ml²⁷.

Le marqueur Apolipoprotéine AI (N° NCBI 490098, également appelé Apo A-I, Apo AI et Apo A1) est une protéine de 243 acides aminés et de 28 kDa. Il est essentiellement synthétisé par le foie et l'intestin. Cette protéine a été montrée sous-abondante dans les sérums de patients souffrant d'un cancer colorectal par rapport aux sujets sains par SELDI-TOF²⁸. Cependant, il est précisé dans cet article que la discrimination des patients atteints de CCR par rapport aux sujets sains est réalisée en combinant l'Apo AI à d'autres marqueurs protéiques. Par ailleurs, cet article précise que le dosage par immunoessai turbidimétrique de l'Apo AI réalisée par une autre équipe ne confirme pas la sous-abondance de cette protéine dans les sérums de patients atteints de CCR²⁹. Hachem et al.³⁰ ont quant à eux dosé l'Apo AI dans des sérums de patients ayant eu le cancer du foie suite à des métastases du cancer colorectal. La Demanderesse a montré quant à elle de façon surprenante qu'un dosage par immunoessai permet de mettre en évidence la diminution de la concentration de cette protéine chez les patients atteints d'un cancer colorectal, contrairement à ce qui était avancé par Engwegen et al.²⁸ qui ont pu mettre en évidence cette diminution uniquement en mettant en oeuvre la technique SELDI-TOF. Le dosage par immunoessai de l'Apo AI dans les échantillons biologiques est un bon procédé de diagnostic du cancer colorectal, lesdits échantillons étant distants de la tumeur, dans la mesure où le dosage par immunoessai mis en oeuvre n'est pas la turbidimétrie comme utilisée par l'équipe de Zhang et al.²⁹.

Le marqueur Apolipoprotéine AII, (N° Swiss Prot P02652, également appelé ApoA II, Apo-AII, et Apo A2) est une protéine de 17380 Da composée de deux chaînes polypeptidiques de 77 acides aminés chacune reliées par un pont disulfure. Comme l'Apolipoprotéine AI, l'Apolipoprotéine AII est essentiellement synthétisée par le foie et l'intestin. Hachem et al.³⁰ ont également dosé, outre l'Apo AI, l'Apo AII dans des sérums de patients ayant eu le cancer du foie suite à des métastases du cancer colorectal. Toutefois, les résultats ne sont pas significatifs et ne permettent pas une conclusion quant à la pathologie recherchée. La Demanderesse a montré quant à elle de façon surprenante, dans la demande de brevet WO2009/019370, que la concentration de cette protéine était diminuée par rapport aux valeurs de référence déterminées pour les patients sains, de sorte que la diminution de la concentration de cette protéine chez les patients atteints d'un cancer colorectal en fait un bon marqueur dans les échantillons biologiques issus d'un patient atteint d'un cancer colorectal, lesdits échantillons étant distants de la tumeur.

Le marqueur Plastine-I (N° Swiss Prot Q14651, également appelé I-plastin, Intestine-specific plastin ou Plastin 1) appartient à la famille des Plastines humaines dont trois représentants sont connus : la Plastine-I, la Plastine-L et la Plastine-T. Certains auteurs appellent les Plastines « Fimbrines », d'autres auteurs encore réservent le nom de Fimbrine à la Plastine-I. Les Plastines sont des protéines se liant à l'Actine pour former le cytosquelette (squelette cellulaire). Ce sont des protéines de 70 kDa relativement bien conservées tout au long de l'évolution des Eucaryotes. Elles présentent une forte spécificité tissulaire, seulement une isoforme à la fois est présente dans les tissus normaux³¹. L'utilisation des Plastines vis-à-vis du cancer a déjà été décrite dans le brevet US-A-5,360,715, qui propose une méthode pour déterminer si une cellule est hématopoïétique ou néoplasique, c'est-à-dire cancéreuse. Cette méthode revendique le dosage de la Plastine-L et de la Plastine-T au niveau cellulaire, et plus particulièrement le dosage de leurs ARNm. Toutefois, malgré ces propriétés, aucun travail antérieur n'a été réalisé pour évaluer l'intérêt des Plastines dans le cadre du diagnostic du cancer colorectal à partir d'un prélèvement de sérum ou de selles. De plus la Plastine-I n'a même jamais été envisagée comme un marqueur potentiel du cancer³². La Demanderesse a montré quant à elle de façon surprenante, dans la demande de brevet WO2009/019369, que la concentration de cette protéine était augmentée par rapport aux valeurs de référence déterminées pour les patients sains, de sorte que cette protéine est un bon marqueur dans les échantillons biologiques issus d'un patient atteint d'un cancer colorectal, lesdits échantillons étant distants ou non de la tumeur.

Le marqueur Beta 2 Microglobuline (N° Swiss Prot P61769, également appelé β2 Microglobuline, β2M) est une protéine de basse masse moléculaire (11 à 12 kDa) trouvée à la surface de la plupart des cellules humaines nucléées. Le taux de β2 Microglobuline sérique augmente chez certains patients atteints de cancer, sans que cette augmentation soit spécifique, ni corrélée avec la nature de la tumeur, son stade ou la sévérité de la maladie. Une augmentation significative est également observée lors d'autres maladies telles que le lupus érythémateux, l'arthrite rhumatoïde, le syndrome de Sjögren, les maladies malignes du système lymphoïde (myélome multiple, lymphome à cellules B), certaines maladies virales (hépatites ou SIDA) et chez les patients hémophiles. La β2 Microglobuline étant filtrée par les glomérules rénaux et réabsorbée par les tubes contournés proximaux, sa concentration sanguine peut être modifiée en cas de pathologies rénales. C'est ainsi que le dosage de la β2 Microglobuline est le plus souvent réservé au diagnostic de pathologies rénales, ou au suivi d'infection par le virus de l'immunodéficience acquise. Toutefois, ce marqueur est connu comme un marqueur tumoral, notamment du cancer du côlon.

Le marqueur Protéasome 20S (également appelé Prosome) est la structure centrale du protéasome qui est lui-même un complexe moléculaire responsable de la dégradation intracellulaire des protéines ubiquitinylées³³. Le Protéasome est un complexe moléculaire de 700 kDa constitué de 28 sous-unités associées en 4 anneaux de 7 sous-unités. Chez l'homme, 7 unités alpha (α1, α2, α3, α4, α5, α6 et α7) et 10 unités bêta (β1, β2, β3, β4, β5, β6, β7, β1i, β2i et β5i) sont connues. Grâce à ses propriétés catalytiques, le Protéasome joue un rôle central dans les mécanismes de prolifération, de croissance, de régulation et d'apoptose cellulaire et donc dans les voies de cancérisation. L'inhibition du Protéasome par le Bortezomib (Velcade) est un traitement reconnu des myélomes multiples. Des essais thérapeutiques de phase II ou III sont en cours pour des cancers hématologiques ou des tumeurs. Lavabre-Bertrand et al.³⁴ ont montré que le taux sérique de Protéasome pouvait s'élever à l'occasion de certaines pathologies, notamment en cas de cancers (myélome, lymphome et tumeurs solides).

Le marqueur Galectine-3 (N° Swiss Prot P1 7931, également appelé Gal-3. Galactose-specific lectin 3, MAC-2 antigen, IgE-binding protein, 35 kDa lectin, Carbohydrate binding protein 35, CBP 35, Laminin-binding protein, Lectin L-29, L-31,Galactoside-binding protein ou GALBP), est une lectine capable de se lier à des structures beta-galactosidique de type N-acetyllactosamine. C'est une protéine à fonctions multiples impliquée dans diverses fonctions biologiques, incluant l'adhésion des cellules tumorales, la prolifération, la différentiation, l'angiogenèse, l'apoptose, la progression cancéreuse métastasique³⁵. Différents travaux ont montré que Gal-3 pouvait se complexer avec de nombreuses molécules : ACE, IgE, Laminine, Mucine, Mac-2BP, LAMP1, LAMP2, Fibronectine, etc. Un dosage sérique de Gal-3 a été décrit par Iurisci et al.³⁶. Gal-3 était capturée sur des microplaques revêtue de Mac-2-binding protein (une protéine liant Gal-3) puis révélée avec un anticorps de rat anti-Gal-3. Cette étude a montré une élévation sérique de Gal-3 en cas de cancers gastro-intestinaux, du sein, du poumon, de l'ovaire, de mélanomes et de lymphomes non-Hodgkinien.

Le marqueur L-Lactate Deshydrogénase Chaîne B (N° Swiss Prot P07195, également appelé LDH-B, LDH Heart Unit ou LDH-H) est une protéine pouvant se complexer sous forme d'homotétramère. Cette protéine peut également se complexer avec la protéine L-Lactate Deshydrogénase Chaîne A (N° Swiss Prot P00338, également appelé LDH-A, LDH Muscle Unit ou LDH-M) sous forme d'hétérotétramères. La dose sérique et/ou l'activité enzymatique sérique des complexes tétramerique, baptisé LDH, augmente dans la circulation sanguine proportionnellement à la masse tumorale pour de nombreuses tumeurs solides. Son utilisation est recommandée en association avec la gonadotrophine chorionique humaine (beta-hCG) et la phosphatase alcaline placentaire pour le suivi des cancers séminaux. La LDH est considérée comme un marqueur d'intérêt pour le pronostic des lymphomes, de la leucémie et du cancer du côlon³⁷.

Le marqueur Calréticuline (N° Swiss Prot P27797, également appelé CRP55, Calregulin, HACBP, ERp60 ou grp60) est une protéine multifonctionnelle. C'est une lectine capable d'interagir transitoirement avec la quasi-totalité des protéines monoglycosylées du réticulum endoplasmique. C'est ainsi que McCool et al.³⁸ ont montré que la Calréticuline était impliquée dans la maturation de la mucine colique MUC2. Une méthode de diagnostic du CCR utilisant un dosage de la Calréticuline dans un tissu, les selles ou un fluide corporel est décrit dans la demande de brevet WO03/065003.

Le marqueur Regenerating Islet-Derived Protein 3 Alpha (N° Swiss Prot Q06141, également appelé Reg III-alpha, Pancreatitis-associated protein 1 ou Pancreatis Associated Protein 1 (PAP 1)) est une protéine faiblement exprimée dans le pancréas sain. Elle est surexprimée durant les phases aiguës de pancréatite et chez certains patients souffrant de pancréatite chronique. Elle apparaît alors dans le liquide pancréatique et dans la circulation sanguine³⁹. Motoo et al.⁴⁰ ont montré par dosage ELISA que le taux de PAP 1 sanguine augmentait chez certains patients atteints de cancer du côlon, de l'estomac, du foie ou du pancréas, ainsi qu'en cas d'insuffisance rénale. Ils ont pour ce faire utilisé le test ELISA (PANCEPAP) commercialisé par la société Dynabio (La Gaude, France).

Le marqueur Tumor-Associated Calcium Signal Transducer 1 (N° Swiss Prot P16422, également appelé Major gastrointestinal tumor-associated protein GA733-2, Epithelial cell surface antigen, EpCAM, Epithelial glycoprotein, EGP, Adenocarcinoma-associated antigen, KSA, KS 1/4 antigen, Cell surface glycoprotein Trop-1 ou CD326 antigen) a été caractérisé en 1979 par sa capacité à être reconnu par un anticorps dirigé contre des cellules de cancer colorectal⁴¹. Cette protéine est connue sous différents noms, comme indiqué précédemment, mais l'usage le plus fréquent est de l'appeler EpCAM. C'est une protéine transmembranaire exprimée sur la surface basolatérale des cellules, dans certains épithéliums et de nombreux cancers⁴². Dès 1982 Herlyn et al.⁴³ ont montré que l'injection d'un anticorps monoclonal anti-EpCAM pouvait inhiber la croissance tumorale de patients atteints de cancer colorectal. Ces résultats ont conduit au développement d'un traitement anti-tumoral à base d'un anticorps anti-EpCAM nommé Edrecolomab. Ce traitement est commercialisé sous le nom de Panorex™. Par ailleurs, Abe et al.⁴⁴ ont montré par dosage ELISA qu'une forme soluble d'EpCAM, baptisée MK-1, était augmentée dans la circulation sanguine de 10% chez les patients cancéreux étudiés.

Les cytokératines font partie des protéines qui composent les filaments intermédiaires du cytosquelette des cellules épithéliales. Actuellement, plus de 20 cytokératines humaines ont été identifiées. Les cytokératines 8 (N° Swiss Prot P05787, également appelé Cytokeratin-8, CK-8, Keratin-8 ou K8), 18 (N° Swiss Prot P05783, également appelé Cytokeratin-18, CK-18, Keratin-18 ou K18), et 19 (N° Swiss Prot P08727, également appelé Cytokeratin-19, CK-19, Keratin-19 ou K19) sont les plus abondantes dans les cellules épithéliales et sont des outils utiles pour le diagnostic de pathologies cancéreuses⁴⁵. Cet intérêt clinique est lié à la libération de cytokératines par les cellules épithéliales en phase d'apoptose ou de prolifération. En cas d'apoptose, cette libération se fait sous forme de fragments solubles qui semble apparaître sous l'action protéolytique de Caspases. Des formes de cytokératines non dégradées n'ont jamais été décrites dans la circulation sanguine. Les trois dosages de cytokératines les plus utilisés en clinique sont le dosage de l'antigène polypeptidique tissulaire (TPA), de l'antigène polypeptidique tissulaire spécifique (TPS), et CYFRA 21-1. TPA est un test de spectre large qui mesure les cytokératines 8, 18, et 19. Les dosages de TPS et de CYFRA 21-1 sont plus spécifiques et mesurent respectivement des fragments de la cytokératine 18 et de la cytokératine 19. Ces 3 dosages détectent des fragments solubles de cytokératines pouvant être présents isolément ou sous forme de complexes protéiques. TPA, TPS ou CYFRA-21-1 ont été utilisés pour le suivi thérapeutique des cancers colorectaux, du sein, du poumon, de la vessie, de l'ovaire, du pancréas, de la prostate et de certains cancers ORL. Le dosage sanguin des fragments solubles de cytokératines a en effet une valeur clinique pour dépister les récidives ou évaluer la réponse à la thérapie engagée (radiothérapie, chimiothérapie, traitement hormonal). Un dosage régulier permet notamment d'évaluer la progression de la masse tumorale. La dose de cytokératines sanguines solubles a également un aspect pronostique vis-à-vis du stade tumoral et de la formation de métastase. Actuellement le dosage sanguin de cytokératine le plus utilisé est CYFRA 21-1. Il est fortement recommandé pour le suivi de patients atteints de cancer du poumon non à petites cellules. Il existe divers dosages commerciaux pour TPA (AB Sangtec Medical Co., Byk-Roland...), TPS (IDL Biotech AB, BEKI Diagnostics...) et CYFRA-21-1 (Roche Diagnostics, CIS Bio-Intemational, Fujirebio Diagnostics...). Par ailleurs, Kim et al.⁴⁶ ont montré que le dosage dans les selles de cytokératine 19 (DiNonA Inc.) pouvait être utile au dépistage de maladies digestives en association avec un dosage de sang occulte dans les selles.

Le marqueur Epithelial-Cadhérine (N° Swiss Prot P12830, également appelé E-cadherin, Uvomorulin, Cadherin-1, CAM 120/80 ou CD324 antigen) est une protéine transmembranaire médiatrice de l'adhésion cellulaire calcium dépendante. Elle est spécifiquement exprimée dans les cellules épithéliales, où elle est impliquée dans le maintien de leur phénotype. Le domaine cytoplasmique de l'E-Cadhérine se lie à la β-Caténine, qui est elle-même liée aux réseaux de filaments d'actine du cytosquelette. Cette liaison l'E-Cadhérine/β-Caténine joue un rôle critique pour stabiliser les adhésions cellules/cellules du tissu épithélial. La perte d'E-Cadhérine peut donc réduire l'adhésion cellulaire et augmenter le pouvoir invasif des cellules cancéreuses. Une réduction d'expression d'E-Cadhérine ou de β-Caténine est généralement associée avec une dédifférenciation et une agressivité plus importante de la tumeur, notamment pour les cancers digestifs. C'est ainsi que Roca et al.⁴⁷ ont montré que les patients atteints d'un cancer colorectal et sous-exprimant l'E-Cadhérine avaient un pronostic plus péjoratif que les patients ayant un niveau d'expression normal. Dès 1983, Damsky et al.⁴⁸ ont montré qu'une forme soluble d'E-Cadhérine pouvait être libérée par la lignée cellulaire du cancer du sein MCF-7. Cette forme soluble'correspond au clivage de la partie extracellulaire de l'E-Cadhérine. Plus tard, Katayama et al.⁴⁹ ont montré que la forme soluble de l'E-Cadhérine pouvait être libérée dans la circulation sanguine en cas de cancer et Willmanns et al.⁵⁰ ont montré que l'augmentation de la dose d'E-Cadhérine sanguine était corrélée au stade tumoral pour les cancers colorectaux. Un kit commercial est par ailleurs proposé par la société Takara BioChemicals (Tokyo, Japon).

Le dosage de l'ACE (Antigène Carcino-Embryonnaire) pour le diagnostic du cancer colorectal a été proposé depuis 1965 par Gold et Freedman⁵¹, mais un dosage sanguin de ce marqueur a une sensibilité médiocre pour le diagnostic de cancers colorectaux à un stade peu avancé. C'est ainsi que le dosage de l'ACE sérique est surtout recommandé pour évaluer le risque de métastases hépatiques⁵² et pour le suivi thérapeutique. En outre, c'est un marqueur peu spécifique du cancer colorectal, il peut en effet être augmenté dans de nombreux autres cancers (poumon, sein, ...). En revanche, le dosage d'ACE dans les selles semble plus sensible et plus spécifique que le dosage de l'ACE sérique ou que le dosage de sang dans les selles⁵³. Ce dosage n'est toutefois pas encore proposé en routine.

Les déterminants antigéniques 1116-NS-19-9 réactifs, plus communément baptisés CA19-9 (Carbohydrate Antigen 19.9), sont portés par des protéines de poids moléculaires élevés⁵⁴. Le dosage sanguin de CA 19-9 est plus spécifique que celui de l'ACE. Le taux de CA 19-9 sanguin augmente en cas de cancer colorectal, du pancréas et du foie (cholangiocarcinome), mais aussi en cas de pathologies non cancéreuses (cholangites...). Son usage en association avec l'ACE est recommandé tant au moment du diagnostic d'un cancer que pour le suivi de la pathologie.

J. Holmgren et al.⁵⁵ ont montré que la dose sérique d'antigène CA 50 était augmentée en cas de cancer colorectal. L'antigène CA 50 est défini par sa faculté à être reconnu par un anticorps monoclonal spécifique.

S'agissant du marqueur CA 72, T.L. Klug et al.⁵⁶ ont montré que la dose sérique d'antigène CA 72 était augmentée en cas de cancer colorectal. L'antigène CA 72 est défini par sa faculté à être reconnu par un anticorps monoclonal spécifique.

De même, P. Kuusela et al.⁵⁷ ont montré que la dose sérique d'antigène CA 242 était augmentée en cas de cancer colorectal. L'antigène CA 242 est défini par sa faculté à être reconnu par un anticorps monoclonal spécifique.

Le dosage de la Testostérone pour le diagnostic du cancer colorectal a été proposé chez les hommes par M. Holland et al.⁵⁸. Ces auteurs ont montré un effondrement du taux de Testostérone sanguine en cas de cancer colorectal.

S'agissant du marqueur TIMP-1, ou Tissue Inhibitor of Matrix Metalloproteinase Type-1, la demande de brevet US 2007/0020707 décrit notamment le dosage de TIMP-1 pour le diagnostic du cancer colorectal à l'aide d'un dosage dans un fluide corporel.

F. Model et al.59 ont montré en juillet 2006, lors du congrès World Congress on Gastrointestinal Cancer, qu'il était possible de détecter des formes méthylées du gène de la Septin-9 dans le plasma de patients atteints de cancer colorectal.

M.P. Ebert et al.⁶⁰ ont montré que le gène ALX4, ou Aristaless-like homeobox-4, était plus souvent méthylé dans les sérums de patients atteints d'un cancer colorectal que dans les sérum contrôles (P < 0,0001). En utilisant une valeur seuil de 41,4 pg/mL, ils ont obtenu une sensibilité de 83,3% et une spécificité de 70%.

La Villine est décrite en tant que marqueur sanguin pour le diagnostic du cancer colorectal dans la demande de brevet FR2581456.

C. Bianco et al.⁶¹ ont montré que la dose sérique de Cripto-1 était augmentée en cas de cancer colorectal.

L'induction de la tumorogenèse intestinale par la Macrophage Migration Inhibitory Factor (MIF) a été décrite par Wilson et al.⁶². Plus récemment, il a été également montré par Lee et al.⁶³ que MIF était un marqueur sanguin potentiel pour le diagnostic précoce du cancer colorectal.

Le Marqueur Protéine Disulfide Isomérase (N° Swiss Prot P07237, également appelé EC 5.3.4.1, PDI, Prolyl 4-hydroxylase subunit beta, Cellular thyroid hormone-binding protein ou p55) est une protéine multifonctionelle qui catalyse la formation, la rupture et le réarrangement des ponts disulfures intramoléculaire. A la surface des cellules, elle agit en tant que réductase et clive les ponts disulfure des protéines attachées aux cellules. A l'intérieur des cellules, c'est une molécule soluble localisée dans la lumière du réticulum endoplasmique, où elle forme et réarrange les ponts disulfures des protéines néosynthétisées. Elle comporte 2 domaines catalytiques de type Thiorédoxine ayant un motif CXXC caractéristique. A haute concentration, la PDI fonctionne comme une protéine chaperonne qui inhibe l'agrégation des protéines mal repliées. A faible concentration, elle a un rôle antagoniste et facilite l'agrégation. La PDI forme également la sous-unité structurale de différentes enzymes, comme la Prolyl Hydroxylase qui catalyse l'hydroxylation des résidus proline des chaînes pro-alpha du procollagène. Dans la demande de brevet EP1724586, la PDI a été décrite comme marqueur de diagnostic pour certains cancers, comme le cancer du côlon.

Le dosage de l'Intélectine-1 (N° Swiss Prot Q8WWA0, également appelé Intestinal lactoferrin receptor, Galactofuranose-binding lectin, Endothelial lectin HL-1 ou Omentin) pour le diagnostic du cancer colorectal a été décrit dans la demande de brevet US2003/0082533.

L'utilisation de la Translationally-Controlled Tumor Protein (N° Swiss Prot P13693, également appelé TCTP, p23, Histamine-releasing factor, HRF ou Fortilin) et de la Prodéfensine-A5 (N° Swiss Prot Q01523) en tant que marqueurs dans le cancer colorectal est décrite respectivement dans les demandes de brevet US2003/0172388 et US2006/0179496. Par (Pro)défensine, on entend, le précurseur, à savoir la Prodéfensine avant clivage, le propeptide, à savoir la moitié N-terminale après clivage de la Prodéfensine, et la protéine mature, à savoir la Défensine, correspondant à la moitié C-terminale après clivage.

M2-PK est un isoenzyme de pyruvate kinase qui est retrouvé sous forme dimérique ou tétramérique. La forme dimérique est prédominante dans les cellules tumorales et pour cette raison appelée M2-PK tumorale. De nombreuses études ont utilisé le dosage ELISA de M2-PK dans les selles en tant que marqueur de cancer colorectal, comme par exemple Hardt et al.⁶⁴

L'utilisation de la Calgranuline C ou protéine S100 A12 comme marqueur du cancer colorectal est décrite dans la demande de brevet WO2007/134779.

Sagiv et al.⁶⁵ ont montré une augmentation de l'expression de CD24 en cas de cancer colorectal.

Les protéines Colon Cancer Specific Antigen (CCSA)-3 et -4 sont des marqueurs sériques qui ont aussi été associées au cancer colorectal par Leman et al.⁶⁶

Enfin, le dosage d'hémoglobine humaine dans les selles est connu et peut être mis en oeuvre comme décrit précédemment.

La concentration du marqueur tumoral autres que la ProDéfensine-A6 sera, selon le marqueur considéré, augmentée ou diminuée dans l'échantillon biologique dans lequel on met en oeuvre le procédé de l'invention par rapport aux valeurs de référence déterminées pour les patients sains.

De préférence, le ou les marqueurs autres que la Prodéfensine-A6 son choisis parmi : Leucocyte Elastase Inhibitor, Ezrine, Aminoacylase 1, Liver Fatty Acid-Binding Protein, Intestinal Fatty Acid-Binding Protein, Apolipoprotéine AI, Apolipoprotéine AII, Plastine-I, Protéine Disulfide Isomérase, Intélectine-1, Cytokératine 20, Translationally-Controlled Tumor Protein, (Pro)défensine-A5, Galectine-3, Beta 2 Microglobuline, ACE, CA19-9, TIMP-1, M2-PK et MIF.

De préférence encore, le ou les marqueurs tumoraux autres que la ProDéfensine-A6 sont choisis parmi les marqueurs : L-FABP, Beta 2 Microglobuline, Galectine-3, ACE, CA19-9, MIF et Plastine-I.

Selon un mode de réalisation particulier, le procédé de l'invention comprend ou consiste en la détection des marqueurs suivants :
- ProDéfensine-A6 et L-FABP,
- ProDéfensine-A6, CA19-9 et ACE,
- ProDéfensine-A6, Béta-2 Microglobuline et ACE
- ProDéfensine-A6, Béta-2 Microglobuline, CA19-9 et ACE,
- ProDéfensine-A6, Béta-2 Microglobuline, L-FABP et ACE,
- ProDéfensine-A6, L-FABP, CA19-9 et ACE,
- ProDéfensine-A6, Béta-2 Microglobuline, CA19-9 et ACE,
- ProDéfensine-A6, Béta-2 Microglobuline, CA19-9, L-FABP et ACE,
- ProDéfensine-A6, Béta-2 Microglobuline, CA19-9, Galectine-3, L-FABP, MIF et ACE,
- ProDéfensine-A6, Béta-2 Microglobuline, CA19-9, Galectine-3, L-FABP, MIF, Plastine I et ACE.

Bien entendu, le procédé de l'invention peut également inclure la détection de tout autre marqueur du cancer colorectal connu de l'homme du métier.

Comme indiqué précédemment, on détecte le ou les marqueurs tumoraux d'intérêt soit sous forme de protéine, soit sous forme d'ARN messager, soit par altération de l'ADN correspondant (mutation ou modification des méthylations), étant entendu que la ProDéfensine-A6 n'est détectée que sous forme protéique, complète ou sous forme de fragment de protéine

La détermination de la présence, dans l'échantillon biologique, du marqueur tumoral d'intérêt « protéine » peut être mis en oeuvre par tout procédé de détermination de la présence d'une protéine dans un échantillon, connu de l'homme du métier, comme par exemple par un test biochimique, y compris un dosage immunologique, ou par spectrométrie de masse.

Le test biochimique peut être tout test largement connu de l'homme du métier impliquant des interactions moléculaires, à savoir des réactions entre ledit marqueur tumoral et un ou des partenaire(s) de liaison spécifique(s) ou non dudit marqueur tumoral.

De préférence, le test biochimique est un dosage immunologique connu de l'homme du métier impliquant des réactions immunologiques entre le marqueur tumoral qui est l'antigène et un ou des partenaire(s) de liaison spécifique(s) que sont les anticorps dirigés contre cet antigène.

Les partenaires de liaison spécifiques ou non du ou des marqueurs tumoraux recherchés dans le procédé de l'invention sont tout partenaire susceptible de se lier à ce ou ces marqueurs. Ils sont dits spécifiques quand ils sont capables de se lier à ces marqueurs avec une spécificité élevée, voire une spécificité de 100%. Ils sont dits non spécifiques lorsque leur spécificité de liaison à ces marqueurs est faible et qu'ils sont alors capables de se lier à d'autres ligands, tels que des protéines. A titre d'exemple, on peut citer les anticorps, les fractions d'anticorps, les récepteurs et toute autre molécule capable de se lier à ce marqueur.

Les anticorps partenaires de liaison sont par exemple soit des anticorps polyclonaux, soit des anticorps monoclonaux.

Le partenaire de liaison spécifique de la ProDéfensine-A6 est un anticorps monoclonal qui reconnaît tout épitope, linéaire ou conformationnel, compris dans la séquence SEQ ID N°2.

Selon l'invention, le procédé met en oeuvre un anticorps monoclonal spécifique de la ProDéfensine-A6 reconnaissant l'épitope 1 de SEQ ID N°6 (épitope 1).

Les anticorps monoclonaux anti-ProDéfensine-A6 reconnaissant spécifiquement l'épitope 1 de séquence SEQ ID N°6 sont nouveaux et constituent un autre objet de l'invention.

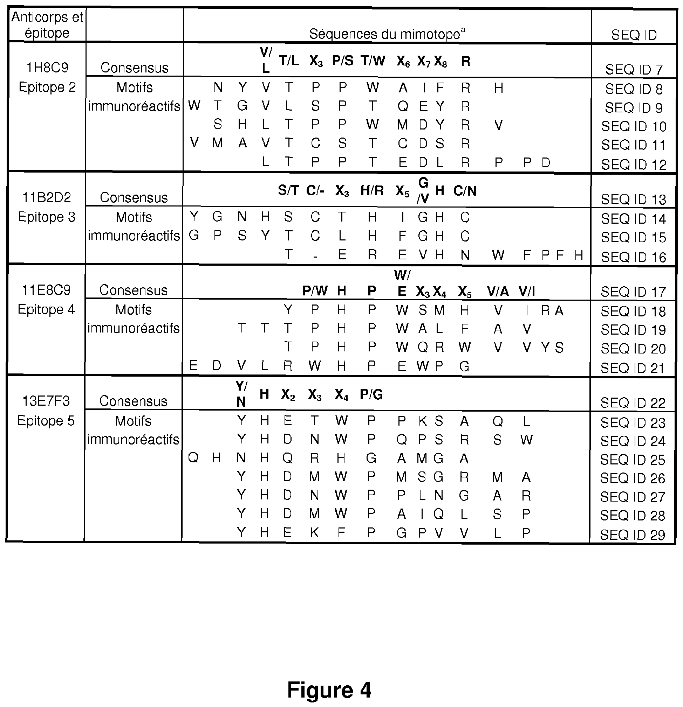

D'autres anticorps monoclonaux anti-ProDéfensine-A6 reconnaissent spécifiquement un épitope conformationnel choisi parmi les épitopes suivants :
- un épitope d'au moins la séquence SEQ ID N°7, dans laquelle X₁ est V ou L, X₂ est T ou L, X₃ est P, S ou C, X₄ est P ou S, X₅ est W ou T, X₆ est A, Q, M, C ou E, X₇ est I, E ou D et X₈ est F, Y, S ou L, et d'au plus la séquence SEQ ID N°8, SEQ ID N°9, SEQ ID N°10, SEQ ID N°11, SEQ ID N°12, telles qu'indiquées sur la figure 4 (épitope 2),
- un épitope d'au moins la séquence SEQ ID N°13, dans laquelle X₁ est S ou T, X₂ est C ou absent, X₃ est T, L ou E, X₄ est H ou R, X₅ est I, F ou E, X₆ est G ou V et X₇ est C ou N, et d'au plus la séquence SEQ ID N°14, SEQ ID N°15 ou SEQ ID N°16, telles qu'indiquées sur la figure 4 (épitope 3),
- un épitope d'au moins la séquence SEQ ID N°17, dans laquelle X₁ est P ou W, X₂ est W ou E, X₃ est S, A, Q ou W, X₄ est M, L, R ou P, X₅ est H, F, W ou G, X₆ est V ou A et X₇ est I ou V, et d'au plus la séquence SEQ ID N°18, SEQ ID N°19, SEQ ID N°20 ou SEQ ID N°21 , telles qu'indiquées sur la figure 4 (épitope 4),
- un épitope d'au moins la séquence SEQ ID N°22, dans laquelle X₁ est Y ou N, X₂ est E, D ou Q, X₃ est T, N, R, M ou K, X₄ est W, H ou F et X₅ est P ou G, et d'au plus la séquence SEQ ID N°23, SEQ ID N°24, SEQ ID N°25, SEQ ID N°26, SEQ ID N°27, SEQ ID N°28 ou SEQ ID N°29, telles qu'indiquées sur la figure 4 (épitope 5).

Selon un mode de réalisation, le procédé de l'invention met en oeuvre un anticorps monoclonal spécifique d'un épitope 1 de séquence SEQ ID N °6 et un anticorps monoclonal spécifique d'un épitope 2, 3, 4 ou 5. De préférence, le procédé de l'invention met en oeuvre un anticorps monoclonal spécifique d'un épitope 1 et un anticorps monoclonal spécifique d'un épitope 2 ou 4. De préférence encore, le procédé de l'invention met en oeuvre un anticorps monoclonal spécifique d'un épitope 1 et un anticorps monoclonal spécifique d'un épitope 2.

Par épitope, on entend un peptide ayant au moins les séquences telles que définies par les séquences SEQ ID N°1 à 29, et au plus 10, 8, 6 ou 4 acides aminés supplémentaires répartis de part et d'autre de la séquence considérée, de façon homogène ou non, ou bien d'un seul côté, ainsi que leurs analogues, homologues et équivalents structuraux.

De façon générale, le terme « analogue » se réfère à des peptides ayant une séquence et une structure polypeptidique native présentant une ou plusieurs additions, substitutions (généralement conservatrice en termes de nature) et/ou délétions d'acide aminé, par rapport à la molécule native, dans la mesure où les modifications ne détruisent pas la réactivité antigénique.

Les analogues particulièrement préférés incluent les substitutions conservatrices en nature, c'est-à-dire les substitutions qui prennent place dans une famille d'acides aminés. Spécifiquement, les acides aminés sont généralement divisés en 4 familles, à savoir (1) les acides aminés acides tels que l'aspartate et le glutamate, (2) les acides aminés basiques tels que la lysine, l'arginine et l'histidine, (3) les acides aminés non polaires tels que l'alanine, la leucine, l'isoleucine, la proline, la phénylalanine, la méthionine et le tryptophane et (4) les acides aminés non chargés polaires tels que la glycine, l'asparagine, la glutamine, la cystéine, la sérine, la thréonine et la tyrosine. La phénylalanine, le tryptophane et la tyrosine sont parfois classés en acides aminés aromatiques. Par exemple, on peut prédire de façon raisonnable qu'un remplacement isolé de leucine par de l'isoleucine ou de la valine, d'un aspartate par un glutamate, d'une thréonine par une sérine, ou un remplacement conservateur similaire d'un acide aminé par un autre acide aminé ayant un rapport structurel, n'aura pas d'effet majeur sur l'activité biologique. L'homme du métier déterminera facilement les régions de la molécule peptidique d'intérêt qui peuvent tolérer un changement par référence aux plots Hopp/Woods et Kyte-Doolite, biens connus dans la technique.

Par « homologie », on entend le pourcentage d'identité entre deux molécules peptidiques. Deux séquences d'acides aminés sont « sensiblement homologues » l'une par rapport à l'autre lorsque les séquences présentent au moins 60%, de préférence au moins 75%, de préférence encore au moins 80-85%, de préférence encore au moins 90% et d'avantage préféré au moins 95-98% ou plus d'identité de séquence sur une longueur définie des molécules peptidiques.

Par « équivalent structural », on entend toute séquence peptidique, linéaire ou non, incluse dans la protéine d'intérêt, présentant la même structure tridimensionnelle que l'épitope conformationnel d'intérêt, tel les épitopes de séquences SEQ ID N°7 à 29, tout en conservant la réactivité antigénique. Un tel « équivalent structural » peut être obtenu facilement par l'homme du métier à partir de l'épitope conformationnel d'intérêt en utilisant un système bioinformatique permettant de trouver des similarités de structures ou substructures 3D de protéines, tels que les systèmes SuMo⁶⁷ ou Superimposé⁶⁸.

Les anticorps polyclonaux peuvent être obtenus par immunisation d'un animal avec le marqueur tumoral concerné, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixé un antigène spécifiquement reconnu par les anticorps, notamment ledit marqueur.

Les anticorps monoclonaux peuvent être obtenus par la technique des hybridomes dont le principe général est rappelé ci-après.

Dans un premier temps, on immunise un animal, généralement une souris avec le marqueur tumoral d'intérêt, dont les lymphocytes B sont alors capables de produire des anticorps contre ledit antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis dudit marqueur tumoral pourront être testées par exemple en ELISA, par immunotransfert (Western blot) en une ou deux dimensions, en immunofluorescence, ou à l'aide d'un biocapteur. Les anticorps monoclonaux ainsi sélectionnés, sont par la suite purifiés notamment selon la technique de chromatographie d'affinité décrite ci-dessus.

Les anticorps monoclonaux peuvent être également des anticorps recombinants obtenus par génie génétique, par des techniques bien connues de l'homme du métier.

Des exemples d'anticorps anti-Défensine-A6 sont connus et sont disponibles notamment dans le catalogue Alpha Diagnostic International Inc., anticorps polyclonal de lapin anti-Défensine-A6, Cat. No. HDEFA61-A. Aucun anticorps monoclonal dirigé contre la Prodéfensine-A6 n'est disponible à ce jour.

Des exemples d'anticorps anti-Leucocyte Elastase Inhibitor sont connus et sont disponibles notamment dans le catalogue Abcam, anticorps polyclonal de lapin anti-LEI, Cat. No. Ab47731. Un anticorps monoclonal anti-LEI Clone ELA-1 a été décrit dans l'article de Yasumatsu et al.⁶⁸.

Des exemples d'anticorps anti-Ezrine sont connus et sont disponibles notamment dans le catalogue Abcam, anticorps monoclonal anti-Ezrine Clone 3C12, Cat. No. Ab4069 et anticorps polyclonal de lapin anti-Ezrine, Cat. No. Ab47418.

Des exemples d'anticorps anti-Aminoacylase 1 sont connus et sont disponibles notamment dans le catalogue Abnova, anticorps monoclonal anti-Aminoacylase 1 Clone 4F1-B7, Cat. No. H00000095-M01, et dans le catalogue Abcam, anticorps polyclonal de poule anti-Aminoacylase 1, Cat. No. Ab26173.

Des exemples d'anticorps anti-Liver Fatty Acid-Binding Protein sont connus et sont disponibles notamment dans le catalogue Abcam, anticorps monoclonal anti-L-FABP Clone 6B6, Cat. No. Ab10059 et anticorps polyclonal de lapin anti-L-FABP, Cat. No. Ab7807.

Des exemples d'anticorps anti-Intestinal Fatty Acid-Binding Protein sont connus et sont disponibles notamment dans le catalogue R&D Systems, anticorps monoclonal anti-I-FABP Clone 323701, Cat. No. MAB3078, et dans le catalogue Abcam, anticorps polyclonal de lapin anti-I-FABP, Cat. No. Ab7805.

Des exemples d'anticorps anti-Apolipoprotéine AI sont connus et sont disponibles notamment dans le catalogue Biodesign Meridian Life Sciences, anticorps monoclonal anti-Apo AI Clone 4A90, Cat. No. H45402M et anticorps polyclonal de chèvre anti-Apo AI, Cat. No. K45252P.

Des exemples d'anticorps anti-Apolipoprotéine AII sont connus et sont disponibles notamment dans le catalogue US Biological, anticorps monoclonal anti-Apo AII Clone 1402, Cat. No. A2299-31C et dans le catalogue Biodesign Meridian Life Sciences anticorps polyclonal de chèvre anti-Apo AII, Cat. No. K74001P.

Des exemples d'anticorps polyclonaux anti-Plastine-I sont connus et sont disponibles notamment dans le catalogue Santa Cruz Biotechnology. L'anticorps polyclonal de lapin H-300 (Cat. No. sc-28531) réagit avec les Plastines-I, L et T. La Demanderesse a mis au point des anticorps monoclonaux dirigés contre la Plastine-I.

Des exemples d'anticorps anti-Beta2 Microglobuline, anti-ACE, anti-CA19-9 et anti-Testostérone sont connus et sont notamment utilisés dans les trousses de dosage de la Demanderesse, respectivement Vidas® Beta2 Microglobulin, Vidas® ACE, Vidas® CA19-9™ et Vidas® Testostérone.

Des exemples d'anticorps anti-Protéasome 20S sont connus et sont disponibles notamment dans le catalogue d'Affinity Research Products.

Des exemples d'anticorps anti-Galectine-3, anti-L-Lactate Deshydrogénase Chaîne B, anti-Calréticuline, anti-Tumor-Associated Calcium Signal Transducer 1, anti-Keratine type II Cytoskeletal 8, anti-Keratine type I Cytoskeletal 18, anti-Keratine type I Cytoskeletal 19, anti-Epithelial-Cadhérine, anti-Villine et anti-TIMP-1 sont connus et sont disponibles notamment dans le catalogue Abcam.

Des exemples d'anticorps anti-Regenerating Islet-Derived Protein 3 Alpha sont connus et sont notamment utilisés dans les trousses de dosage de Dynabio (La Gaude, France).

Des exemples d'anticorps anti-CA 242, anti-CA 50, anti-CA 72-4 sont connus et sont disponibles notamment dans le catalogue Fujirebio.

Des exemples d'anticorps anti-Intélectine-1 sont connus et sont disponibles notamment dans le catalogue Alexis Biochemicals, anticorps monoclonal anti-Intélectine-1 Clone Saly-1, Cat. No. ALX-804-850-C100 et anticorps polyclonal de lapin anti-Intélectine-1, Cat. No. ALX-210-941.

Des exemples d'anticorps anti-Cytokératine 20 sont connus et sont disponibles notamment dans le catalogue Abcam, anticorps monoclonal anti-Cytokératine 20 Clone Ks20.8, Cat. No. Ab962 et anticorps polyclonal de lapin anti-Cytokératine 20, Cat. No. Ab36756.

Des exemples d'anticorps anti-TCTP sont connus et sont disponibles notamment dans le catalogue Abnova, anticorps monoclonal anti-TCTP Clone 3C7, Cat. No. 157H00007178-M01 et anticorps polyclonal anti-TCTP, Cat. No. 157H00007178-A01.

Des exemples d'anticorps anti-Défensine-A5 sont connus et sont disponibles notamment dans le catalogue Santa Cruz Biotechnology, anticorps monoclonal anti-Défensine-A5 Clone 8C8, Cat. No. sc-53997, et dans le catalogue Alpha Diagnostic International Inc., anticorps polyclonal de lapin anti-Défensine-A5, Cat. No. HDEFA51-A.

Les partenaires de liaison spécifiques ou non du ou des marqueurs tumoraux recherchés dans le procédé de l'invention peuvent être utilisés comme réactif de capture, comme réactif de détection ou comme réactifs de capture et de détection.

Selon un mode de réalisation, le partenaire de liaison spécifique de la ProDéfensine-A6 est utilisé pour la capture de la ProDéfensine-A6, ce qui permet d'améliorer la spécificité du procédé de diagnostic de l'invention.

De préférence, l'anticorps monoclonal reconnaissant spécifiquement un épitope 1 est utilisé en capture et/ou l'anticorps monoclonal reconnaissant spécifiquement un épitope 2, 3, 4 ou 5, de préférence 2, est utilisé en détection.

La visualisation des réactions immunologiques, c'est-à-dire de la liaison marqueur tumoral/partenaire de liaison, peut être effectuée par tout moyen de détection, tels que des moyens directs ou indirects.

Dans le cas de la détection directe, c'est-à-dire sans l'intermédiaire d'un marquage, on observe les réactions immunologiques par exemple par résonance plasmonique de surface ou par voltamétrie cyclique sur une électrode portant un polymère conducteur.

La détection indirecte se fait par l'intermédiaire d'un marquage, soit du partenaire de liaison dit réactif de révélation, soit du marqueur tumoral d'intérêt lui-même. On parle alors dans ce dernier cas de méthode de compétition.

Par marquage, on entend la fixation d'un réactif marqueur capable de générer directement ou indirectement un signal détectable. Une liste non limitative de ces réactifs marqueurs consiste en :
- les enzymes qui produisent un signal détectable par exemple par colorimétrie, fluorescence, luminescence, comme la peroxydase de raifort, la phosphatase alcaline, la β-galactosidase, la glucose-6-phosphate déshydrogénase,
- les chromophores comme les composés fluorescents, luminescents, colorants,
- les molécules radioactives comme le ³²P, le ³⁵S ou le ¹²⁵I, et
- les molécules fluorescentes telles que les Alexa ou les phycocyanines.

Des systèmes indirects de détection peuvent être aussi utilisés, comme par exemple des ligands capables de réagir avec un anti-ligand. Les couples ligand/anti-ligand sont bien connus de l'homme du métier, ce qui est le cas par exemple des couples suivants : biotine/streptavidine, haptène/anticorps, antigène/anticorps, peptide/anticorps, sucre/lectine, polynucléotide/complémentaire du polynucléotide. Dans ce cas, c'est le ligand qui porte le partenaire de liaison. L'anti-ligand peut être détectable directement par les réactifs marqueurs décrits au paragraphe précédent ou être lui-même détectable par un ligand/anti-ligand.

Ces systèmes indirects de détection peuvent conduire, dans certaines conditions, à une amplification du signal. Cette technique d'amplification du signal est bien connue de l'homme du métier, et l'on pourra se reporter aux demandes de brevet antérieures FR98/10084 ou WO-A-95/08000 de la Demanderesse ou à l'article de Chevalier et al.⁷⁰.

Selon le type de marquage utilisé, l'homme du métier ajoutera des réactifs permettant la visualisation du marquage.

A titre d'exemple de tests immunologiques tels que définis ci-dessus, on peut citer les méthodes « sandwich » telles qu'ELISA, IRMA et RIA, les méthodes dites de compétition et les méthodes d'immunodétection directe comme l'immunohistochimie, l'immunocytochimie, le Western-blot et le Dot-blot.

Lorsque le partenaire de liaison spécifique de la ProDéfensine-A6 est utilisé en capture dans un test « sandwich », par exemple l'anticorps spécifique de l'épitope 1, on utilisera en détection soit un partenaire de liaison spécifique de la partie mature de la Prodéfensine-A6 (acides aminés 66 à 100), soit un partenaire de liaison reconnaissant un épitope de la partie propeptide de la ProDéfensine-A6 (acides aminés 20-65) autre que celui reconnu par le partenaire de liaison utilisé pour la capture.

La spectrométrie de masse peut également être utilisée pour la détection dans le fluide biologique du ou des marqueurs tumoraux recherchés dans le procédé de l'invention. Le principe de la spectrométrie est largement connu de l'homme du métier et est décrit par exemple dans Patterson⁷¹.

Pour ce faire, l'échantillon biologique préalablement traité ou non est analysé dans un spectromètre de masse et on compare le spectre obtenu avec celui du ou des marqueurs tumoraux recherchés dans le procédé de l'invention. Un exemple de traitement préalable de l'échantillon consiste à le faire passer sur un support d'immunocapture, comportant un des partenaires de liaison du ou des marqueurs tumoraux recherchés dans le procédé de l'invention, par exemple un anticorps dirigé contre le ou les marqueurs tumoraux recherchés dans le procédé de l'invention. Un autre exemple de traitement préalable de l'échantillon peut être le pré-fractionnement de l'échantillon biologique, afin de séparer entre elles les protéines de l'échantillon. Dans des techniques bien connues de l'homme du métier, on peut par exemple tout d'abord dépléter les protéines majoritaires de l'échantillon.

La détermination de la présence, dans l'échantillon biologique, du marqueur tumoral d'intérêt « ARNm » peut être mis en oeuvre par tout procédé de détermination de la présence d'ARNm dans un échantillon, à savoir soit la détection directe de l'ARNm, soit la détection indirecte de l'ARNm, ou tout autre procédé de détermination de la présence d'un ARN dans un échantillon, connu de l'homme du métier.

Par détection directe de l'ARNm, on entend la mise en évidence de l'ARNm lui-même dans l'échantillon biologique.

La détection directe de l'ARNm dans l'échantillon biologique peut être mise en oeuvre par tout moyen connu de l'homme du métier, comme par exemple par hybridation avec un partenaire de liaison spécifique de l'ARNm, le cas échéant après amplification par la technique PCR ou NASBA.

Par hybridation, on entend le processus au cours duquel, dans des conditions appropriées, deux fragments nucléotidiques se lient avec des liaisons hydrogènes stables et spécifiques pour former un complexe double brin. Ces liaisons hydrogène se forment entre les bases complémentaires Adénine (A) et Thymine (T) (ou Uracile (U)) (on parle de liaison A-T) ou entre les bases complémentaires Guanine (G) et Cytosine (C) (on parle de liaison G-C). L'hybridation de deux fragments nucléotidiques peut être totale (on parle alors de fragments nucléotidiques ou de séquences complémentaires), c'est-à-dire que le complexe double brin obtenu lors de cette hybridation comprend uniquement des liaisons A-T et des liaisons C-G. Cette hybridation peut être partielle (on parle alors de fragments nucléotidiques ou de séquences suffisamment complémentaires), c'est-à-dire que le complexe double brin obtenu comprend des liaisons A-T et des liaisons C-G permettant de former le complexe double brin, mais également des bases non liées à une base complémentaire. L'hybridation entre deux fragments nucléotidiques dépend des conditions opératoires qui sont utilisées, et notamment de la stringence. La stringence est définie notamment en fonction de la composition en bases des deux fragments nucléotidiques, ainsi que par le degré de mésappariement entre deux fragments nucléotidiques. La stringence peut également être fonction des paramètres de la réaction, tels que la concentration et le type d'espèces ioniques présentes dans la solution d'hybridation, la nature et la concentration d'agents dénaturants et/ou la température d'hybridation. Toutes ces données sont bien connues et les conditions appropriées peuvent être déterminées par l'homme du métier. En général, selon la longueur des fragments nucléotidiques que l'on souhaite hybrider, la température d'hybridation est comprise entre environ 20 et 70°C, en particulier entre 35 et 65°C dans une solution saline à une concentration d'environ 0,5 à 1 M. Les partenaires de liaison spécifiques ou non de l'ARNm sont tout partenaire susceptible de se lier à cet ARNm. A titre d'exemple, on peut citer les sondes nucléiques, les amorces d'amplification, et toute autre molécule capable de se lier à cet ARNm.

Par sonde d'hybridation, on entend un fragment nucléotidique comprenant de 5 à 100 motifs nucléiques, notamment de 10 à 35 motifs nucléiques, possédant une spécificité d'hybridation dans des conditions déterminées pour former un complexe d'hybridation avec le matériel spécifique du gène cible d'intérêt. La sonde d'hybridation peut comprendre un marqueur permettant sa détection.

Au sens de la présente invention, on entend par amorce d'amplification, un fragment nucléotidique comprenant de 5 à 100 motifs nucléiques, préférentiellement de 15 à 30 motifs nucléiques permettant l'initiation d'une polymérisation enzymatique, telle que notamment une réaction d'amplification enzymatique. Par réaction d'amplification enzymatique, on entend un processus générant de multiples copies d'un fragment nucléotidique par l'action d'au moins une enzyme. De telles réactions d'amplification sont bien connues de l'homme du métier et on peut citer notamment les techniques suivantes :
- PCR (Polymerase Chain Reaction), telle que décrite dans les brevets US 4,683,195, US 4,683,202 et US 4,800,159,
- NASBA (Nucleic Acid Sequence-Based Amplification) avec la demande de brevet WO 91/02818, et
- TMA (Transcription Mediated Amplification) avec le brevet US 5,399,491.

Par détection, on entend soit une méthode physique, soit une méthode chimique avec un agent colorant intercalant tel que SYBR® Green I ou le bromure d'éthydium, soit une méthode de détection à l'aide d'un marqueur. De nombreuses méthodes de détection existent pour la détection des acides nucléiques⁷². Les marqueurs appropriés sont tels que définis précédemment.

Au sens de la présente invention, la sonde d'hybridation peut être une sonde dite de détection. Dans ce cas, la sonde dite de détection est marquée au moyen d'un marqueur tel que défini précédemment. Grâce à la présence de ce marqueur, on peut détecter la présence d'une réaction d'hybridation entre une sonde de détection donnée et le transcrit à détecter.

La sonde de détection peut être notamment une sonde de détection « molecular beacons »⁷³. Ces « molecular beacons » deviennent fluorescentes lors de l'hybridation. Elles possèdent une structure de type tige-boucle et contiennent un fluorophore et un groupe « quencher ». La fixation de la séquence de boucle spécifique avec sa séquence complémentaire d'acide nucléique cible provoque un déroulement de la tige et l'émission d'un signal fluorescent lors de l'excitation à la longueur d'onde qui convient.

La sonde d'hybridation peut être également une sonde dite de capture. Dans ce cas, la sonde dite de capture est immobilisée ou immobilisable sur un support solide par tout moyen approprié, c'est-à-dire directement ou indirectement, par exemple par covalence ou adsorption. Les supports solides appropriés sont connus de l'homme du métier et on peut citer à titre d'exemples les matériaux de synthèse ou les matériaux naturels, les latex, les particules magnétiques, les dérivés métalliques, les gels, etc. Le support solide peut être sous la forme d'une plaque de microtitration, d'une membrane comme décrit dans la demande WO-A-94/12670, d'une particule. On peut également immobiliser sur le support plusieurs sondes de capture différentes, chacune étant spécifique d'un transcrit cible. En particulier, on peut utiliser comme support une biopuce sur laquelle peuvent être immobilisées un grand nombre de sondes.

L'immobilisation des sondes sur le support est également connue de l'homme du métier et on peut citer un dépôt de sondes par transfert direct, la micro-déposition, la synthèse *in situ* et la photolithographie.

La mise en évidence, dans l'échantillon biologique, des modifications ou anomalies d'ADN au niveau du gène codant pour le marqueur tumoral d'intérêt peut être réalisée par tout procédé de détermination des altérations de l'ADN dans un échantillon, à savoir soit la détection directe de mutations, soit la mise en évidence d'altérations dans le profil de méthylation des loci d'intérêt, ou tout autre procédé de détermination d'altérations de l'ADN dans un échantillon, connu de l'homme du métier.

Les mutations peuvent inclure des substitutions ponctuelles d'un nucléotide par un autre, des délétions de un ou plusieurs nucléotides et des insertions de un ou plusieurs nucléotides. Les mutations peuvent être situées dans la partie codant du gène du marqueur tumoral d'intérêt, ou dans les parties 5' et 3' non codant comme la région promotrice ou la région terminatrice de transcription.

Les stratégies de mise en évidence de mutations s'appuient sur les techniques de la biologie moléculaire et comprennent des étapes d'extraction d'ADN, d'amplification par PCR ou autre technique d'amplification, d'hybridation et/ou de séquençage. Dans le cas du cancer colorectal, le procédé suivant a été utilisé avec succès pour réaliser la détection de mutations dans l'ADN des selles : concentration de l'ADN par précipitation, enrichissement en cible en utilisant des oligonucléotides de capture sur billes magnétiques, amplification PCR des gènes d'intérêt, séquençage en phase solide pour identifier les mutations ponctuelles⁷⁴. Les délétions ont été identifiées par rapport à la différence de taille entre le fragment de référence attendu et le fragment muté. Imperiale et al.⁷⁴ ont décrit un panel de 21 mutations situées dans les gènes K*-ras,* APC, et p53 qui permet de détecter 16/31 des cancers invasifs.

D'autres marqueurs ADN utilisés sont la délétion BAT-26, qui est un marqueur d'instabilité des microsatellites et l'ADN hautement amplifiable appelé long ADN (L-ADN), qui n'est pas un marqueur spécifique mais qui semble refléter l'apoptose désordonné des cellules tumorales exfoliées dans le lumen colique⁷⁵. Ces marqueurs ne sont satisfaisants ni par rapport à leur sensibilité, ni par rapport à leur spécificité.

Comme indiqué précédemment, les altérations de l'ADN peuvent aussi correspondre à une modification du profil de méthylation du gène correspondant au marqueur tumoral d'intérêt. La modification du profil de méthylation peut correspondre à une hypométhylation (diminution du nombre de méthylations) ou à une hyperméthylation (augmentation du nombre de méthylations). Les motifs altérés peuvent être situés dans la partie codant du gène du marqueur tumoral d'intérêt, ou dans les parties 5' et 3' non codant comme la région promotrice ou la région terminatrice de transcription.

L'analyse de la méthylation de l'ADN peut être effectuée en utilisant des techniques basées sur la PCR qualitative et/ou quantitative comme la MSP (methylation-specific PCR), le séquençage bisulfite, la digestion par une enzyme de restriction sensible aux méthylations couplée avec la PCR, COBRA (combined bisulfite restriction analysis) et Ms-SNuPE (methylation-sensitive single nucleotide primer extension). L'ensemble de ces techniques a été revu en détail et de façon comparée dans un article de méthodologie⁷⁶.

Dans la littérature plusieurs gènes hyperméthylés en cas de cancer colorectal ont été rapportés. A titre d'exemple on peut citer le gène ALX4 (Aristaless-like homeobox-4)⁶⁰, la région promotrice du gène TPEF/HHP1 (transmembrane protein containing epidermel growth factor and follistatin domain)⁷⁷ ou encore le gène Septin-9⁷⁸.

Lorsque, dans le procédé de l'invention, on détecte au moins deux marqueurs, ils peuvent être mis en évidence de façon séparée, par exemple à l'aide de dosages immunoessais différents, ou bien de façon simultanée, en dosage multiplex.

Lorsque, dans le procédé de l'invention, on détecte deux marqueurs de nature différente, par exemple un marqueur protéique et un marqueur ARNm, on peut utiliser deux procédés de détection différents, choisis parmi ceux décrits précédemment. On peut également les détecter simultanément, dans le même milieu de détection et dans les mêmes conditions réactionnelles, comme décrit dans la demande de brevet WO03/104490. Les étapes du procédé de détection décrit dans cette demande de brevet, qui consiste à détecter simultanément des réactions d'hybridation et immunologiques dans un échantillon susceptible de contenir des analytes cibles constitués d'au moins un acide nucléique et d'au moins un autre ligand de nature différente, consistent à :
(i) déposer une quantité connue en volume de l'échantillon dilué dans un tampon de réaction, sur une surface de capture préalablement revêtue des partenaires de capture desdits analytes cibles, lesdits partenaires de capture consistant en au moins une sonde nucléique et au moins un anti-ligand,
(ii) mettre à réagir à une température comprise entre 15°C et 60°C et
(iii) visualiser les réactions d'hybridation et immunologiques ainsi obtenues.

L'échantillon biologique peut nécessiter un traitement particulier car il peut contenir le ou les marqueurs tumoraux recherchés dans le procédé de l'invention en tant que tels, ou bien il peut contenir des cellules tumorales circulantes qui contiennent les marqueurs recherchés dans le procédé de l'invention et/ou des cellules tumorales circulantes qui sont capables de sécréter le ou les marqueurs recherchés dans le procédé de l'invention.

Ainsi, selon un mode de réalisation de l'invention, l'échantillon biologique est préalablement traité pour isoler les cellules tumorales circulantes contenues dans le dit fluide.

Par isoler les cellules tumorales circulantes, on entend obtenir une fraction cellulaire enrichie en cellules tumorales circulantes.

Le traitement de l'échantillon biologique pour isoler les cellules tumorales circulantes peut être effectué par tri cellulaire dans un cytomètre de flux, par enrichissement sur Ficoll, par enrichissement par billes magnétiques recouvertes d'anticorps spécifiques, ou par toute autre méthode d'enrichissement spécifique connue de l'homme du métier.

Dans le cas du sang à titre d'échantillon biologique, les cellules tumorales circulantes peuvent être isolées grâce à une technique de séparation cellulaire sur Ficoll associée à une déplétion des cellules sanguines utilisant des anticorps anti-CD45 couplés à des billes magnétiques (Dynal Biotech ASA, Norvège).

La détection du ou des marqueurs tumoraux recherchés dans le procédé de l'invention peut alors être effectuée directement à partir de cellules tumorales circulantes isolées de l'échantillon biologique, par exemple par marquage immunocytochimique de ces cellules avec un anticorps anti-marqueur(s) tumoral(aux) recherché(s) dans le procédé de l'invention, après avoir déposé les cellules tumorales circulantes sur une lame par cytospin. La détection du ou des marqueurs tumoraux recherchés dans le procédé de l'invention peut également être effectuée directement dans les cellules tumorales circulantes en utilisant la méthode de cytométrie de flux telle que décrite dans Métézeau et al.⁷⁹.

Dans ces conditions, lesdites cellules circulantes peuvent être traitées dans des conditions permettant le blocage du ou des marqueurs tumoraux recherchés dans le procédé de l'invention à l'intérieur desdites cellules. Un tel traitement est décrit par exemple dans Mathieu et al.⁸⁰.

La détection du ou des marqueurs tumoraux recherchés dans le procédé de l'invention se fait alors après avoir rendu perméable la membrane des cellules pour faire rentrer les partenaires de liaison spécifique du ou des marqueurs recherchés dans le procédé de l'invention.

La détection directe du ou des marqueurs tumoraux utilisés dans le procédé de l'invention à partir des cellules circulantes peut également être effectuée à l'aide d'un procédé ELISPOT, par exemple à l'aide du procédé décrit dans la demande de brevet WO03/076942 déposée par la Demanderesse. Ce procédé est un procédé de détection et/ou quantification de cellules tumorales circulantes d'un échantillon biologique, lesquelles sont capables de relarguer ou sécréter *in vitro* un ou plusieurs marqueurs tumoraux, comprenant les étapes consistant à :
(i) déposer une quantité desdites cellules au fond d'une surface de culture sur laquelle est fixé au moins un partenaire de liaison spécifique dudit ou desdits marqueurs tumoraux,
(ii) cultiver lesdites cellules en conditions telles qu'elles relarguent ou sécrètent lesdits marqueurs tumoraux qui sont immunocapturés au fond de la surface de culture,
(iii) éliminer les cellules par lavage,
(iv) ajouter au moins un conjugué marqué spécifique desdits marqueurs tumoraux et
(v) visualiser le marquage ainsi obtenu.

La détection directe du ou des marqueurs tumoraux utilisés dans le procédé de l'invention dans les cellules tumorales peut encore être effectuée dans le milieu de culture desdites cellules après les avoir cultivées dans des conditions telles qu'elles sécrètent du ou des marqueurs tumoraux utilisés dans le procédé de l'invention.

Les conditions de culture pour le relarguage ou l'expression des marqueurs tumoraux sont des conditions classiques telles que 37°C sous atmosphère humide et à 5% de CO₂.

Lorsque l'échantillon biologique est un échantillon solide, la présence du ou des marqueurs tumoraux peut également être montrée *in vivo, in situ* dans les tumeurs.

Pour montrer la présence d'un marqueur tumoral au sein d'une tumeur *in vivo,* on peut utiliser toute méthode d'imagerie connue de l'homme du métier. Pour cela, on peut coupler un partenaire de liaison dudit marqueur tumoral à un traceur d'imagerie. Par couplage des partenaires de liaison à un traceur d'imagerie, on entend la fixation d'un traceur capable d'être détecté par toute méthode d'imagerie connue de l'homme du métier, et de générer directement ou indirectement un signal détectable. Ainsi, le traceur peut être un traceur radioactif comme le technétium-99. Dans ce cas, l'organe atteint du cancer primitif ou des métastases va fixer le marqueur tumoral et son traceur. Le rayonnement émis par l'organe peut être filmé par une caméra spéciale, par exemple une gamma-caméra. L'appareil recueille les scintillations générées par la substance radioactive et permet ainsi de visualiser l'organe.

Dans un autre procédé de l'invention, le traceur peut comprendre un corps radioactif émettant des positrons (Fluor 18). Les images seront ensuite acquises par un système de Tomographie par Émission de Positrons.

Dans un autre procédé préféré de l'invention, le partenaire du ou des marqueurs tumoraux peut être couplé à des nanoparticules. A titre d'exemple, il peut s'agir de nanoparticules supramagnétiques. Par exemple des nanoparticules magnétiques anioniques pour l'application au marquage cellulaire direct et la détection *in vivo* par imagerie par résonance magnétique nucléaire. Il peut également s'agir de nanoparticules d'or.

Grâce aux procédés de l'invention permettant la détection du marqueur tumoral *in vivo,* on pourra visualiser les zones de l'organisme où il y a eu fixation du partenaire de liaison du marqueur tumoral, les cancers produisant du marqueur tumoral, et en particulier le cancer colorectal, ainsi que les localisations de leurs métastases à distance et les atteintes ganglionnaires.

Le procédé de l'invention peut être utilisé tant pour le diagnostic précoce, que pour le dépistage, le suivi thérapeutique, le pronostic et le diagnostic des rechutes dans le cadre du cancer colorectal puisque seules les cellules cancéreuses sécrètent de la Prodéfensine-A6 et que cette production est fonction du grade du cancer, ce qui constitue un autre objet de l'invention.

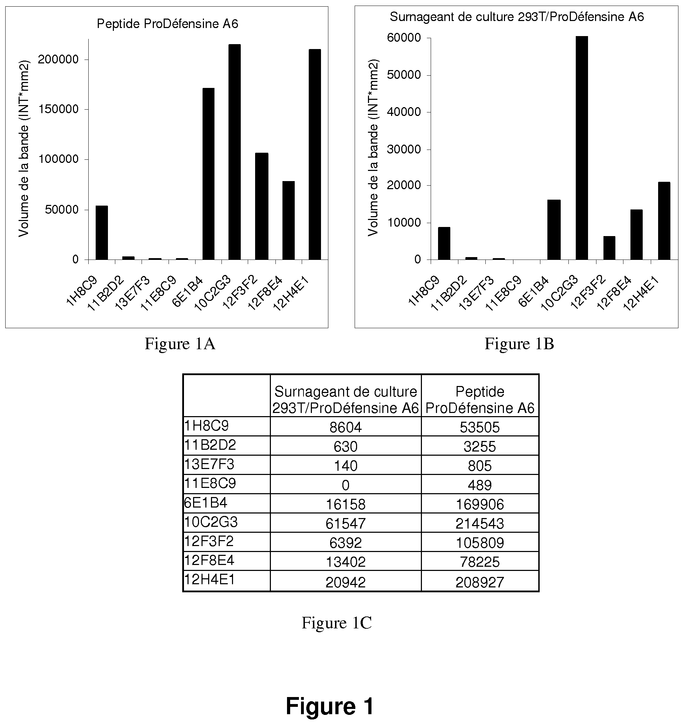

L'invention sera mieux comprise à l'aide des exemples suivants donnés à titre illustratif et non limitatif, ainsi qu'à l'aide des figures 1 à 6 annexées, sur lesquelles :
- la figure 1 représente la comparaison par la technique du Western blot des 9 anticorps monoclonaux anti-ProDéfensine-A6. Les graphiques montrent les signaux correspondant au volume de la bande (en intensité*mm2) pour le peptide ProDéfensine-A6 (SEQ ID°1, 7ng/puits) (Figure 1A) et pour la protéine ProDéfensine-A6 sécrétée dans les surnageants de culture 293T transfectées (Figure 1B). Les valeurs exactes des volumes de bande sont indiquées dans le tableau (Figure 1C) ;

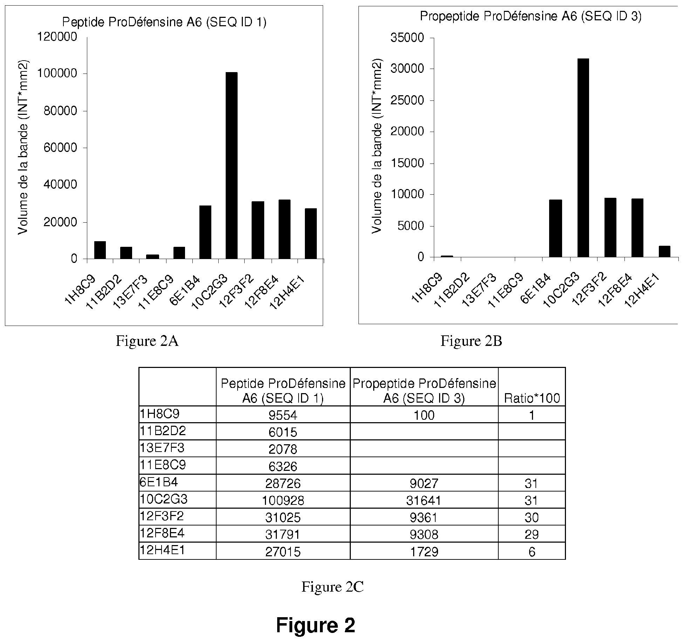

- la figure 2 représente la comparaison par la technique du dot-blot des 9 anticorps monoclonaux anti-ProDéfensine-A6. Les graphiques montrent les signaux correspondant au volume moyen de 2 spots (en intensité*mm2) pour le peptide ProDéfensine-A6 (SEQ ID°1) (Figure 2A) et pour le propeptide ProDéfensine-A6 (SEQ ID°3) (Figure 2B). Les valeurs exactes des volumes de bande sont indiquées dans le tableau (Figure 2C). Le ratio* 100 donné dans ce tableau est calculé selon la formule : signal propeptide ProDéfensine A6/signal peptide ProDéfensine-A6* 100 ;

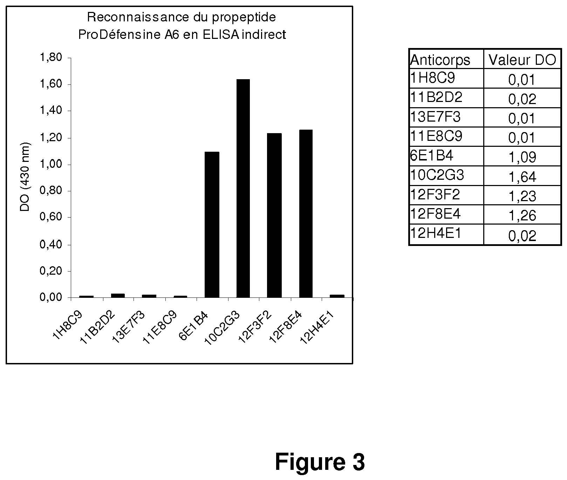

- la figure 3 est un graphe relatif à l'analyse de la reconnaissance du propeptide ProDéfensine-A6 par la technique de l'ELISA indirect. Les valeurs exactes du signal ELISA en unité d'absorbance sont indiquées dans le tableau à côté du graphe ;
- la figure 4 récapitule les séquences reconnues par chacun des anticorps anti-ProDéfensine-A6 1H8C9, 11B2D2, 11E8C9, 13E7F3. La séquence en acides aminés de chaque motif porté par banque de phage et se fixant sur l'anticorps étudié est donné dans la case « motifs immunoréactifs ». Pour chaque anticorps ces motifs immunoréactifs sont alignés afin de déterminer la séquence consensus qui est indiquée en gras ;

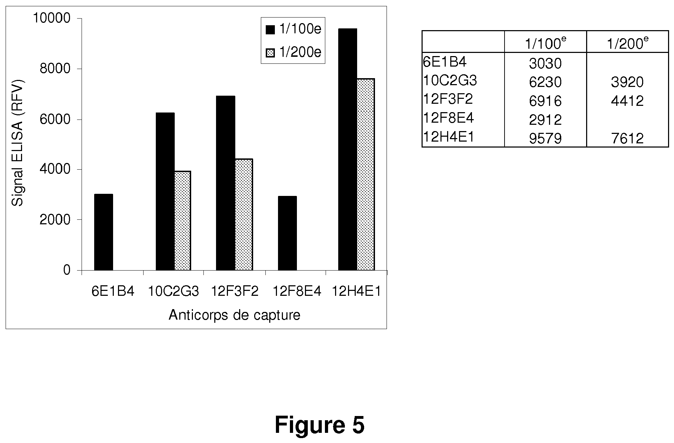

- la figure 5 représente l'analyse de la réactivité par ELISA sandwich des 5 anticorps monoclonaux anti-ProDéfensine-A6 dirigés contre l'épitope 1 avec l'anticorps de détection 1H8C9. L'antigène utilisé est le surnageant de culture 293T transfectées contenant de la ProDéfensine-A6 sécrétée. Ce surnageant a été dilué au 1/100 ou 1/200. Les valeurs exactes du signal ELISA en RFV sont indiquées dans le tableau à côté du graphe ;

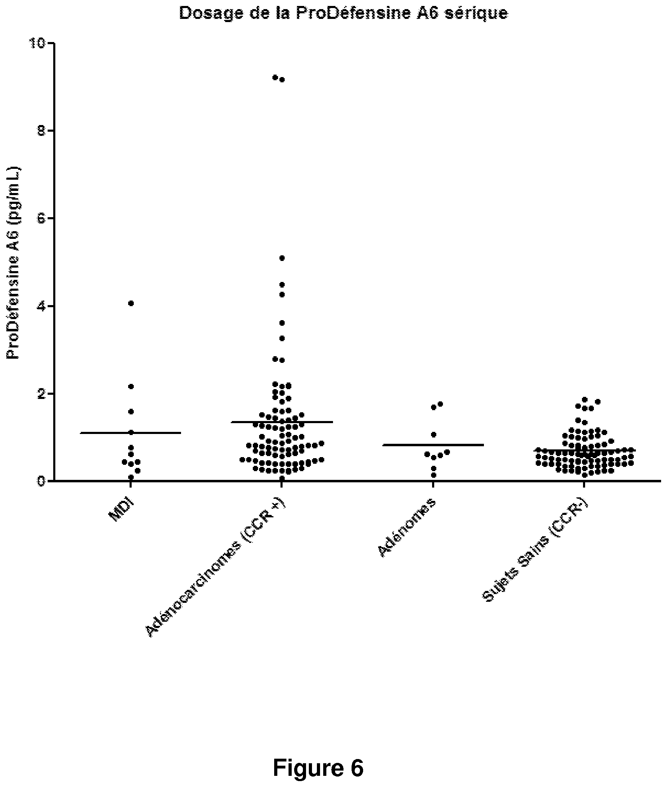

- la figure 6 est un graphe relatif au dosage par ELISA de la ProDéfensine-A6, en pg/mL, dans le sérum de patients présentant un adénocarcinome colorectal (CCR+), de sujets sains (CCR-), de patients ayant une maladie digestive inflammatoire (MDI) et de patients présentant un adénome colorectal.

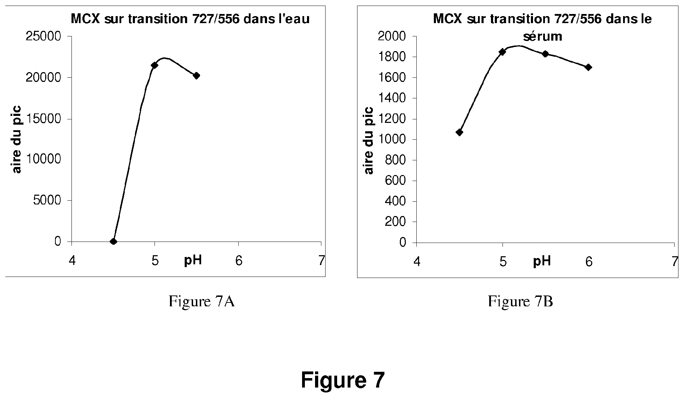

- la figure 7 représente l'optimisation du fractionnement SPE sur cartouches MCX pour le peptide EPLQAEDDPLQAK (SEQ ID N°30) de la ProDéfensine A6. Les graphes représentent l'aire du pic correspondant à la transition 727/556 du peptide en fonction du pH du tampon utilisé pour effectuer le fractionnement sur cartouche MCX. Figure 7A : la ProDéfensine-A6 mise en solution dans l'eau ; Figure 7B : la ProDéfensine-A6 mise en solution dans du sérum humain de sujets sains.

### Exemple 1 : Clonage des gènes codant pour les marqueurs tumoraux et expression des protéines recombinantes

Pour les marqueurs tumoraux Aminoacylase-1, LEI, L-FABP, Ezrine, Plastine-I, Gal-3, Villine, I-FABP et Calréticuline, l'amplification de l'ADNc et clonage, la construction des vecteurs d'expression ainsi que l'expression et purification des protéines recombinantes ont été décrits en détail dans la demande de brevet WO2009/024691.

### 1. Expression de la protéine Prodéfensine-A6 en cellules humaines par transfection

Un vecteur d'expression contenant l'ADNc du gène Prodéfensine A6 (pCMV6-XL5 DEFA6) a été acheté auprès de la société Origene (Cat. No SC303095). Ce vecteur, permet d'exprimer la protéine Prodéfensine-A6 sous le contrôle du promoteur CMV après introduction dans des cellules de mammifères.

Les cellules humaines de rein embryonnaire HEK 293T ont été maintenues en culture dans du milieu DMEM contenant 10% SVF, à 37°C avec 5% CO₂. Les tapis cellulaires entre 50% et 70% de confluence ont été transfectés avec le plasmide d'expression pCMV6-XL5 DEFA6 en utilisant l'un des deux réactifs de transfection suivants : la Lipofectamine LTX commercialisée par Invitrogen (Cat No. 15338-100) ou TransIT LT-1 (Cat No. MIR2300) commercialisée par Euromedex. Dans les 2 cas, les transfections ont été réalisées selon le mode opératoire fourni par les fabricants du réactif. Après transfection, les cultures ont été incubées pendant 48h pour permettre la production de la protéine ProDéfensine-A6. Ensuite, le surnageant de culture est récolté, centrifugé puis filtré pour enlever les débris cellulaires. La protéine ProDéfensine sécrétée dans le surnageant de culture a subi toutes les modifications post-traductionnelles nécessaires à son repliement, elle est native. C'est ce surnageant qui a été utilisé comme source de protéine ProDéfensine-A6 lors de criblage des anticorps monoclonaux anti-ProDéfensine-A6 et lors de la caractérisation de ces anticorps.

### 2. Synthèse chimique de peptides

Trois peptides correspondants à différentes parties de la protéine Prodéfensine-A6 ont été obtenus par synthèse chimique selon les procédures bien connus de l'homme du métier (NeoMPS). Le peptide dont la séquence est indiquée en SEQ ID 1 correspond à la totalité de la séquence du ProDéfensine-A6 sans le peptide signal qui est clivé lors de la translocation de la chaîne polypeptidique vers le réticulum endoplasmique. Ce peptide est appelé peptide ProDéfensine-A6 ou précurseur ProDéfensine-A6. Le peptide dont la séquence est indiquée en SEQ ID 2 correspond à la totalité de la partie propeptide du précurseur Prodéfensine-A6. Ce peptide est appelé propeptide ProDéfensine-A6 ou partie propeptide de la ProDéfensine-A6. Le peptide dont la séquence est indiquée en SEQ ID 3 correspond à la quasi totalité de la partie propeptide du précurseur Prodéfensine-A6. Quatre acides aminés de l'extrémité N-terminale ne sont pas inclus dans la séquence. Ce peptide est plus facile à produire en grande quantité que le peptide de séquence SED ID°2 et a été utilisé en remplacement lorsque la présence des quatre acides aminés n'est pas critique.
SEQ ID 2 : EPLQAEDDPLQAKAYEADAQEQRGANDQDFAVSFAEDASSSLRALGS
SEQ ID 3 : AEDDPLQAKAYEADAQEQRGANDQDFAVSFAEDASSSLRALGS

### Exemple 2 : Obtention d'anticorps monoclonaux dirigés contre les marqueurs tumoraux

### 1. Modèle animal

Les expériences d'immunisation ont été réalisées chez des souris BALB/c (H-2^{d}) femelles âgées de 6 à 8 semaines au moment de la première immunisation.

### 2. Immunogènes et immunisations

Afin d'augmenter les réponses immunes obtenues chez les souris et pouvoir générer des anticorps monoclonaux, les marqueurs tumoraux ont été produits sous forme de protéines recombinantes ou de peptides de synthèse produites selon les modes opératoires décrits dans l'exemple 1. La protéine LDH a été obtenue auprès de la société SciPac (Cat. No. 103-133). Lorsque les peptides de synthèse ont été utilisés en tant qu'immunogène, ils ont été couplés sur des protéines porteuses comme l'albumine sérique bovine (BSA) ou la KLH (Keyhole Limpet Hemocyanine). Ces protéines ont été mélangées volume pour volume avec l'adjuvant de Freund (Sigma), préparé sous forme d'émulsion eau-dans-huile et dont il est connu qu'il présente un bon pouvoir immunogène. Pour chaque marqueur tumoral, 3 souris ont été immunisées. Les souris ont reçu 3 doses successives de 10 µg des immunogènes à 0, 2 et 4 semaines. Toutes les injections ont été réalisées par voie sous-cutanée. La première injection est faite en mélange avec l'adjuvant de Freund complet, les deux suivantes se font en mélange avec l'adjuvant de Freund incomplet. Entre J50 et J70 après la première injection, les réponses humorales ont été restimulées avec une injection intraveineuse de 100 µg de la protéine recombinante. Pour la ProDéfensine-A6, deux différentes séries d'immunisation ont été réalisées. Un premier groupe de souris a reçu le peptide ProDéfensine A6 (SEQ ID 1) couplé à la BSA et à la KLH (en alternance selon les injections). Un second groupe de souris a reçu le propeptide ProDéfensine-A6 (SEQ ID 2) couplé à la BSA et à la KLH (en alternance selon les injections). Des anticorps anti-ProDéfensine-A6 ont été obtenus à partir des animaux appartenant à chacun des deux groupes.

### 3. Suivi de l'apparition de la réponse humorale

Afin de suivre l'apparition des anticorps, on effectue régulièrement sur les souris des prélèvements de sang. La présence des anticorps anti-marqueur tumoral est testée en utilisant un ELISA. La protéine d'intérêt est utilisée en capture (1 µg/puits), après saturation on fait réagir avec l'antigène différentes dilutions des sérums à tester (incubation à 37°C, pendant 1h). Les anticorps spécifiques présents dans le sérum sont révélés par un anticorps de chèvre anti-IgG de souris AffiniPure conjugué à la phosphatase alcaline (H+L, Jackson Immunoresearch, Cat no. 115-055-146), qui se lie aux anticorps recherchés (0,1 µg/puits).

### 4. Obtention d'anticorps monoclonaux

Trois jours après la dernière injection, pour chaque marqueur tumoral, une des souris immunisée a été sacrifiée ; le sang et la rate ont été prélevés. Les splénocytes obtenues à partir de la rate ont été mises en culture avec les cellules de myélome Sp2/0-Ag14 pour qu'elles fusionnent et s'immortalisent, selon le protocole décrit par Köhler et Milstein^{81,82}. Après une période d'incubation de 12-14 jours les surnageants des hybridomes obtenus ont été criblés pour déterminer la présence d'anticorps anti-marqueur tumoral en utilisant le test ELISA décrit dans le point 3 de cet exemple. Lorsque des peptides de synthèse couplés sur la BSA ou la KLH ont été utilisés comme immunogène, les clones dirigés contre la BSA et la KLH sont éliminés en effectuant un criblage ELISA avec en capture la BSA ou la KLH non couplée. Les colonies d'hybridome positives ont été sous-clonées deux fois selon la technique de la dilution limite, bien connue par l'homme du métier.

### 5. Caractérisation des anticorps monoclonaux par immunoblot

Les anticorps monoclonaux dirigés contre les marqueurs tumoraux Aminoacylase-1, LEI, Ezrine, Plastine-I, Gal-3, Calréticuline et LDH sont décrits dans la demande de brevet WO2009/024691.

Les anticorps monoclonaux 1H8C9, 11B2D2, 13E7F3, 11E8C9, 6E1B4, 10C2G3, 12F3F2, 12F8E4, 12H4E1 sont dirigés contre la ProDéfensine-A6 et ont été obtenus en mettant en oeuvre les techniques décrites dans les points 1 à 4 de cet exemple.

### 5.1. Méthodologie

Les extraits de culture cellulaires de lignées 293T transfectées sont préparés en lysant directement le culot cellulaire par 600 µl de PBS contenant du Triton X-100 0,5% et des inhibiteurs de protéases, puis traités selon le protocole de préparation d'échantillon des gels NuPAGE Novex (Invitrogen). Pour obtenir les extraits de tissu, les biopsies tumeur et muqueuse du patient CLSP105 ont été dissociées au scalpel, puis ont subi 10 cycles d'extraction dans le système Medimachine (Becton Dickinson) en utilisant des Medicons 50 µm avec 1 ml de tampon PBS, 2,5 mM EDTA, inhibiteurs des protéases (pastilles Roche complete™). Ces 10 ml de suspension cellulaire sont poolés, complétés à 25 ml puis centrifugés 15 min à 600*g*. Le surnageant correspond à l'extrait de tissu qui est traité selon le protocole de préparation d'échantillon des gels NuPAGE Novex. On utilise des échantillons réduits, à une concentration finale en protéine totale de 0,4 mg/ml. Le volume de dépôt est de 20 µl par puits, sur un gel NuPAGE Novex Bis-Tris 4-12%, tampon de migration MES. Après migration (sous 200V, pendant 1 heure) et transfert sur membrane de PVDF (sous 400 mA, pendant 45 min), la qualité du transfert est appréciée par une coloration à l'amidoblack.

Les membranes sont saturées par 5% de lait écrémé (Régilait) dans une solution de TNT (Tris 15 mM, NaCl 0,14M, Tween 20 0,5% pH8) à température ambiante pendant 1 heure. Après saturation, les membranes sont incubées pendant 1 heure avec les différents anticorps à tester dilués à 10 µg/ml dans la solution de saturation. Après rinçages au TNT, les membranes sont incubées 1 heure à température ambiante avec un conjugué anti-souris-peroxydase de raifort dilué au 1 : 5000, (Cat No. 115-035-062, Jackson Immunoresearch) dans la solution de saturation. Après rinçage, la révélation est réalisée avec le kit Substrat Supersignal West Dura Extended (Cat No. 34076, Pierce) suivant les données d'utilisation recommandées. Le temps d'exposition a été de 2 minutes pour l'expérience présentée en Figure 1 et de 100 secondes pour l'expérience présentée dans le Tableau 1.

Le signal de chemiluminescence sur les membranes a été mesuré avec le système d'imagerie VersaDoc 5000 de BioRad. A partir de l'image du Western blot, les volumes des bandes qui correspondent aux différents marqueurs tumoraux ont été évalués avec le logiciel QuantityOne (Bio-Rad). Le volume correspond à l'intensité du signal de chemiluminescence multipliée par la surface de la bande.

### 5.2. Résultats

Les résultats de Western blot récapitulés dans la Figure 1 et le Tableau 1 donnent le volume des bandes correspondant au marqueur tumoral d'intérêt sur les analyses par Western blot, en fonction des différents échantillons testés. Le surnageant de culture 293T transfectées par le plasmide pCMX-XL5-ProDéfensine-A6 contenant de la ProDéfensine-A6 sécrétée et le peptide ProDéfensine-A6 (7 ng par puits) ont été utilisés pour évaluer la réactivité en Western blot des neuf anticorps anti-ProDéfensine A6 (Figure 1). Tous les anticorps reconnaissent le peptide ProDéfensine A6. Tous les anticorps, sauf le monoclonal 11E8C9, reconnaissent la ProDéfensine-A6 sécrétée dans le surnageant de culture dans les conditions expérimentales utilisées. Cependant l'intensité des signaux obtenus est très disparate, montrant que tous les anticorps n'ont pas la même réactivité. Les résultats présentés dans le Tableau 1 montrent que les marqueurs tumoraux testés sont bien exprimés dans le tissu tumoral obtenu à partir des patients. La Prodéfensine-A6 n'est pas exprimée par les lignées cellulaires de côlon Caco-2 ou HT-29, pour cette raison ces lysats n'ont pas été inclus dans les analyses.

L'intensité du signal obtenu avec un anticorps sur un échantillon peut être comparée aux signaux obtenus avec les autres échantillons et le même anticorps. La technique utilisée permet de confirmer la présence ou l'absence du marqueur dans le tissu (échantillon non distant) et la spécificité des anticorps vis-à-vis des marqueurs. Cette technique n'a pas été mise en oeuvre dans cet exemple dans les échantillons distants car elle ne permettrait pas de conclure à la présence ou non du marqueur tumoral dans les échantillons distants, ni de déterminer si sa concentration sera augmentée ou diminuée dans ceux-ci. De plus, le schéma expérimental utilisé ne permet pas de comparer la réactivité d'un anticorps à l'autre.

**Tableau 1**

| Anticorps anti-ProDEFA6 | Surnageant de culture 293T/ProDEFA6 | Tissu muqueuse CLSP105 | Tissu tumeur CLSP105 |
|---|---|---|---|
| 1H8C9 | 2475 | Négatif | 457 |
| 11B2D2 | 556 | Négatif | 140 |
| 13E7F3 | 134 | Négatif | Négatif |
| 11E8C9 | Négatif | Négatif | Négatif |

### 6. Analyse de la reconnaissance du propeptide ProDéfensine A6 par les anticorps monoclonaux par dot-blot

### 6.1. Méthodologie

L'analyse par dot-blot a été réalisée en utilisant le peptide ProDéfensine-A6 (SEQ ID°1, concentration 0,1 mg/mL) et le propeptide ProDéfensine A6 (SEQ ID°3, concentration 2,38 mg/mL). Une goutte de chaque échantillon a été déposée en duplicat sur des membranes de nitrocellulose (Transblot transfer medium, Bio-rad) puis séchée à l'air libre.

Le protocole de révélation immunologique est identique à celui décrit dans le paragraphe 5.1 de cet exemple. Le temps d'exposition utilisé pour cette expérience a été de 1 minute.

### 6.2. Résultats

Tous les anticorps testés reconnaissent le peptide ProDéfensine-A6 (SEQ ID°1) en dot-blot (Figure 2). L'anticorps le plus réactif dans cette technique est le clone 10C2G3. Les anticorps 6E1B4, 12F3F2, 12F8E4, 12H4E1 forment un groupe homogène, moins réactifs que le 10C2G3 mais plus que le groupe 1H8C9, 11B2D2, 13E7F3, 11E8C9 qui est plus hétérogène. D'ailleurs les réactivités en Western blot et en dot-blot ne corrèlent pas toujours : les anticorps 6E1B4, 12F3F2, 12F8E4, 12H4E1 ont une réactivité similaire en dot-blot alors que les intensités des signaux obtenus en Western blot sont différentes (Figures 1 et 2). Le Western blot a été réalisé en conditions dénaturantes, alors que les peptides sont spottés directement sans dénaturation pour le dot-blot.

Seuls les anticorps 6E1B4, 10C2G3, 12F3F2, 12F8E4, 12H4E1 reconnaissent le propeptide ProDéfensine-A6 (SEQ ID°3) en dot-blot. Les anticorps 1H8C9, 11B2D2, 13E7F3, 11E8C9 ne montrent pas de réactivité significative avec le propeptide ProDéfensine-A6 (SEQ ID°3), indiquant que leur épitope est situé dans la partie Défensine-A6 mature du précurseur ProDéfensine-A6. Parmi les 5 anticorps qui réagissent avec le propeptide ProDéfensine-A6 (SEQ ID°3), dont l'épitope minimal est donc contenu dans cette séquence, les clones 6E1B4, 10C2G3, 12F3F2 et 12F8E4 présentent tous le même profil de réactivité : le ratio des signaux obtenus (propeptide ProDéfensine-A6/ peptide ProDéfensine-A6* 100, Figure 2) est entre 29 et 31 pour tous ces anticorps. Le clone 12H4E1 se distingue de ces autres anticorps anti-propeptide ProDéfensine-A6 parce que ce ratio est à 6. Il reconnaît le propeptide moins bien que les 4 autres anticorps.

### 7. Analyse de la reconnaissance du propeptide ProDéfensine-A6 par les anticorps monoclonaux par ELISA indirect

### 7.1. Méthodologie

Des plaques 96 puits (type Nunc, Maxisorp) ont été coatées avec le propeptide ProDéfensine-A6 (SEQ ID°3) dilué en PBS à 2 µg par puits, sur la nuit à température ambiante. Les plaques sont saturées par 10% de lait dans du PBS-Tween 20 0,05% (PBS-T) pendant 1h à 37°C. On effectue 3 lavages en PBS-T, on dépose sur les plaques 0,2 µg/puits de l'anticorps biotinylé à tester dilué en PBS-T 1% BSA et on incube 1h à 37°C. Après 3 lavages PBS-T, on rajoute la streptavidine couplée à la peroxydase de raifort (Jackson Immunoresearch Cat. No. 016-030-084, dilution 1/20000 en PBS-T 1% BSA, 100 µl/puits). Après 1h d'incubation à 37°C et 3 lavages PBS-T, on ajoute le substrat OPT EIA (BD), 100 µl/puits. Au bout de 20 min, lorsque le développement de la coloration a lieu, on stoppe la réaction par de l'acide sulfurique 5N et on mesure l'absorbance à 450 nm. Les résultats présentés sont la moyenne de 2 mesures, le bruit de fond moyen (0,02 unités de densité optique) n'a pas été soustrait.

### 7.2. Résultats

Les résultats du test ELISA indirect sont présentés en Figure 3. Dans les conditions expérimentales utilisées, seuls les 4 anticorps 6E1B4, 10C2G3, 12F3F2 et 12F8E4 se fixent sur le propeptide proDéfensine-A6 adsorbé sur la phase solide. Les 5 autres anticorps, y compris le 12H4E1, ne se fixent pas sur le propeptide dans ce format. Cette expérience confirme les résultats de dot-blot (Figure 2) et établit définitivement que même si le monoclonal 12H4E1 reconnaît le propeptide proDéfensine-A6, sa réactivité est bien moindre en comparaison des 4 autres monoclonaux 6E1B4, 10C2G3, 12F3F2 et 12F8E4.

### 8. Caractérisation des épitopes reconnus par les anticorps monoclonaux en utilisant les techniques du Spotscan et criblage de banque de peptides portés par des phages

### 8.1. Méthodologie

La technique du Spotscan, adaptée d'après Frank et Döring⁷⁸, permet de synthétiser de façon simultanée un grand nombre de peptides fixés sur membrane de cellulose. Ces peptides reproduisent la séquence de l'antigène cible sous forme de peptides de 8 à 12 acides aminés, se chevauchant de 1 à 4 résidus. Ces peptides sont ensuite mis en contact avec l'anticorps à étudier dans un test colorimétrique de type Blot, et l'identification des peptides immunoréactifs permet de déduire la séquence minimale de l'épitope de l'anticorps et de le localiser précisément sur l'antigène.

La synthèse s'effectue sur une membrane de cellulose portant uniformément des bras en polyéthylène glycol (PEG) d'une longueur de 8 à 10 unités, présentant une fonction NH₂ libre en bout de chaîne. Elle se déroule de l'extrémité C-terminale vers l'extrémité N-terminale des peptides. Les acides aminés ont leur fonction aminée protégée par un groupement Fmoc (9-fluoréméthyloxycarbonyl), et leurs chaînes latérales, susceptibles de réagir au cours de la synthèse, sont également protégées par des groupements trityl, t-butyl ou t-butyl-éther. Les solutions-mères d'acides aminés sont préparées à une concentration de 0,33 M dans du NMP (N-méthyl-pyrrolidone) contenant 0,5 M de HOBt (hydroxybenzotriazole). Le dépôt des acides aminés s'effectue à l'aide du robot ASP 222 (Abimed, Langenfeld, Allemagne), piloté par l'intermédiaire du logiciel AutoSpot XL. L'utilisation de ce robot permet de faire en simultané jusqu'à 4 membranes de 96 spots, soit 384 peptides.

Pour un cycle de couplage d'un acide aminé, le robot dépose 0,7 µl de la solution d'acide aminé activé extemporanément (un volume de solution de diisopropyl-carbodiimide 1,1 M dilué en NMP pour 3 volumes de solution-mère d'acide aminé) sur les membranes. Ce dépôt est répété une seconde fois, puis les membranes sont rincées en DMF (N,N-diméthylformamide). Les groupements NH₂ n'ayant pas réagi sont ensuite été acétylés par 4 à 6 incubations de 10 minutes dans une solution d'anhydride acétique 10 % en DMF, afin d'éviter l'apparition de peptides abortifs ou tronqués. Après 3 lavages de 2 minutes en DMF, les groupements Fmoc protégeant la fonction aminée des acides aminés sont clivés par une incubation de 5 minutes dans une solution de pipéridine 20 % en DMF. Après 4 lavages en DMF, les spots sont colorés à l'aide d'une solution de bleu de bromophénol 1 % en DMF, puis la membrane est rincée 3 fois en méthanol et séchée à l'air libre avant le cycle de couplage suivant.

Ce protocole est répété pour l'ajout de chaque nouvel acide aminé. Après le couplage du dernier acide aminé, les peptides sont acétylés afin de permettre le blocage de tous les groupements NH₂ libres, empêchant ainsi l'ajout d'un autre acide aminé. Puis les chaînes latérales de tous les peptides sont déprotégées par l'incubation des membranes dans un bain acide trifluoroacétique / dichlorométhane / triisobutylsilane (5 : 5 : 0,3) pendant 1 heure. Les membranes sont ensuite rincées 4 fois en dichlorométhane, 3 fois en DMF et 3 fois en méthanol avant d'être séchées à l'air libre et conservées à -20°C jusqu'à l'immunorévélation.

Pour immunorévéler les spots avec un anticorps monoclonal, les membranes sont d'abord rincées en méthanol, puis lavées en TBS (Tris-HCl 50 mM pH 8,0, NaCl 140 mM, KCl 3 mM) avant d'être incubées sur la nuit à température ambiante dans la solution de saturation (solution concentrée 10X à base de caséine (Western Blocking reagent, Roche) diluée en TBS-Tween 20 0,05% (TBS-T) et contenant 5% de saccharose). Après un lavage de 10 minutes en TBS-T, les membranes sont incubées pendant 1h30 à 37°C avec l'anticorps monoclonal dilué à 20 µg/ml en solution de saturation. Les membranes sont ensuite lavées 3 fois en TBS-T, puis sont incubées avec le conjugué anti-souris couplé à la phosphatase alcaline (Jackson Immunoresearch), dilué au 1/2000^{ème} en solution de saturation. Après 2 lavages de 10 minutes en TBS-T, puis 2 lavages en CBS (acide citrique 10 mM pH 7, NaCl 140 mM, KCl 3 mM), le révélateur, préparé extemporanément (5-bromo, 4-chloro, 3-indoyl, phosphate 600 µM, thiazolyl blue tetrazolium bromide 720 µM, et MgCl₂ 5 mM en CBS), est mis en contact avec la membrane pendant 30 à 45 minutes à l'obscurité. Les peptides immunoréactifs apparaissent en bleu-violet. Après 3 rinçages en eau distillée, les membranes sont scannées puis conservées dans l'eau jusqu'à la régénération.

La régénération permet d'éliminer les anticorps et les conjugués fixés sur les peptides, ce qui permet ainsi de réaliser un nouveau test d'immunoréactivité vis-à-vis d'un autre anticorps. Les membranes subissent une série de lavages de 10 minutes chacun : 1 lavage en eau distillée, 6 lavages en DMF, 3 lavages en tampon de régénération A (urée 8 M, SDS (sodium dodecyl sulfate) 35 mM, β-mercaptoethanol 0,1 %), 3 lavages en tampon de régénération B (eau distillée / éthanol / acide acétique 4 : 5 : 1), puis 2 lavages en méthanol. Les membranes sont ensuite séchées à l'air libre avant d'être stockées à -20°C.

La caractérisation des épitopes par criblage de banques de peptides portés par des phages a été réalisée en utilisant le kit commercial PhD12 Phage Display Peptide Library Kit (Cat. No. E#8110S) de New England Biolabs, en suivant les instructions fournies avec le kit, version 2.7 du protocole datant de novembre 2007.

### 8.2. Résultats

Le Tableau 2 récapitule les épitopes reconnus par cinq anticorps anti-propeptide ProDéfensine-A6 dont l'épitope a été analysé par la technique du Spotscan. Tous ces anticorps reconnaissent la même région du précurseur ProDéfensine-A6, située entre les acides aminés 25 et 30 selon la numérotation commençant à la méthionine initiale (épitope 1). Par contre la séquence exacte reconnue par les différents monoclonaux n'est pas identique. Ainsi, le clone 10C2G3 reconnaît une séquence minimale de 2 acides aminés (DP) alors que les clones 12H4E1 et 6E1B4 nécessitent une séquence minimale de 6 acides aminés (EDDPLQ) pour se fixer. Les résultats présentés en Figures 2 et 3 ont montré que c'est le clone 10C2G3 qui permet d'obtenir le signal le plus élevé en dot blot et ELISA indirect pour la reconnaissance des peptides ProDéfensine-A6. De façon surprenante, le clone 12H4E1 se distingue des autres anticorps dirigés contre l'épitope 1 de la Pro-Défensine-A6 (Figures 2 et 3) même si la séquence minimale reconnue est identique avec l'anticorps 6E1B4. Le clone 12H4E1 reconnaît mieux la séquence EDDPLQ (SEQ ID N°6) dans le contexte de la protéine précurseur totale (SEQ ID N°1) que dans le contexte du propeptide proDéfensine-A6 (SEQ ID N°3).

**Tableau 2**

| Anticorps | N° épitope | Séquence de l'épitope^{a} (SEQ ID N°) |
|---|---|---|
| 10C2G3 | 1 | DP (27-28) (SEQ ID N°4) |
| 12F3F2 | 1 | DPL (27-29) (SEQ ID N°5) |
| 12F8E4 | 1 | DPL (27-29) (SEQ ID N°5) |
| 12H4E1 | 1 | EDDPLQ (25-30) (SEQ ID N°6) |
| 6E1B4 | 1 | EDDPLQ (25-30) (SEQ ID N°6) |

| | | |
|---|---|---|
| ^{a}Séquence en acide aminé de la région de fixation de la ProDéfensine-A6 à l'anticorps testé. Les nombres entre parenthèses correspondent à la position de l'épitope sur la séquence en acides aminés de la ProDéfensine A6, la numérotation commençant à la méthionine initiale. | | |

Les épitopes reconnus par les anticorps 1H8C9, 11B2C2, 13E7F3 et 11E8C9 n'ont pas pu être déterminés par la technique du Spotscan, ce qui indique qu'ils ne sont pas linéaires. Le criblage des banques de peptides portés par des phages a permis de sélectionner 5, 3, 7 et 4 mimotopes (séquence linéaire mimant un épitope) qui réagissent respectivement avec les anticorps 1H8C9, 11B2C2, 11E8C9 et 13E7F3. Les séquences de ces mimotopes sont présentées à la Figure 4. Pour chaque anticorps les séquences des mimotopes reconnus ou immunoréactifs ont été alignées afin de déterminer une séquence consensus qui est indiquée en gras sur le Figure 4, qui représente la séquence minimale reconnue par l'anticorps. Il n'a pas été possible de retrouver une seule de ces séquences consensus dans la structure primaire (ou séquence peptidique) de la ProDéfensine-A6. Ainsi, ces séquences consensus correspondent à des résidus éparpillés sur la structure primaire de la protéine mais qui partagent une proximité dans sa structure tridimensionnelle afin de former un épitope conformationnel.

### 9. Détection de la ProDéfensine-A6 par ELISA sandwich

### 9.1. Méthodologie

La ProDéfensine-A6 a été détectée en immunoessai de type sandwich en utilisant par exemple l'automate d'ELISA Vidas® (bioMérieux). Ce type de test peut aussi être réalisé en microplaque, de façon automatisée ou manuelle. Pour ce faire, le test ELISA a été construit en utilisant les réactifs du kit Vidas® HBs Ag Ultra (bioMérieux, Cat. No. 30315). Les réactifs ont été utilisés tels que décrits dans la notice correspondante (réf. 11728 D - FR - 2005/05), modifiée ainsi :
1. Des cônes ont été sensibilisés avec les 9 anticorps de capture à tester à une concentration de 10 µg/ml.
2. Le contenu du deuxième puits de la cartouche HBs Ag Ultra a été remplacé par 300 µl d'anticorps de révélation à tester (1H8C9 et 11E8C9) couplés à la biotine, dilués à 1 µg/ml dans le tampon (sans sérum de chèvre) du deuxième puits du kit Vidas® HBs Ag Ultra.
3. Le surnageant de culture 293T transfectées par le plasmide pCMX-XL5-ProDéfensine A6 contenant de la ProDéfensine-A6 sécrétée est ajouté pur ou dilué en PBS directement dans le deuxième puits de la cartouche HBs Ag Ultra (50 µL).
4. La réaction ELISA a été réalisée à l'aide de l'automate Vidas® et du protocole HBsAg ULTRA dont l'étape d'incubation de l'échantillon avec les anticorps de capture et de révélation avait été porté à 100 cycles.
5. Les résultats ont été obtenus sous forme de valeurs brutes. Le signal est en RFV, relative fluorescence value.

### 9.2. Résultats

Le Tableau 3 récapitule la réactivité des différentes combinaisons d'anticorps sur une dilution au ½ du surnageant de culture 293T transfectées par le plasmide pCMX-XL5-ProDéfensine-A6 et contenant de la protéine ProDéfensine-A6 native.

**Tableau 3^{a}**

| Anticorps de capture | Anticorps de détection biotinylé | |
|---|---|---|
| | 1H8C9 | 11E8C9 |
| 1H8C9 (épitope 2) | - | 43 |
| 11B2D2 (épitope 3) | 955* | 11 |
| 13E7F3 (épitope 5) | 1088* | 2881 |
| 11E8C9 (épitope 4) | 4015* | - |
| 6E1B4 (épitope 1) | 11646 | 3407 |
| 10C2G3 (épitope 1) | 11583 | 7420 |
| 12F3F2 (épitope 1) | 11481 | 6440 |
| 12F8E4 (épitope 1) | 11436 | 3064 |
| 12H4E1 (épitope 1) | 11868 | 11037 |

| | | |
|---|---|---|
| ^{a}Signal en immunoessai sandwich VIDAS, en RFV (relative fluorescence unit). * : signal pour surnageant dosé pur. Les autres surnageants ont été dilués au demi. | | |

Les résultats présentés dans le Tableau 3 montrent qu'il est possible de détecter la ProDéfensine-A6 en utilisant des ELISA sandwich basés sur différentes combinaisons d'épitopes : par exemple épitopes n°5 et 4, épitopes n°1 et 4, ou encore de façon préférentielle une combinaison de l'épitope 2, avec n'importe lequel des autres épitopes identifiés (1, 3, 4, 5). De façon encore plus préférentielle, l'épitope 2 est utilisé en détection. Par contre, les combinaisons des épitopes n°2 et 4 ou encore n°3 et 4 ne permettent pas de détecter la ProDéfensine-A6.

Une analyse plus fine de la réactivité des anticorps dirigés contre l'épitope 1, utilisés en capture, et combinés au clone 1H8C9 est présentée en Figure 5. Le clone 12H4E1 est le meilleur anticorps de capture, en deuxième position sont les clones 10C2G3 et 12F3F2. Les signaux les moins élevés sont obtenus avec les anticorps 6E1B4 et 12F8E4. Ce résultat est très surprenant car les clones 6E1B4 et 12H4E1 reconnaissent la même séquence minimale.

### Exemple 3 : Dosages sériques des marqueurs tumoraux

### 1. Patients et prélèvements

La collecte des échantillons de sang se fait au niveau d'un réseau de 8 centres cliniques répartis dans toute la France, dans le cadre de 2 protocoles loi Huriet.

Pour l'obtention de sérum, le prélèvement sanguin se fait sur tube sec. Pour l'obtention du plasma, le prélèvement sanguin se fait sur tube EDTA. Après coagulation, le tube est centrifugé 10 min à 1000 *g,* le sérum est prélevé, aliquoté et conservé à -80°C. Le tube de plasma est directement centrifugé 10 min à 1000 *g,* le plasma est prélevé, aliquoté et conservé à -80°C. Les échantillons sont parfaitement documentés pour l'histoire clinique des patients.

### 2. Dosage sérique du marqueur tumoral ProDefensine-A6

La protéine précurseur ProDefensine-A6 a été dosée à l'aide des anticorps décrits en détail dans l'exemple 2 et d'un test ELISA utilisant l'automate Vidas® (bioMérieux). Pour ce faire, le test ELISA a été construit en utilisant les réactifs du kit Vidas® HBs Ag Ultra (bioMérieux, Cat. No. 30315). Les réactifs ont été utilisés tels que décrits dans la notice correspondante (réf. 11728 D - FR - 2005/05), avec les modifications suivantes :
1. Les cônes ont été sensibilisés avec l'anticorps de capture 12H4E1 à une concentration de 15 µg/ml.
2. Le contenu du deuxième puits de la cartouche HBs Ag Ultra a été remplacé par 300 µl d'anticorps de révélation 1H8C9, couplé à la biotine, dilué à 1 µg/ml dans le tampon (sans sérum de chèvre) du deuxième puits du kit Vidas® HBs Ag Ultra.
3. Les échantillons de sérum ou de plasma (50µL) ont été dilués directement dans le deuxième puits de la cartouche HBs Ag Ultra.
4. La réaction ELISA a été réalisée à l'aide de l'automate Vidas® et du protocole HBS AG ULTRA dont l'étape d'incubation de l'échantillon avec les anticorps de capture et de révélation avait été porté à 100 cycles.
5. Les résultats ont été obtenus sous forme de valeurs brutes après soustraction du bruit de fond (lecture du substrat avant réaction). Une courbe étalon a été établie en dosant une gamme de concentrations du peptide synthétique Prodéfensine A6 (SEQ ID°1). La courbe étalon a été tracée en reportant en abscisse la concentration du marqueur tumoral et en ordonnée le signal lu par Vidas® (RFV ou Relative Fluorescence Value). La concentration de marqueur tumoral présente dans le fluide corporel à doser (sang, sérum, plasma) a été calculée en reportant la concentration correspondant au signal RFV lu par Vidas®.

Les résultats du dosage de ProDéfensine-A6 sérique chez les patients par ELISA sur automate Vidas sont donnés dans le Tableau 4. Les concentrations sériques de Prodéfensine-A6 se situent entre 0 et 10 pg/mL chez les patients ayant un adénocarcinome colorectal (CCR+) et entre 0 et 2 pg/mL chez les sujets sains (CCR-), ce qui nécessite un dosage ELISA extrêmement sensible pour pouvoir mettre en oeuvre l'invention dans un fluide biologique ou échantillon distant. Les seuls dosages ELISA de la ProDéfensine-A6 qui permettent d'atteindre une limite de détection aussi basse sont basés sur la combinaison des anticorps qui reconnaissent les épitopes 1 et 4. Il est préférable de capturer la ProDéfensine-A6 par l'épitope 1. La combinaison qui donne le dosage le plus sensible est celle qui a été utilisée ici avec l'anticorps 12H4E1 en capture et l'anticorps 1H8C9 en détection.

**Tableau 4**

| **Pathologie^{a}** | **Identifiant Patient** | **Stade** | **TNM^{b}** | **Age** | **Sexe** | **ProDéfensine A6 (pg/mL)** |
|---|---|---|---|---|---|---|
| MDI | CLSP046 | | | 43 | Homme | 0,40 |
| MDI | CLSP049 | | | 30 | Femme | 1,11 |
| MDI | CLSP051 | | | 47 | Femme | 0,61 |
| MDI | CLSP056 | | | 23 | Femme | 2,16 |
| MDI | CLSP065 | | | 41 | Femme | 1,60 |
| MDI | CLSP084 | | | 34 | Femme | 0,10 |
| MDI | CLSP134 | | | 59 | Homme | 0,77 |
| MDI | CLSP135 | | | 47 | Femme | 0,44 |
| MDI | CLSP137 | | | 35 | Femme | 0,23 |
| MDI | CLSP151 | | | 55 | Homme | 0,44 |
| MDI | CLSP152 | | | 36 | Homme | 4,06 |
| CCR + | CBSE011 | 0 | TisN0M0 | 76 | Homme | 1,91 |
| CCR + | CLSP059 | 0 | TisN0M0 | 72 | Homme | 0,49 |
| CCR + | CLSP104 | 0 | TisN0M0 | 48 | Homme | 0,41 |
| CCR + | CBSE001 | I | T1N0M0 | 69 | Femme | 1,44 |
| CCR + | CBSE016 | I | T1N0M0 | 74 | Homme | 2,19 |
| CCR + | CBSE022 | I | T1N0M0 | 78 | Homme | 0,29 |
| CCR + | CBSE025 | I | T2N0M0 | 81 | Femme | 1,45 |
| CCR + | CLSP047 | I | T1N0M0 | 76 | Femme | 0,59 |
| CCR + | CLSP062 | I | T2N0M0 | 83 | Homme | 0,56 |
| CCR + | CLSP067 | I | T2N0M0 | 61 | Homme | 0,45 |
| CCR + | CLSP080 | I | T2N0M0 | 74 | Femme | 1,29 |
| CCR + | CLSP085 | I | T2N0M0 | 74 | Femme | 3,26 |
| CCR + | CLSP086 | I | T2N0M0 | 61 | Homme | 0,23 |
| CCR + | CLSP093 | I | T2N0M0 | 71 | Homme | 0,39 |
| CCR + | CLSP100 | I | T3N0M0 | 53 | Homme | 0,41 |
| CCR + | CLSP118 | I | T2N0M0 | 60 | Femme | 0,76 |
| CCR + | CLSP145 | I | T1N0M0 | 71 | Homme | 1,38 |
| CCR + | CLSP146 | I | T2N0M0 | 55 | Femme | 0,24 |
| CCR + | CLSP150 | I | T2N0M0 | 61 | Homme | 0,24 |
| CCR + | CBSE004 | II | T3N0M0 | 85 | Homme | 0,07 |
| CCR + | CBSE017 | II | T3N0M0 | 74 | Homme | 1,00 |
| CCR + | CBSE018 | II | T4N0M0 | 82 | Homme | 0,26 |
| CCR + | CLSP043 | II | T3N0M0 | 75 | Homme | 1,25 |
| CCR + | CLSP060 | II | T4N0M0 | 84 | Homme | 1,26 |
| CCR + | CLSP069 | II | T3N0M0 | 46 | Homme | 1,07 |
| CCR + | CLSP075 | II | T3N0M0 | 65 | Homme | 1,20 |
| CCR + | CLSP087 | II | T3N0M0 | 75 | Homme | 1,52 |
| CCR + | CLSP088 | II | T3N0M0 | 88 | Femme | 0,72 |
| CCR + | CLSP096 | II | T3N0M0 | 79 | Femme | 0,40 |
| CCR + | CLSP105 | II | T3N0M0 | 73 | Homme | 1,61 |
| CCR + | CLSP107 | II | T3N0M0 | 79 | Homme | 0,38 |
| CCR + | CLSP110 | II | T3N0M0 | 67 | Femme | 0,39 |
| CCR + | CLSP113 | II | T3N0M0 | 50 | Homme | 0,69 |
| CCR + | CLSP115 | II | T3N0M0 | 65 | Femme | 3,62 |
| CCR + | CLSP117 | II | T3N0M0 | 78 | Femme | 1,62 |
| CCR + | CLSP119 | II | T3N0M0 | 78 | Homme | 0,80 |
| CCR + | CLSP122 | II | T3N0MX | 54 | Homme | 0,81 |
| CCR + | CLSP133 | II | T3N0M0 | 78 | Femme | 0,83 |
| CCR + | CLSP136 | II | T3N0M0 | 67 | Femme | 0,22 |
| CCR + | CLSP143 | II | T3N0M0 | 76 | Femme | 1,25 |
| CCR + | CLSP147 | II | T3N0M0 | 83 | Homme | 0,77 |
| CCR + | CLSP154 | II | T3N0M0 | 76 | Femme | 0,86 |
| CCR + | CLSP157 | II | T3N0 | 45 | Femme | 0,42 |
| CCR + | GHBD020 | II | T3N0M0 | 75 | Homme | 1,01 |
| CCR + | GHBD023 | II | T3N0M0 | 57 | Homme | 0,23 |
| CCR + | GHBD025 | II | T3N0M0 | 73 | Homme | 0,73 |
| CCR + | GHBD029 | II | T3N0M0 | 75 | Femme | 2,22 |
| CCR + | CBSE005 | III | T3N1 M0 | 73 | Homme | 4,27 |
| CCR + | CBSE006 | III | T3N1 M0 | 84 | Homme | 9,24 |
| CCR + | CBSE007 | III | T3N1 M0 | 77 | Femme | 2,02 |
| CCR + | CBSE010 | III | T4N2M0 | 67 | Homme | 9,17 |
| CCR + | CBSE013 | III | T3N1 M0 | 82 | Homme | 4,50 |
| CCR + | CBSE023 | III | T4N2M0 | 76 | Homme | 2,04 |
| CCR + | CLSP044 | III | T3N1M0 | 80 | Homme | 0,47 |
| CCR + | CLSP050 | III | T2N1M0 | 88 | Femme | 2,18 |
| CCR + | CLSP072 | III | T3N1M0 | 79 | Homme | 2,17 |
| CCR + | CLSP073 | III | T2N1M0 | 77 | Homme | 1,59 |
| CCR + | CLSP074 | III | T3N2M0 | 79 | Femme | 0,64 |
| CCR + | CLSP089 | III | T4N2M0 | 84 | Femme | 0,81 |
| CCR + | CLSP090 | III | T1N1M0 | 65 | Femme | 1,47 |
| CCR + | CLSP091 | III | T3N1M0 | 55 | Homme | 0,29 |
| CCR + | CLSP094 | III | T3N1M0 | 72 | Homme | 0,47 |
| CCR + | CLSP097 | III | T3N1M0 | 71 | Femme | 0,74 |
| CCR + | CLSP098 | III | T3N2M0 | 61 | Homme | 0,69 |
| CCR + | CLSP103 | III | T2N1 MX | 60 | Homme | 0,40 |
| CCR + | CLSP106 | III | T4N2M0 | 85 | Femme | 1,90 |
| CCR + | CLSP121 | III | T3N2M0 | 76 | Homme | 0,49 |
| CCR + | CLSP123 | III | T4N1 MX | 68 | Homme | 1,81 |
| CCR + | CLSP138 | III | T3N1 MX | 78 | Homme | 1,03 |
| CCR + | CLSP141 | III | T3N1M0 | 70 | Homme | 1,21 |
| CCR + | CLSP144 | III | T3N1M0 | 52 | Femme | 0,93 |
| CCR + | CLSP153 | III | T3N2MX | 85 | Homme | 5,09 |
| CCR + | GHBD019 | III | T3N1M0 | 74 | Homme | 0,92 |
| CCR + | CBSE012 | IV | T4N3M1 | 37 | Femme | 0,50 |
| CCR + | CBSE019 | IV | T3N2M1 | 58 | Homme | 1,52 |
| CCR + | CBSE026 | IV | T4N1M1 | 72 | Homme | 0,61 |
| CCR + | CBSE027 | IV | T4N2M1 | 78 | Femme | 1,04 |
| CCR + | CLSP042 | IV | T3N2M1 | 56 | Homme | 1,39 |
| CCR + | CLSP057 | IV | T3N2M1 | 61 | Femme | 0,27 |
| CCR + | CLSP068 | IV | TXNXM1 | 60 | Femme | 1,29 |
| CCR + | CLSP079 | IV | T3N0M1 | 81 | Femme | 0,81 |
| CCR + | CLSP083 | IV | T3N1 M1 | 67 | Femme | 0,79 |
| CCR + | CLSP095 | IV | T3N1 M1 | 64 | Homme | 0,65 |
| CCR + | CLSP109 | IV | T3N1 M1 | 60 | Homme | 0,88 |
| CCR + | CLSP132 | IV | T3N1 M1 | 62 | Homme | 2,80 |
| CCR + | CLSP156 | IV | T4N0M1 | 59 | Homme | 1,25 |
| CCR + | CLSP159 | IV | T3N0M1 | 68 | Femme | 0,89 |
| CCR + | CLSP160 | IV | TXNXM1 | 70 | Homme | 2,77 |
| CCR + | CLSP161 | IV | T3N2M1 | 78 | Femme | 0,64 |
| Adénome | CLSP055 | | | 73 | Homme | 0,55 |
| Adénome | CLSP058 | | | 55 | Femme | 0,58 |
| Adénome | CLSP061 | | | 61 | Femme | 0,62 |
| Adénome | CLSP099 | | T0N0M0 | 62 | Homme | 0,13 |
| Adénome | CLSP116 | | | 63 | Homme | 0,66 |
| Adénome | CLSP120 | | | 56 | Femme | 0,30 |
| Adénome | CLSP142 | | T0 | 21 | Homme | 1,77 |
| Adénome | CLSP148 | | TisN0M0 | 50 | Femme | 1,07 |
| Adénome | CLSP149 | | T1N0M0 | 50 | Femme | 1,69 |
| CCR - | N00656 | | | 47 | Femme | 0,22 |
| CCR - | N006615 | | | 43 | Femme | 0,45 |
| CCR - | N00664- | | | 44 | Homme | 0,13 |
| CCR - | N006658 | | | 48 | Homme | 0,46 |
| CCR - | N009901 | | | 52 | Homme | 0,65 |
| CCR - | N011147 | | | 50 | Homme | 0,87 |
| CCR - | N011155 | | | 51 | Homme | 0,61 |
| CCR - | N011243 | | | 52 | Homme | 0,33 |
| CCR - | N017218 | | | 44 | Femme | 0,49 |
| CCR - | N017234 | | | 37 | Homme | 0,47 |
| CCR - | N017250 | | | 48 | Femme | 0,48 |
| CCR - | N017269 | | | 40 | Homme | 0,82 |
| CCR - | N017365 | | | 44 | Femme | 0,54 |
| CCR - | N017402 | | | 25 | Homme | 0,65 |
| CCR - | N017410 | | | 37 | Homme | 0,53 |
| CCR - | N018552 | | | 42 | Homme | 0,33 |
| CCR - | N041082 | | | 58 | Homme | 1,16 |
| CCR - | N041138 | | | 58 | Homme | 0,97 |
| CCR - | N044703 | | | 54 | Homme | 0,37 |
| CCR - | N045730 | | | 50 | Homme | 0,57 |
| CCR - | N14397- | | | 58 | Homme | 0,71 |
| CCR - | N143988 | | | 62 | Homme | 0,49 |
| CCR - | N144358 | | | 61 | Femme | 0,67 |
| CCR - | N146601 | | | 57 | Homme | 0,81 |
| CCR - | N14661- | | | 61 | Femme | 1,14 |
| CCR - | N146695 | | | 52 | Homme | 0,91 |
| CCR - | N14813- | | | 57 | Homme | 0,72 |
| CCR - | N148279 | | | 55 | Homme | 1,04 |
| CCR - | N148340 | | | 51 | Homme | 1,05 |
| CCR - | N314164 | | | 48 | Homme | 1,00 |
| CCR - | N318050 | | | 56 | Homme | 0,64 |
| CCR - | N318077 | | | 56 | Homme | 1,02 |
| CCR - | N318368 | | | 60 | Homme | 0,50 |
| CCR - | N318384 | | | 58 | Femme | 0,69 |
| CCR - | N318421 | | | 60 | Femme | 0,64 |
| CCR - | N325015 | | | 42 | Homme | 0,44 |
| CCR - | N329630 | | | 59 | Femme | 0,39 |
| CCR - | N370529 | | | 57 | Homme | 0,40 |
| CCR - | N376461 | | | 58 | Homme | 0,26 |
| CCR - | N376488 | | | 63 | Femme | 0,22 |
| CCR - | N37663- | | | 62 | Homme | 0,40 |
| CCR - | N376760 | | | 58 | Femme | 0,57 |
| CCR - | N376912 | | | 64 | Femme | 0,57 |
| CCR - | N418599 | | | 56 | Femme | 0,23 |
| CCR - | N418687 | | | 28 | Femme | 0,19 |
| CCR - | N418716 | | | 53 | Femme | 0,65 |
| CCR - | N418740 | | | 54 | Homme | 0,75 |
| CCR - | N418759 | | | 49 | Femme | 0,84 |
| CCR - | N418804 | | | 54 | Femme | 0,24 |
| CCR - | N440216 | | | 60 | Femme | 0,40 |
| CCR - | N440478 | | | 60 | Femme | 0,49 |
| CCR - | N440507 | | | 64 | Homme | 1,02 |
| CCR - | N469775 | | | 36 | Homme | 0,47 |
| CCR - | N491028 | | | 50 | Homme | 0,59 |
| CCR - | N491191 | | | 52 | Femme | 1,16 |
| CCR - | N491247 | | | 58 | Homme | 0,35 |
| CCR - | N491386 | | | 58 | Homme | 0,71 |
| CCR - | N491685 | | | 56 | Homme | 0,52 |
| CCR - | N511463 | | | 52 | Femme | 0,46 |
| CCR - | N511471 | | | 59 | Femme | 0,68 |
| CCR - | N511498 | | | 55 | Femme | 0,58 |
| CCR - | N518059 | | | 0 | Homme | 0,69 |
| CCR - | N518518 | | | 58 | Femme | 0,78 |
| CCR - | N518542 | | | 60 | Homme | 0,81 |
| CCR - | N519086 | | | 59 | Homme | 0,80 |
| CCR - | N527135 | | | 56 | Femme | 0,39 |
| CCR - | N527450 | | | 56 | Femme | 0,38 |
| CCR - | N557699 | | | 55 | Homme | 1,40 |
| CCR - | N557701 | | | 57 | Homme | 1,66 |
| CCR - | N557736 | | | 56 | Homme | 0,41 |
| CCR - | N557760 | | | 60 | Homme | 1,68 |
| CCR - | N593116 | | | 52 | Femme | 0,29 |
| CCR - | N593167 | | | 53 | Homme | 1,12 |
| CCR - | N593183 | | | 52 | Femme | 0,25 |
| CCR - | N593255 | | | 51 | Homme | 1,88 |
| CCR - | N593351 | | | 57 | Homme | 1,34 |
| CCR - | N744056 | | | 51 | Homme | 0,61 |
| CCR - | N748022 | | | 50 | Femme | 0,33 |
| CCR - | N835966 | | | 45 | Homme | 0,62 |
| CCR - | N836299 | | | 52 | Femme | 1,13 |
| CCR - | N857704 | | | 52 | Femme | 0,29 |
| CCR - | N858037 | | | 63 | Femme | 1,15 |
| CCR - | N858248 | | | 62 | Homme | 1,72 |
| CCR - | N862239 | | | 53 | Homme | 0,24 |
| CCR - | N862298 | | | 63 | Homme | 0,42 |
| CCR - | N862300 | | | 51 | Homme | 1,81 |

| | | | | | | |
|---|---|---|---|---|---|---|
| a : MDI = Maladies Digestives Inflammatoires (Crohn et Rectocolite Hémorragique) CCR+ = patients atteints d'un cancer colorectal (Adénocarcinome), CCR- = sujets sains. b : TNM : stade d'invasion tissulaire (T), ganglionnaire (lymph nodes, N) et à distance (métastases, M) | | | | | | |

Les doses obtenues pour les patients analysés sont reportées sur la Figure 6. On peut noter sur cette figure et dans le Tableau 4 que 2, 2, 10 et 2 patients ayant un cancer colorectal respectivement de stade I, II, III ou IV montrent une nette augmentation de leur dose de ProDéfensine-A6 sérique, strictement supérieure à la valeur la plus élevée observée dans le groupe des sujets sains (1,88 pg/mL). Dans le groupe Adénome aucune dose observée ne dépasse cette valeur, alors que dans le groupe MDI, il y en a 2.

### Exemple 4 : Utilisation des dosages sériques des marqueurs tumoraux en combinaison

La Demanderesse a montré dans l'exemple 3 que des doses anormalement élevées de protéine précurseur ProDéfensine-A6 pouvaient être observées dans la circulation sanguine de certains patients atteints d'un cancer colorectal. D'autre part, la Demanderesse a montré dans les demandes de brevet WO2009/024691, WO2009019365, WO2009019368, WO2009019369, WO2009019366, WO2009019370, WO2009019367 que des doses anormalement élevées ou anormalement diminuées de d'autres marqueurs tumoraux comme LEI, Ezrine, Aminoacylase-1, L-FABP, Apo A1, Apo A2, Plastine I, Beta2 Microglobuline, ACE, CA19-9, Testostérone, Galectine-3, LDH-B, Protéasome 20S, E-Cadhérine, Regenerating islet-derived protein 3 alpha, autrement nommée Pancreatitis Associated Protein (PAP1) pouvaient être observées dans la circulation sanguine de certains patients atteints d'un cancer colorectal. Les procédés de dosage de ces marqueurs tumoraux ont été décrits dans les demandes de brevets suscités. Le procédé de dosage de MIF a été mis en oeuvre avec le kit ELISA human MIF Quantikine de R&D Systems (Cat No. DMF00) selon les instructions du fabricant.

De façon surprenante, l'augmentation ou la diminution de la dose sanguine de deux marqueurs donnés n'est pas systématiquement observée chez les mêmes patients. De ce fait, la combinaison de plusieurs marqueurs tumoraux permet d'augmenter le nombre de patients identifiés comme ayant un cancer colorectal. C'est ainsi qu'un patient A peut présenter une augmentation ou une diminution d'un ou plusieurs marqueurs tumoraux (groupe X), les dit marqueurs du groupe X pouvant être normaux chez un patient B ; chez ce même patient B un ou plusieurs autres marqueurs tumoraux (groupe Y) peuvent être élevés ou diminués, les dit marqueurs du groupe Y pouvant être normaux chez le patient A.

Les différents marqueurs tumoraux dosés par la Demanderesse peuvent ainsi être combinés au moyen de divers algorithmes mathématiques bien connus de l'homme du métier. A titre d'illustration et sans que cet exemple ait un caractère exhaustif, il a été mis en oeuvre le procédé suivant :
1. Une valeur seuil a été fixée pour chaque marqueur tumoral.
2. Lorsque la dose sanguine du marqueur tumoral était augmentée en cas de cancer colorectal, la dose sanguine obtenue pour un patient donné a été divisée par sa valeur seuil. Lorsque la dose sanguine du marqueur tumoral était diminuée en cas de cancer colorectal, la dose sanguine obtenue pour un patient donné a été inversée puis multipliée par sa valeur seuil.
3. Lorsque le ratio, dose sanguine divisée par valeur seuil, était supérieur à 1, le ratio a été multiplié par un coefficient, par exemple 10. La valeur ainsi obtenue a été baptisée « score » pour le patient étudié du marqueur tumoral considéré.
4. Les scores obtenus pour différents marqueurs tumoraux ont été ajoutés en les pondérant d'un facteur propre à chaque marqueur. Dans le cas de l'exemple ci-dessous tous les facteurs de pondération ont été fixés à 1.
5. La somme des scores a été divisée par le nombre total de scores sommés et la valeur ainsi obtenue a été baptisée « score total ».
6. Le patient est diagnostiqué comme ayant un cancer colorectal lorsque sont score total est augmenté par rapport à un score seuil.

Les scores totaux pour une sélection de 2, 3, 4, 5, 7 et 8 marqueurs comprenant la ProDéfensine-A6 sont donnés dans le Tableau 5.

L'association des marqueurs tumoraux ProDéfensine-A6 et ACE permet ainsi d'obtenir pour le même groupe de 78 patients des scores totaux « 2^{a} » augmentés chez 41 patients atteints d'un adénocarcinome colorectal alors que le seul dosage de ProDéfensine-A6 et d'ACE était augmenté respectivement chez 17 et 28 patients seulement.

L'association des marqueurs tumoraux ProDéfensine-A6 et CA19-9 permet ainsi d'obtenir pour le même groupe de 78 patients des scores totaux « 2^{b} » augmentés chez 28 patients atteints d'un adénocarcinome colorectal alors que le seul dosage de ProDéfensine-A6 et d'ACE était augmenté respectivement chez 17 et 15 patients seulement.

L'association des marqueurs tumoraux ProDéfensine-A6 et Béta-2 Microglobuline permet ainsi d'obtenir pour le même groupe de 78 patients des scores totaux « 2^{c} » augmentés chez 49 patients atteints d'un adénocarcinome colorectal alors que le seul dosage de ProDéfensine-A6 et de Béta-2 Microglobuline était augmenté respectivement chez 17 et 43 patients seulement.

L'association des marqueurs tumoraux ProDéfensine-A6 et L-FABP permet ainsi d'obtenir pour le même groupe de 78 patients des scores totaux « 2^{d} » augmentés chez 41 patients atteints d'un adénocarcinome colorectal alors que le seul dosage de ProDéfensine-A6 et de L-FABP était augmenté respectivement chez 17 et 35 patients seulement.

L'association des marqueurs tumoraux ProDéfensine-A6, CA19-9 et ACE permet ainsi d'obtenir pour le même groupe de 78 patients des scores totaux « 3^{e} » augmentés chez 46 patients atteints d'un adénocarcinome colorectal alors que le seul dosage de ProDéfensine-A6, de CA19-9 et d'ACE était augmenté respectivement chez 17, 15 et 28 patients seulement.

L'association des marqueurs tumoraux ProDéfensine-A6, Béta-2 Microglobuline et ACE permet ainsi d'obtenir pour le même groupe de 78 patients des scores totaux « 3^{f} » augmentés chez 60 patients atteints d'un adénocarcinome colorectal alors que le seul dosage de ProDéfensine-A6, de Béta-2 Microglobuline et d'ACE était augmenté respectivement chez 17, 43 et 28 patients seulement.

L'association des marqueurs tumoraux ProDéfensine-A6, Béta-2 Microglobuline, CA19-9 et ACE permet ainsi d'obtenir pour le même groupe de 78 patients des scores totaux « 4^{g} » augmentés chez 63 patients atteints d'un adénocarcinome colorectal alors que le seul dosage de ProDéfensine-A6, de Béta-2 Microglobuline, de CA19-9 et d'ACE était augmenté respectivement chez 17, 43, 15 et 28 patients seulement.

L'association des marqueurs tumoraux ProDéfensine-A6, Béta-2 Microglobuline, L-FABP et ACE permet ainsi d'obtenir pour le même groupe de 78 patients des scores totaux « 4^{h} » augmentés chez 69 patients atteints d'un adénocarcinome colorectal alors que le seul dosage de ProDéfensine-A6, de Béta-2 Microglobuline, de L-FABP et d'ACE était augmenté respectivement chez 17, 43, 35 et 28 patients seulement.

L'association des marqueurs tumoraux ProDéfensine-A6, CA19-9, L-FABP et ACE permet ainsi d'obtenir pour le même groupe de 78 patients des scores totaux « 4ⁱ » augmentés chez 59 patients atteints d'un adénocarcinome colorectal alors que le seul dosage de ProDéfensine-A6, de CA19-9, de L-FABP et d'ACE était augmenté respectivement chez 17, 15, 35 et 28 patients seulement.

L'association des marqueurs tumoraux ProDéfensine-A6, Béta-2 Microglobuline, CA19-9, L-FABP et ACE permet ainsi d'obtenir pour le même groupe de 78 patients des scores totaux « 5^{j} » augmentés chez 71 patients atteints d'un adénocarcinome colorectal alors que le seul dosage de ProDéfensine-A6, de Béta-2 Microglobuline, de CA19-9, de L-FABP et d'ACE était augmenté respectivement chez 17, 43, 15, 35 et 28 patients seulement.

L'association des marqueurs tumoraux ProDéfensine-A6, Béta-2 Microglobuline, CA19-9, Galectine-3, L-FABP, MIF et ACE permet ainsi d'obtenir pour le même groupe de 78 patients des scores totaux « 7^{k} » augmentés chez 74 patients atteints d'un adénocarcinome colorectal alors que le seul dosage de ProDéfensine-A6, de Béta-2 Microglobuline, de CA19-9, de Galectine-3, de L-FABP, de MIF et d'ACE était augmenté respectivement chez 17, 43, 15, 13, 35, 23 et 28 patients seulement.

L'association des marqueurs tumoraux ProDéfensine-A6, Béta-2 Microglobuline, CA19-9, Galectine-3, L-FABP, MIF, Plastine I et ACE permet ainsi d'obtenir pour le même groupe de 78 patients des scores totaux « 8^{l} » augmentés chez 75 patients atteints d'un adénocarcinome colorectal alors que le seul dosage de ProDéfensine-A6, de Béta-2 Microglobuline, de CA19-9, de Galectine-3, de L-FABP, de MIF, de Plastine I et d'ACE était augmenté respectivement chez 17, 43, 15, 13, 35, 23, 3 et 28 patients seulement.

**Tableau 5**

| Pathologie | Identifiant patient | Score 2^{a} | Score 2^{b} | Score 2^{c} | Score 2^{d} | Score 3^{e} | Score 3^{f} | Score 4^{g} | Score 4^{h} | Score 4ⁱ | Score 5^{j} | Score 7^{k} | Score 8^{l} |
|---|---|---|---|---|---|---|---|---|---|---|---|---|---|
| CCR - | N011155 | 0.32 | 0.32 | 0.32 | 0.32 | 0.32 | 0.32 | 0.32 | 0.32 | 0.32 | 0.32 | 0.32 | 0.32 |
| CCR - | N011243 | 0.18 | 0.18 | 0.18 | 0.14 | 0.18 | 0.18 | 0.18 | 0.14 | 0.14 | 0.14 | 0.14 | 0.14 |
| CCR - | N14661- | 0.61 | 0.61 | 0.61 | 0.39 | 0.61 | 0.61 | 0.61 | 0.39 | 0.39 | 0.39 | 0.39 | 0.39 |
| CCR - | N044703 | 0.20 | 0.20 | 0.20 | 0.21 | 0.20 | 0.20 | 0.20 | 0.21 | 0.21 | 0.21 | 0.21 | 0.21 |
| CCR - | N318050 | 0.34 | 0.34 | 0.34 | 0.31 | 0.34 | 0.34 | 0.34 | 0.31 | 0.31 | 0.31 | 0.31 | 0.31 |
| CCR - | N418599 | 0.12 | 0.12 | 0.12 | 0.23 | 0.12 | 0.12 | 0.12 | 0.23 | 0.23 | 0.23 | 0.23 | 0.23 |
| CCR - | N017410 | 0.28 | 0.28 | 0.28 | 0.27 | 0.28 | 0.28 | 0.28 | 0.27 | 0.27 | 0.27 | 0.27 | 0.27 |
| CCR - | N329630 | 0.21 | 0.21 | 0.21 | 0.33 | 0.21 | 0.21 | 0.21 | 0.33 | 0.33 | 0.33 | 0.33 | 0.33 |
| CCR - | N045730 | 0.30 | 0.30 | 0.30 | 0.32 | 0.30 | 0.30 | 0.30 | 0.32 | 0.32 | 0.32 | 0.32 | 0.32 |
| CCR - | N593116 | 0.15 | 0.15 | 0.15 | 0.27 | 0.15 | 0.15 | 0.15 | 0.27 | 0.27 | 0.27 | 0.27 | 0.27 |
| CCR - | N018552 | 0.18 | 0.18 | 0.18 | 0.27 | 0.18 | 0.18 | 0.18 | 0.27 | 0.27 | 0.27 | 0.27 | 0.27 |
| CCR - | N017218 | 0.26 | 0.26 | 0.26 | 0.38 | 0.26 | 0.26 | 0.26 | 0.38 | 0.38 | 0.38 | 0.38 | 0.38 |
| CCR - | N011147 | 0.46 | 0.46 | 0.46 | 0.43 | 0.46 | 0.46 | 0.46 | 0.43 | 0.43 | 0.43 | 0.43 | 0.43 |
| CCR - | N017365 | 0.29 | 0.29 | 0.29 | 0.33 | 0.29 | 0.29 | 0.29 | 0.33 | 0.33 | 0.33 | 0.33 | 0.33 |
| CCR - | N440216 | 0.21 | 0.21 | 0.21 | 0.33 | 0.21 | 0.21 | 0.21 | 0.33 | 0.33 | 0.33 | 0.33 | 0.33 |
| CCR - | N376912 | 0.30 | 0.30 | 0.30 | 0.38 | 0.30 | 0.30 | 0.30 | 0.38 | 0.38 | 0.38 | 0.38 | 0.38 |
| CCR - | N527450 | 0.20 | 0.20 | 0.20 | 0.34 | 0.20 | 0.20 | 0.20 | 0.34 | 0.34 | 0.34 | 0.34 | 0.34 |
| CCR - | N748022 | 0.18 | 0.18 | 0.18 | 0.26 | 0.18 | 0.18 | 0.18 | 0.26 | 0.26 | 0.26 | 0.26 | 0.26 |
| CCR - | N593183 | 0.13 | 0.13 | 0.13 | 0.32 | 0.13 | 0.13 | 0.13 | 0.32 | 0.32 | 0.32 | 0.32 | 0.32 |
| CCR - | N009901 | 0.35 | 0.35 | 0.35 | 0.46 | 0.35 | 0.35 | 0.35 | 0.46 | 0.46 | 0.46 | 0.46 | 0.46 |
| CCR - | N017269 | 0.44 | 0.44 | 0.44 | 0.49 | 0.44 | 0.44 | 0.44 | 0.49 | 0.49 | 0.49 | 0.49 | 0.49 |
| CCR - | N376461 | 0.14 | 0.14 | 0.14 | 0.37 | 0.14 | 0.14 | 0.14 | 0.37 | 0.37 | 0.37 | 0.37 | 0.37 |
| CCR - | N862300 | 0.96 | 0.96 | 0.96 | 0.77 | 0.96 | 0.96 | 0.96 | 0.77 | 0.77 | 0.77 | 0.77 | 0.77 |
| CCR - | N376488 | 0.12 | 0.12 | 0.12 | 0.42 | 0.12 | 0.12 | 0.12 | 0.42 | 0.42 | 0.42 | 0.42 | 0.42 |
| CCR - | N017234 | 0.25 | 0.25 | 0.25 | 0.47 | 0.25 | 0.25 | 0.25 | 0.47 | 0.47 | 0.47 | 0.47 | 0.47 |
| CCR - | N527135 | 0.21 | 0.21 | 0.21 | 0.44 | 0.21 | 0.21 | 0.21 | 0.44 | 0.44 | 0.44 | 0.44 | 0.44 |
| CCR - | N440478 | 0.26 | 0.26 | 0.26 | 0.44 | 0.26 | 0.26 | 0.26 | 0.44 | 0.44 | 0.44 | 0.44 | 0.44 |
| CCR - | N017402 | 0.35 | 0.35 | 0.35 | 0.56 | 0.35 | 0.35 | 0.35 | 0.56 | 0.56 | 0.56 | 0.56 | 0.56 |
| CCR - | N491191 | 0.62 | 0.62 | 0.60 | 0.62 | 0.62 | 0.60 | 0.60 | 0.60 | 0.62 | 0.60 | 0.55 | 0.55 |
| CCR - | N835966 | 0.33 | 0.33 | 0.56 | 0.25 | 0.33 | 0.56 | 0.56 | 0.43 | 0.25 | 0.43 | 0.45 | 0.45 |
| CCR - | N017250 | 0.21 | 0.22 | 0.26 | 0.29 | 0.20 | 0.21 | 0.20 | 0.25 | 0.23 | 0.23 | 0.23 | 0.23 |
| CCR - | N144358 | 0.36 | 0.36 | 0.49 | 0.28 | 0.36 | 0.49 | 0.49 | 0.39 | 0.28 | 0.39 | 0.40 | 0.40 |
| CCR - | N143988 | 0.26 | 0.26 | 0.51 | 0.33 | 0.26 | 0.51 | 0.51 | 0.47 | 0.33 | 0.47 | 0.48 | 0.48 |
| CCR - | N511498 | 0.30 | 0.23 | 0.51 | 0.35 | 0.25 | 0.44 | 0.37 | 0.43 | 0.29 | 0.37 | 0.34 | 0.33 |
| CCR - | N318384 | 0.44 | 0.19 | 0.49 | 0.31 | 0.30 | 0.50 | 0.38 | 0.44 | 0.29 | 0.35 | 0.37 | 0.33 |
| CCR - | N148340 | 0.32 | 0.33 | 0.62 | 0.54 | 0.24 | 0.44 | 0.35 | 0.46 | 0.32 | 0.39 | 0.42 | 0.39 |
| CCR - | N491386 | 0.20 | 0.20 | 0.48 | 0.44 | 0.14 | 0.32 | 0.25 | 0.37 | 0.23 | 0.30 | 0.33 | 0.29 |
| CCR - | N518518 | 0.49 | 0.29 | 0.59 | 0.48 | 0.38 | 0.59 | 0.48 | 0.57 | 0.42 | 0.49 | 0.44 | 0.39 |
| CCR - | N518542 | 0.32 | 0.22 | 0.57 | 0.22 | 0.22 | 0.45 | 0.34 | 0.34 | 0.16 | 0.27 | 0.28 | 0.29 |
| CCR - | N858248 | 0.61 | 0.63 | 0.87 | 0.72 | 0.52 | 0.68 | 0.60 | 0.65 | 0.53 | 0.59 | 0.55 | 0.48 |
| CCR - | N491247 | 0.35 | 0.19 | 0.42 | 0.35 | 0.30 | 0.46 | 0.39 | 0.47 | 0.36 | 0.42 | 0.43 | 0.39 |
| CCR - | N148279 | 0.38 | 0.35 | 0.70 | 0.46 | 0.30 | 0.53 | 0.44 | 0.49 | 0.32 | 0.42 | 0.44 | 0.40 |
| CCR - | N418687 | 0.26 | 0.06 | 0.45 | 0.23 | 0.18 | 0.44 | 0.33 | 0.42 | 0.22 | 0.34 | 0.41 | 0.36 |
| CCR - | N857704 | 0.22 | 0.33 | 0.44 | 0.22 | 0.32 | 0.39 | 0.42 | 0.37 | 0.31 | 0.39 | 0.46 | 0.41 |
| CCR - | N593255 | 0.65 | 0.54 | 0.97 | 0.72 | 0.46 | 0.74 | 0.58 | 0.67 | 0.45 | 0.55 | 0.50 | 0.43 |
| CCR - | N862298 | 0.37 | 0.15 | 0.22 | 0.22 | 0.27 | 0.37 | 0.27 | 0.37 | 0.27 | 0.27 | 0.41 | 0.33 |
| CCR - | N318421 | 0.58 | 0.21 | 0.67 | 0.47 | 0.41 | 0.72 | 0.56 | 0.69 | 0.46 | 0.57 | 0.56 | 0.49 |
| CCR - | N511471 | 0.29 | 0.21 | 0.53 | 0.47 | 0.22 | 0.43 | 0.33 | 0.46 | 0.31 | 0.38 | 0.38 | 0.36 |
| CCR - | N325015 | 0.40 | 0.48 | 0.50 | 0.33 | 0.51 | 0.52 | 0.58 | 0.50 | 0.49 | 0.55 | 0.54 | 0.47 |
| CCR - | N146695 | 0.27 | 0.30 | 0.54 | 0.46 | 0.22 | 0.38 | 0.32 | 0.39 | 0.27 | 0.34 | 0.36 | 0.32 |
| CCR - | N318077 | 0.47 | 0.32 | 0.64 | 0.59 | 0.35 | 0.56 | 0.44 | 0.58 | 0.42 | 0.48 | 0.51 | 0.46 |
| CCR - | N858037 | 0.40 | 0.32 | 0.71 | 0.50 | 0.28 | 0.53 | 0.41 | 0.49 | 0.30 | 0.40 | 0.47 | 0.42 |
| CCR - | N511463 | 0.16 | 0.20 | 0.46 | 0.29 | 0.16 | 0.33 | 0.29 | 0.34 | 0.20 | 0.30 | 0.43 | 0.40 |
| CCR - | N593167 | 0.45 | 0.62 | 0.59 | 0.47 | 0.51 | 0.49 | 0.53 | 0.46 | 0.47 | 0.50 | 0.50 | 0.55 |
| CCR - | N557736 | 0.57 | 0.14 | 0.41 | 0.23 | 0.40 | 0.58 | 0.45 | 0.50 | 0.36 | 0.41 | 0.41 | 0.39 |
| CCR - | N314164 | 0.36 | 0.46 | 0.58 | 0.56 | 0.37 | 0.45 | 0.43 | 0.48 | 0.42 | 0.46 | 0.54 | 0.47 |
| CCR - | N862239 | 0.31 | 0.29 | 0.44 | 0.24 | 0.36 | 0.46 | 0.46 | 0.43 | 0.36 | 0.44 | 0.46 | 0.40 |
| CCR - | N491685 | 0.26 | 0.17 | 0.64 | 0.42 | 0.20 | 0.51 | 0.40 | 0.52 | 0.29 | 0.43 | 0.49 | 0.44 |
| CCR - | N518059 | 0.27 | 0.22 | 0.55 | 0.58 | 0.20 | 0.42 | 0.34 | 0.52 | 0.35 | 0.43 | 0.47 | 0.48 |
| CCR - | N519086 | 0.31 | 0.32 | 0.56 | 0.52 | 0.28 | 0.44 | 0.38 | 0.48 | 0.36 | 0.43 | 0.43 | 0.45 |
| CCR - | N37663- | 0.33 | 0.61 | 0.55 | 0.35 | 0.55 | 0.52 | 0.64 | 0.51 | 0.54 | 0.61 | 0.62 | 0.55 |
| CCR - | N440507 | 0.43 | 0.28 | 0.76 | 0.54 | 0.30 | 0.61 | 0.47 | 0.59 | 0.36 | 0.48 | 0.53 | 0.46 |
| CCR - | N376760 | 0.65 | 0.52 | 0.53 | 0.43 | 0.68 | 0.69 | 0.70 | 0.65 | 0.65 | 0.67 | 0.64 | 0.59 |
| CCR - | N469775 | 0.24 | 0.17 | 0.50 | 0.39 | 0.20 | 0.41 | 0.33 | 0.44 | 0.28 | 0.37 | 0.47 | 0.41 |
| CCR - | N491028 | 0.26 | 0.20 | 0.54 | 0.35 | 0.20 | 0.43 | 0.34 | 0.42 | 0.25 | 0.35 | 0.44 | 0.39 |
| CCR - | N146601 | 0.51 | 0.42 | 0.63 | 0.48 | 0.47 | 0.61 | 0.56 | 0.59 | 0.49 | 0.55 | 0.62 | 0.54 |
| CCR - | N557701 | 0.55 | 0.68 | 0.83 | 0.75 | 0.52 | 0.63 | 0.59 | 0.63 | 0.55 | 0.59 | 0.57 | 0.50 |
| CCR - | N557760 | 0.56 | 0.56 | 0.80 | 0.95 | 0.45 | 0.60 | 0.51 | 0.70 | 0.58 | 0.61 | 0.58 | 0.56 |
| CCR - | N557699 | 0.60 | 0.72 | 0.82 | 0.56 | 0.63 | 0.70 | 0.70 | 0.62 | 0.57 | 0.63 | 0.63 | 0.61 |
| CCR - | N593351 | 0.76 | 0.48 | 0.63 | 0.59 | 0.59 | 0.68 | 0.58 | 0.63 | 0.56 | 0.55 | 0.55 | 0.61 |
| CCR - | N744056 | 0.30 | 0.48 | 0.47 | 0.28 | 0.42 | 0.41 | 0.47 | 0.36 | 0.37 | 0.42 | 0.49 | 0.42 |
| CCR + | CBSE011 | 5.13 | 5.12 | 12.17 | 5.43 | 3.45 | 8.15 | 6.13 | 6.29 | 2.76 | 5.05 | 6.17 | 5.30 |
| CCR + | CLSP059 | 0.35 | 0.18 | 0.44 | 5.71 | 0.27 | 0.44 | 0.35 | 3.12 | 2.99 | 2.51 | 3.45 | 3.04 |
| CCR + | CLSP104 | 0.27 | 7.10 | 0.44 | 0.48 | 4.85 | 0.40 | 3.80 | 0.49 | 3.82 | 3.19 | 5.63 | 4.96 |
| CCR + | CBSE001 | 0.77 | 0.77 | 0.77 | 10.28 | 0.77 | 0.77 | 0.77 | 10.28 | 10.28 | 10.28 | 10.28 | 10.28 |
| CCR + | CBSE016 | 19.26 | 5.83 | 12.92 | 6.18 | 12.85 | 17.57 | 13.18 | 13.35 | 9.81 | 10.69 | 9.02 | 7.73 |
| CCR + | CBSE022 | 0.20 | 0.08 | 8.62 | 0.38 | 0.14 | 5.83 | 4.38 | 4.53 | 0.26 | 3.62 | 6.91 | 5.93 |
| CCR + | CBSE025 | 0.72 | 0.50 | 8.93 | 0.87 | 0.55 | 6.18 | 4.69 | 4.87 | 0.65 | 3.94 | 5.58 | 4.80 |
| CCR + | CLSP047 | 0.29 | 0.17 | 5.46 | 0.37 | 0.20 | 3.73 | 2.80 | 2.90 | 0.26 | 2.33 | 1.84 | 1.66 |
| CCR + | CLSP062 | 0.37 | 5.28 | 6.47 | 0.40 | 3.67 | 4.46 | 5.91 | 3.47 | 2.88 | 4.83 | 5.23 | 4.66 |
| CCR + | CLSP067 | 0.21 | 0.14 | 0.54 | 7.28 | 0.16 | 0.42 | 0.33 | 3.90 | 3.70 | 3.13 | 2.40 | 2.16 |
| CCR + | CLSP080 | 0.40 | 0.46 | 5.77 | 0.69 | 0.35 | 3.89 | 2.97 | 3.89 | 0.35 | 2.97 | 2.19 | 1.88 |
| CCR + | CLSP085 | 8.75 | 8.75 | 9.14 | 9.00 | 5.89 | 6.15 | 4.65 | 4.78 | 4.58 | 3.86 | 2.95 | 2.61 |
| CCR + | CLSP086 | 168.1 | 0.24 | 0.35 | 0.31 | 112.2 | 112.3 | 84.30 | 84.34 | 84.29 | 67.54 | 48.35 | 42.34 |
| CCR + | CLSP093 | 0.24 | 0.26 | 0.42 | 8.93 | 0.26 | 0.37 | 0.36 | 4.69 | 4.61 | 3.81 | 2.85 | 2.52 |
| CCR + | CLSP100 | 0.44 | 0.19 | 6.86 | 7.48 | 0.35 | 4.79 | 3.63 | 7.28 | 3.95 | 5.86 | 4.32 | 3.79 |
| CCR + | CLSP118 | 0.21 | 0.22 | 0.63 | 0.44 | 0.15 | 0.42 | 0.33 | 0.44 | 0.23 | 0.36 | 0.40 | 2.32 |
| CCR + | CLSP145 | 0.51 | 0.42 | 7.10 | 0.78 | 0.38 | 4.83 | 3.65 | 3.83 | 0.49 | 3.09 | 2.41 | 2.11 |
| CCR + | CLSP146 | 0.17 | 0.23 | 0.36 | 7.47 | 0.22 | 0.31 | 0.32 | 3.94 | 3.87 | 3.22 | 3.99 | 3.50 |
| CCR + | CBSE004 | 0.37 | 0.27 | 7.18 | 0.18 | 0.41 | 5.02 | 3.89 | 3.85 | 0.39 | 3.18 | 9.53 | 8.19 |
| CCR + | CBSE017 | 17.51 | 0.36 | 7.89 | 0.74 | 11.73 | 16.76 | 12.61 | 12.80 | 9.03 | 10.28 | 8.72 | 7.47 |
| CCR + | CBSE018 | 0.19 | 0.17 | 0.42 | 0.26 | 0.19 | 0.36 | 0.32 | 0.36 | 0.24 | 0.33 | 2.80 | 2.40 |
| CCR + | CLSP043 | 0.54 | 0.56 | 5.38 | 0.66 | 0.51 | 3.72 | 2.90 | 3.72 | 0.51 | 2.90 | 4.01 | 3.45 |
| CCR + | CLSP060 | 0.64 | 0.66 | 8.88 | 6.69 | 0.65 | 6.13 | 4.76 | 7.77 | 3.66 | 6.35 | 5.39 | 4.66 |
| CCR + | CLSP087 | 0.59 | 0.45 | 7.11 | 0.66 | 0.43 | 4.87 | 3.67 | 3.78 | 0.45 | 3.04 | 2.32 | 2.03 |
| CCR + | CLSP088 | 13.90 | 7.95 | 0.67 | 5.87 | 14.44 | 9.58 | 11.07 | 10.03 | 13.67 | 11.13 | 8.01 | 7.01 |
| CCR + | CLSP096 | 19.94 | 0.29 | 8.65 | 0.44 | 13.42 | 18.99 | 14.34 | 14.41 | 10.23 | 11.60 | 8.42 | 7.37 |
| CCR + | CLSP105 | 23.90 | 0.88 | 7.46 | 0.73 | 16.23 | 20.62 | 15.69 | 15.61 | 12.32 | 12.67 | 10.82 | 9.47 |
| CCR + | CLSP107 | 0.44 | 0.47 | 0.36 | 5.91 | 0.54 | 0.47 | 0.54 | 3.26 | 3.31 | 2.75 | 2.07 | 1.83 |
| CCR + | CLSP113 | 0.31 | 0.29 | 0.49 | 8.43 | 0.28 | 0.41 | 0.36 | 4.43 | 4.33 | 3.59 | 2.66 | 2.33 |
| CCR + | CLSP115 | 9.90 | 9.72 | 18.17 | 26.81 | 6.66 | 12.29 | 9.27 | 17.81 | 13.59 | 14.29 | 11.93 | 10.44 |
| CCR + | CLSP117 | 0.75 | 0.57 | 8.98 | 7.36 | 0.60 | 6.20 | 4.72 | 8.12 | 3.92 | 6.55 | 6.25 | 5.48 |
| CCR + | CLSP119 | 0.22 | 0.26 | 7.33 | 5.92 | 0.18 | 4.89 | 3.69 | 6.52 | 2.99 | 5.24 | 3.94 | 3.47 |
| CCR + | CLSP122 | 0.66 | 0.40 | 0.68 | 0.47 | 0.56 | 0.75 | 0.65 | 0.69 | 0.55 | 0.62 | 3.60 | 3.15 |
| CCR + | CLSP136 | 0.26 | 0.25 | 0.47 | 0.48 | 0.31 | 0.45 | 0.43 | 0.55 | 0.44 | 0.52 | 0.62 | 0.56 |
| CCR + | CLSP143 | 7.99 | 0.40 | 0.80 | 0.82 | 5.37 | 5.64 | 4.26 | 4.47 | 4.27 | 3.60 | 2.69 | 2.37 |
| CCR + | CLSP147 | 0.39 | 0.22 | 8.75 | 0.48 | 0.27 | 5.96 | 4.48 | 4.60 | 0.34 | 3.69 | 4.34 | 3.81 |
| CCR + | CLSP154 | 9.94 | 0.43 | 6.34 | 0.49 | 6.76 | 10.70 | 8.13 | 8.16 | 5.20 | 6.60 | 4.93 | 4.31 |
| CCR + | GHBD020 | 0.32 | 0.38 | 5.61 | 0.76 | 0.29 | 3.78 | 2.89 | 3.08 | 0.46 | 2.51 | 1.93 | 1.72 |
| CCR + | GHBD025 | 0.27 | 0.25 | 6.97 | 0.46 | 0.21 | 4.69 | 3.55 | 3.65 | 0.29 | 2.94 | 2.29 | 2.00 |
| CCR + | GHBD029 | 5.94 | 6.09 | 6.28 | 6.27 | 4.08 | 4.21 | 3.25 | 3.34 | 3.24 | 2.74 | 2.09 | 1.82 |
| CCR + | CBSE005 | 11.39 | 11.65 | 17.84 | 18.52 | 7.78 | 11.91 | 9.07 | 12.51 | 9.42 | 10.12 | 12.87 | 11.28 |
| CCR + | CBSE006 | 24.67 | 24.68 | 49.15 | 24.98 | 16.52 | 24.67 | 16.52 | 16.71 | 12.59 | 12.59 | 12.59 | 10.10 |
| CCR + | CBSE007 | 5.49 | 5.45 | 10.74 | 25.72 | 3.71 | 5.49 | 3.71 | 17.23 | 12.96 | 12.96 | 12.96 | 10.38 |
| CCR + | CBSE010 | 30.57 | 73.65 | 30.99 | 32.35 | 53.22 | 24.78 | 43.22 | 22.56 | 43.90 | 37.76 | 34.94 | 29.99 |
| CCR + | CBSE013 | 12.31 | 17.52 | 20.52 | 18.49 | 11.91 | 13.91 | 13.20 | 13.69 | 12.19 | 13.17 | 12.77 | 10.95 |
| CCR + | CBSE023 | 5.78 | 5.68 | 5.84 | 5.83 | 4.02 | 4.13 | 3.22 | 3.30 | 3.22 | 2.74 | 2.42 | 2.11 |
| CCR + | CLSP044 | 103.9 | 0.25 | 0.53 | 0.29 | 69.37 | 69.55 | 52.23 | 52.25 | 52.11 | 41.85 | 30.04 | 26.32 |
| CCR + | CLSP050 | 5.97 | 5.86 | 13.93 | 11.14 | 4.03 | 9.40 | 7.09 | 9.73 | 5.70 | 7.81 | 7.77 | 6.84 |
| CCR + | CLSP072 | 11.33 | 14.86 | 14.32 | 11.06 | 13.61 | 13.25 | 14.48 | 12.58 | 12.85 | 13.70 | 9.96 | 8.73 |
| CCR + | CLSP073 | 0.67 | 14.22 | 7.20 | 6.00 | 9.65 | 4.96 | 10.62 | 6.51 | 10.02 | 10.73 | 7.76 | 6.81 |
| CCR + | CLSP074 | 0.29 | 0.41 | 6.82 | 0.66 | 0.36 | 4.63 | 3.59 | 3.71 | 0.51 | 3.07 | 2.31 | 2.07 |
| CCR + | CLSP089 | 25.65 | 0.62 | 9.83 | 0.41 | 17.37 | 23.51 | 17.84 | 17.73 | 13.13 | 14.35 | 10.39 | 9.11 |
| CCR + | CLSP091 | 5.55 | 0.27 | 0.39 | 6.43 | 3.83 | 3.91 | 3.03 | 6.11 | 6.05 | 4.96 | 3.71 | 3.26 |
| CCR + | CLSP094 | 6.67 | 0.36 | 0.25 | 0.31 | 4.61 | 6.67 | 4.61 | 4.57 | 3.54 | 3.54 | 2.89 | 2.44 |
| CCR + | CLSP097 | 0.31 | 0.30 | 0.64 | 0.55 | 0.27 | 0.50 | 0.42 | 0.55 | 0.38 | 0.48 | 0.46 | 0.40 |
| CCR + | CLSP106 | 5.05 | 5.13 | 12.36 | 11.50 | 3.42 | 8.24 | 6.22 | 9.40 | 5.78 | 7.55 | 5.53 | 4.84 |
| CCR + | CLSP121 | 0.22 | 0.23 | 0.51 | 0.41 | 0.21 | 0.40 | 0.35 | 0.44 | 0.30 | 0.39 | 1.93 | 1.69 |
| CCR + | CLSP123 | 0.63 | 0.62 | 9.03 | 14.97 | 0.51 | 6.12 | 4.66 | 11.83 | 7.63 | 9.52 | 11.26 | 9.85 |
| CCR + | CLSP138 | 9.60 | 0.30 | 8.82 | 0.56 | 6.42 | 12.10 | 9.09 | 9.22 | 4.96 | 7.39 | 5.43 | 4.76 |
| CCR + | CLSP141 | 0.43 | 0.38 | 0.63 | 10.19 | 0.32 | 0.49 | 0.40 | 5.30 | 5.18 | 4.27 | 3.31 | 2.90 |
| CCR + | CLSP153 | 33.40 | 23.59 | 21.96 | 29.87 | 28.97 | 27.88 | 25.93 | 29.08 | 29.89 | 27.28 | 19.56 | 17.12 |
| CCR + | GHBD019 | 7.06 | 0.24 | 0.68 | 0.60 | 4.71 | 5.00 | 3.75 | 3.93 | 3.71 | 3.14 | 2.34 | 2.08 |
| CCR + | CBSE012 | 5.81 | 12.11 | 0.47 | 0.30 | 11.85 | 4.10 | 9.06 | 3.16 | 8.98 | 7.32 | 6.19 | 5.30 |
| CCR + | CBSE019 | 6.40 | 0.63 | 0.88 | 7.05 | 4.42 | 4.58 | 3.55 | 6.76 | 6.64 | 5.50 | 4.71 | 4.04 |
| CCR + | CBSE026 | 195.7 | 119.8 | 8.71 | 6.43 | 210.2 | 136.2 | 161.9 | 105.3 | 160.8 | 132.1 | 95.87 | 83.92 |
| CCR + | CBSE027 | 175.0 | 0.55 | 5.36 | 5.79 | 175.0 | 120.0 | 120.0 | 92.78 | 120.3 | 92.78 | 68.71 | 61.25 |
| CCR + | CLSP042 | 153.8 | 0.76 | 5.43 | 8.15 | 102.8 | 105.9 | 79.62 | 83.31 | 80.98 | 66.81 | 50.00 | 43.77 |
| CCR + | CLSP057 | 15.32 | 0.19 | 0.40 | 10.84 | 10.30 | 10.44 | 7.89 | 13.21 | 13.11 | 10.62 | 12.94 | 12.80 |
| CCR + | CLSP068 | 337.0 | 120.0 | 5.60 | 7.64 | 304.4 | 228.2 | 231.0 | 174.8 | 232.0 | 187.7 | 138.3 | 121.2 |
| CCR + | CLSP079 | 0.43 | 25.92 | 0.59 | 0.60 | 25.92 | 0.59 | 17.53 | 0.65 | 17.54 | 13.34 | 9.16 | 7.91 |
| CCR + | CLSP083 | 0.37 | 0.46 | 0.46 | 0.54 | 0.41 | 0.42 | 0.43 | 0.48 | 0.47 | 0.48 | 0.44 | 0.39 |
| CCR + | CLSP095 | 150.8 | 0.59 | 0.59 | 5.56 | 100.8 | 100.8 | 75.81 | 78.29 | 78.29 | 62.80 | 46.47 | 40.69 |
| CCR + | CLSP109 | 0.47 | 9.79 | 0.63 | 34.00 | 9.79 | 0.63 | 6.79 | 22.93 | 29.04 | 21.97 | 17.37 | 14.96 |
| CCR + | CLSP132 | 7.74 | 7.84 | 15.99 | 17.61 | 5.42 | 10.86 | 8.34 | 13.23 | 9.15 | 10.74 | 9.52 | 8.36 |
| CCR + | CLSP156 | 45.79 | 24.26 | 8.49 | 0.73 | 46.47 | 35.97 | 38.94 | 27.17 | 35.05 | 31.31 | 28.46 | 24.92 |
| CCR + | CLSP159 | 67.36 | 33.80 | 6.71 | 0.66 | 67.28 | 49.22 | 53.70 | 37.13 | 50.67 | 43.13 | 32.70 | 28.61 |
| CCR + | CLSP160 | 7.86 | 7.49 | 7.73 | 42.10 | 5.32 | 5.48 | 4.17 | 21.48 | 21.36 | 17.23 | 12.46 | 10.90 |
| CCR + | CLSP161 | 18.00 | 0.52 | 8.72 | 0.61 | 12.23 | 17.70 | 13.45 | 13.49 | 9.40 | 10.94 | 7.99 | 6.99 |
| Seuil | | 0.96 | 0.96 | 0.97 | 0.95 | 0.96 | 0.96 | 0.96 | 0.77 | 0.77 | 0.77 | 0.77 | 0.77 |
| Spécificité (%) | | 100 | 100 | 100 | 100 | 100 | 100 | 100 | 100 | 100 | 100 | 100 | 100 |
| Sensibilité (%) | | 52.56 | 35.90 | 62.82 | 89.13 | 58.97 | 76.92 | 80.77 | 88.46 | 75.64 | 91.03 | 94.87 | 96.15 |
| CCR + supérieur au seuil | | 41 | 28 | 49 | 41 | 46 | 60 | 63 | 69 | 59 | 71 | 74 | 75 |

| | | | | | | | | | | | | | |
|---|---|---|---|---|---|---|---|---|---|---|---|---|---|
| Score 2^{a}: association ACE, ProDéfensine-A6 Score 2^{b}: association CA19-9, ProDéfensine-A6 Score 2^{c}: association Béta-2 Microglobuline, ProDéfensine-A6 Score 2^{d}: association L-FABP,-ProDéfensine A6 Score 3^{e}: association ACE, CA19-9, ProDéfensine-A6 Score 3^{f}: association ACE, Béta-2 Microglobuline, ProDéfensine-A6 Score 4^{g}: association ACE, Béta-2 Microglobuline, CA19-9, ProDéfensine-A6 Score 4^{h}: association ACE, Béta-2 Microglobuline, L-FABP, ProDéfensine-A6 Score 4ⁱ: association ACE, CA19-9, L-FABP, ProDéfensine-A6 Score 5^{j}: association ACE, Béta-2 Microglobuline, CA19-9, L-FABP, ProDéfensine-A6 Score 7^{k}: association ACE, Béta-2 Microglobuline, CA19-9, Galectine 3, ProDéfensine-A6, L-FABP, MIF Score 8^{l}: association ACE, Béta-2 Microglobuline, CA19-9, ProDéfensine-A6, Galectine-3, L-FABP, MIF, Plastine I CCR+ = patients atteints d'un cancer colorectal (Adénocarcinome), CCR- = sujets sains. | | | | | | | | | | | | | |

### Exemple 5 : Détection des marqueurs tumoraux par la technique LC-MRM-MS

### 1. Méthodologie

Afin de pouvoir baisser la limite de détection à quelques ng/ml, un procédé amélioré de MRM-MS a été mis en oeuvre. Les étapes successives de ce procédé sont : 1) immunodéplétion des protéines abondantes, 2) digestion trypsique, 3) fractionnement SPE (solid-phase extraction) des peptides, 4) chromatographie liquide (LC) couplée à la MRM-MS.

La mise au point a été réalisée sur des échantillons surchargés (spike) en ajoutant le peptide synthétique ProDéfensine-A6 (SEQ ID°1).

*Immunodéplétion.* La déplétion des protéines abondantes du sérum a été réalisée en utilisant le kit commercial Vivapure anti-HSA de Vivascience. Alternativement, le kit Proteoextract Albumin/IgG de Calbiochem et le Aurum™ serum Protein Minikit de Bio-Rad ont aussi été utilisés. Il est aussi possible de produire les résines spécifiques au laboratoire, en couplant un anticorps monoclonal dirigé contre la protéine à dépléter sur une résine Sepharose 4B activée au CNBr (Amersham Bioscience), en suivant les instructions du fabricant.

*Digestion enzymatique.* Les échantillons de sérums déplétés sont dénaturés dans une solution d'urée 6M tamponnée par 10 mM de Tris pH 8 et contenant 30 mM de dithiothreitol, pendant 40 minutes à 40°C, puis alkylés par de l'iodoacétamide 50 mM, à température ambiante, pendant 40 minutes, à l'obscurité. Ils sont dilués 6 fois dans l'eau, puis la digestion trypsique est réalisée à 37°C, sur la nuit, en utilisant un ratio enzyme substrat de 1:30 (Promega). La digestion est arrêtée par ajout d'acide formique à une concentration finale de 0,5%. Les échantillons digérés sont dessalés par extraction sur phase solide (SPE, solid phase extraction) en utilisant les cartouches en phase inverse Oasis HLB 3cc (60 mg) (Waters). Après application de l'échantillon, les cartouches sont lavées par 1 ml d'acide formique à 0,1%, puis l'élution a été réalisée par un mélange méthanol/eau (80/20 v/v) contenant 0,1% d'acide formique. Les éluats sont séchés sous vide.

*Fractionnement SPE.* Les échantillons secs sont repris dans 1 ml de tampon acétate et chargés sur les cartouches mixtes (hydrophobe et échange de cation) Oasis MCX (mixed cation exchange) 60 mg (Waters) équilibrées au préalable en tampon acétate et méthanol. Les cartouches sont lavées par 1 ml de tampon acétate et 1 ml de méthanol. Les peptides d'intérêt (Tableau 6) sont élués par 1 ml d'un mélange méthanol/tampon acétate (50/50 v/v)). Le pH du tampon acétate est choisi en fonction du point isoélectrique du peptide d'intérêt. Les éluats sont séchés sous vide, dissous dans 200 µl d'une solution d'acétonitrile/eau (3/97 v/v) contenant 0,1% d'acide formique. Un aliquot de 50 µl a été injecté dans la LC couplée à un système MS-MS.

*Chromatographie liquide et spectrométrie de masse.* L'analyse LC-MS a été effectuée sur un système chromatographique haute pression (HPLC) de type HP 1100 series avec pompe binaire et injecteur (Agilent Technologies) couplé à un spectromètre de masse, soit un Sciex API 2000 triple quadripôle, soit un Sciex API 4000 Qtrap (MS hybride triple quadripôle - trappe ionique) (MDS Sciex) pour une meilleure sensibilité. La séparation LC a été effectuée sur une colonne C₁₈ Symmetry (Waters), à un débit d'élution de 300 µl/min. (Eluent A = 0,1% acide formique dans l'eau, éluent B = 0,1% acide formique dans l'acétonitrile, gradient linéaire de 5%B à 50%B en 25 min, puis de 50%B à 100%B en 3 min). L'analyse MS est réalisée en mode d'ionisation positive à une tension de 5500 V appliquée à l'aiguille permettant l'ionisation dans la source. Le contrôle de l'instrument et l'acquisition des données sont réalisés avec le logiciel Analyst 1.4.1. Les débits du gaz de nébulisation (air) et du gaz rideau (azote) sont de 30 et 20 psi, respectivement. La source ionique Turbo V™ est réglée à 400°C, le flux d'azote auxiliaire à 40 psi. Les transitions MRM enregistrées pour chaque peptide sont récapitulées dans le Tableau 6. L'énergie de collision (CE), la tension d'orifice (DP,declustering potential) et la tension à la sortie de la cellule de collision (CXP, collision cell exit potential) sont optimisées pour chacune des transitions MRM sélectionnées.

### 2. Résultats

La liste des transitions MRM théoriques de la séquence SEQ ID°1 a été générée en utilisant le logiciel MIDAS (MRM-initiated Detection and Sequencing). Cette liste comprend tous les ions parents di- ou tri- chargés des peptides tryptiques théoriques dans un intervalle de masse allant de 800 à 3000 Da et tous les ions fragments possibles de type y ou b. Pour chaque protéine, chaque transition possible a été testée afin de déterminer les transitions les plus sensibles et les plus spécifiques. Le résultat de cette sélection est récapitulé dans le Tableau 6. Un exemple d'optimisation de l'étape SPE pour la transition 727/556 du peptide EPLQAEDDPLQAK (SEQ ID N°30) est présenté sur la Figure 7. La séparation chromatographique MCX a été réalisée à différents pH, le pH choisi pour la suite des expériences étant le pH qui permet d'obtenir l'aire du pic la plus élevée.

De plus, en utilisant un peptide lourd de type AQUA (Sigma) ou encore une protéine recombinante lourde qui serviront d'étalon de dosage, il est possible de quantifier de manière absolue le marqueur tumoral d'intérêt dans un milieu biologique complexe.

**Tableau 6**

| *ProDéfensine-A6* | | | | | | |
|---|---|---|---|---|---|---|
| Séquence (SEQ ID N°) | pI | Q1 | Q3 | DP | CE | CXP |
| EPLQAEDDPLQAK (SEQ ID N°30) | 3,57 | 727,4 | 556,4 | 50 | 40 | 28 |
| | | | 218,2 | 50 | 35 | 8 |
| | | | 915,4 | 50 | 30 | 25 |
| | | | 986,4 | 50 | 35 | 27 |
| AYEADAQEQR (SEQ ID N°31) | 3,93 | 590,8 | 746,4 | 100 | 30 | 11 |
| | | | 817,4 | 100 | 30 | 18 |
| | | | 631,3 | 100 | 30 | 11 |
| | | | 560,3 | 100 | 30 | 11 |
| | | | 946,4 | 100 | 35 | 20 |

### Références bibliographiques

1 : J.D. Potter, 1999, J Natl Cancer Inst, 91, 916-32
2 : J. Faivre, 2001, Epidémiologie et dépistage du cancer colorectal, Ed. Springer
3 : D.E. Jones et C.L. Bevins, 1992, J Biol Chem, 23216-23225
4 : C.L. Bevins et al., 1996, Genomics, 95-106
5 : I. Lawrance et al., 2001, Hum Mol Gen, 445-456
6 : M.J. Nam et al., 2005, J Biol Chem, 8260-8265
7 : E. Remold-O'Donnell et al., 1992, Proc Natl Acad Sci USA, 89, 563-5639
8 : J. Cooley et al., 2001, Biochemistry, 15762-15770
9 : M. Algrain et al., 1993, J Cell Biol, 120, 129-139
10 : W.G. Jiang et S. Hiscox, 1996, Anticancer Res, 16, 861-865
11 : S. Hiscox et W.G. Jiang, 1999, J Cell Sci, 112, 3081-3090
12 : T. Xiao et al, 2005, Mol Cell Proteomics, 4, 1480-1486
13 : M. Anders et W. Dekant, 1994, Advances in Pharmacology, 431-448
14 : K. Lorentz et al., 1975, Clinica Chimica Acta, 263-269
15 : K. Lorentz et B. Flatter, 1975, Clinica Chimica Acta, 271-274
16 : R.M. Cook et al., 1993, J Biol Chem, 17010-17017
17 : Y.E. Miller et al., 1989, J Clin Invest, 2120-2124
18 : S. Balabanov et al., 2001, Eur J Biochem, 5977-5980
19 : E. Chan et al., 1985, J Biol Chem, 260, 2629-2632
20 : R. Das et al., 2001, Clin Cancer Res, 7, 1706-1715
21 : J. Stulik et al., 2001, Electrophoresis, 22, 3019-3025
22 : T. Yamazaki et al., 1999, J Surg Oncol, 72, 83-87
23 : D.A. Sweetser et al., 1987, J Biol Chem, 266, 16060-16071
24 : M. Pelsers et al., 2003, Clin Biochem, 36, 529-535
25 : R. Xiao, et al., 2005, Molecular Cancer, 4, 1-17
26 : E.E. Niederkofler et al., 2003, J Lipid Res, 44, 630-639
27 : G.L. Hortin, 2006, Clinical Chemistry, 52(7), 1223-1237
28 : J.Y. Engwegen et al., 2006, World J Gastroenterol, 12(10), 1536-1544
29 : Z. Zhang et al., 2004, Cancer Research, 64, 5882-5890
30 : H. Hachem et al., 1986, J Chem Clin Biochem, 24, 161-166
31 : C.S. Lin, et al., 1993, J Biol Chem, 268, 2781-92
32 : V. Delanote et al., 2005, Acta Pharama Sinica, 769-779
33 : A.P. Arrigo et al., 1988, Nature, 331, 192-194
34 : T. Lavabre-Bertrand et al., 2001, Cancer, 92, 2493-2500
35 : S. Nakahara et al., 2005, Apoptosis, 10, 267-275
36 : I. Iurisci et al., 2000, Clin Can Res, 6, 1389-1393
37 : M.K. Schwartz, 2006, Clin Chim Acta, 1992, 77-82
38 : D.J. McCool et al., 1999, Biochem J, 593-600
39 : J.L. Iovanna et al., 1994, Gastroenterology, 106, 728-734
40 : Y. Motoo et al., 1999, Dig Dis Sci, 44, 1142-1147
41 : M. Herlyn et al., 1979, Proc Natl Acad Sci USA, 76, 1438-1442
42 : A. Armstrong et S. Eck, 2003, Cancer Biol Ther, 2, 320-325
43 : D. Herlyn et al., 1982, Proc Natl Acad Sci USA, 79, 4761-4765
44 : H. Abe et al., 2002, J Immunol Methods, 270, 227-233
45 : V. Barak et al., 2004, Clin Biochem, 37, 529-540
46 : H. Kim et al., 2006, Ann Clin Lab Sci, 36, 294-298
47 : F. Roca et al., 2006, J Surg Oncol, 151-160
48 : C.H. Damsky et al., 1983, Cell, 455-466
49 : M. Katayama et al., 1994, Br J Cancer, 580-585
50 : C. Willmanns et al., 2004, Clin Exp Metastasis, 75-78
51 : P. Gold et S. Freedman, 1965, J Exp Med, 467-481
52 : M. Duffy, 2001, Clin Chem, 624-630
53 : Y. Kim et al., 2003, Ann Clin Lab Sci, 32-38
54 : J.L. Magnani et al., 1983, Cancer Research, 43, 5489-5492
55 : J. Holmgren et al., 1984, Br Med J (Clin. Re. Ed.), 288, 1479-1482
56 : T.L. Klug et al., 1986, Int J Cancer, 38, 6661-669
57 : P. Kuusela et al., 1991, Br J Cancer, 63, 636-640
58 : M. Holland et al., 1993, Medicina (B. Aires), 53(2), 117-23
59 : F. Model et al., juillet 2006, World Congress on Gastrointestinal Cancer, « Détection of Methylated DNA in Plasma from Colorectal Cancer Patients and Controls by Real-Time PCR Analysis of Septin 9 »
60 : M.P. Ebert et al., 2006, Gastroentrology, 131(5), 1418-1430
61 : C. Bianco et al., 2006, Clin Cancer Res, 12, 5158-5164
62 : Wilson et al., 2005, Gastroenterology, 129 : 1485-1503
63 : Lee et al., 2008, Am. J. Clin. Pathol., 129 : 772-779
64 : P.D. Hardt et al., 2004, Br J Cancer, 91(5): 980-984
65 : E. Sagiv et al., 2008, Cancer Res, 68(8): 2803-2812
66 : E.S Leman et al., 2007, Cancer Res, 67(12): 5600-5605
67 : M. Jambon et al., 2005, Structural bioinformatics, 21(20) : 3929-3930
68 : RA. Bauer et al., 2008, Nucleic Acids Research, 36: 47-54
69 : R. Yasumatsu et al., 2006, Am J Physiol Lung Cell Mol Physiol 291, L619-L627
70 : J. Chevalier et al., 1997, J Histochem Cytochem, 45, 481-491
71 : S. Patterson, 2000, Physiological Genomics 2, 59-65
72 : L.J. Kricka et al., 1999, Clinical Chemistry, 45(4), 453-458
73 : S. Tyagi et F.R. Kramer, 1996, Nature Biotech, 14, 303-308
74 : T. F. Imperiale et al., 2004, N Engl J Med, 351(26), 2704-2714
75 : D.A. Ahlquist et al., 2000, Gastroenterology, 119, 1219-1227
76 : I. H. Wong, 2006, Methods Mol Biol, 336, 33-46
77 : M. P. Ebert et al., 2005, Neoplasia, 7(8), 771-778
78 : C. Lofton-Day et al., 2007, AACR Annual Meeting 2007, Los Angeles, U.S.A., Poster no LB-165, Clinical case-control study in plasma shows that the DNA methylation biomarker, Septin-9, detects 70% of stage I-III colorectal cancer patients
79 : P. Métézeau et al., La cytométrie en flux pour l'étude de la cellule normale ou pathologique (Tome I), Eds Medsi-MacGrawhill
80 : J. Mathieu et al, 2006, Fonctions cellulaires et métabolisme. In: (coordonnateurs : Ronot X.et al.). La cytométrie en flux. Tec & Doc, 255-298. ISBN 978-2-7430-0898-7
81 : G. Köhler et C. Milstein, 1975, Nature, 256, 495-497
82 : G. Köhler et C. Milstein, 1976, Eur J Immunol, 6, 511-519

## Revendications

1. Procédé de diagnostic *in vitro* du cancer colorectal par détermination de l'augmentation de la concentration, par rapport aux valeurs de référence déterminées pour les sujets sains, du marqueur protéique ProDéfensine-A6 dans un échantillon biologique issu d'un patient suspecté d'être atteint du cancer colorectal, mettant en oeuvre un anticorps monoclonal spécifique de la ProDéfensine-A6 reconnaissant l'épitope 1 de SEQ ID N°6.

2. Procédé de diagnostic selon la revendication 1, dans lequel ledit anticorps est utilisé pour la capture de la ProDéfensine-A6.

3. Procédé selon l'une quelconque des revendications 1 à 2, **caractérisé en ce que** l'échantillon biologique est un échantillon distant de la tumeur.

4. Procédé selon l'une quelconque des revendications 1 à 3, **caractérisé en ce qu'**il consiste également à déterminer la présence dans un ou plusieurs échantillons biologiques issus d'un patient suspecté d'être atteint du cancer coloréctal, d'au moins un autre marqueur tumoral choisi parmi les marqueurs suivants : Leucocyte Elastase Inhibitor, Ezrine, Aminoacylase 1, Liver Fatty Acid-Binding Protein, Intestinal Fatty Acid-Binding Protein, Apolipoprotéine AI, Apolipoprotéine AII, Plastine-I, Beta 2 Microglobuline, Protéasome 20S, Galectine-3, L-Lactate Deshydrogénase Chaîne B, Calréticuline, Regenerating Islet-Derived Protein 3 Alpha, Tumor-Associated Calcium Signal Transducer 1, Keratine type II Cytoskeletal 8, Keratine type I Cytoskeletal 18, Keratine type I Cytoskeletal 19, Epithelial-Cadhérine, ACE, Villine, CA19-9, CA 242, CA 50, CA 72-2, Testostérone, TIMP-1, Cripto-1, Protéine Disulfide Isomérase, Intélectine-1, Cytokératine 20, Translationally-Controlled Tumor Protein, (Pro)défensine-A5, MIF, Pyruvate kinase M2-PK, Calgranuline C, CD24, CCSA-3 (colon cancer specific antigen) et CCSA-4, la détection d'ADN méthylé dans le sang, de préférence l'ADN méthylé du gène AXL4 ou l'ADN méthylé du gène Septin-9, la détection d'altérations spécifiques de fragments d'ADN dans les selles comme des mutations spécifiques de l'ADN dans les selles ou des altérations spécifiques du profil de méthylation de l'ADN dans les selles, la détection d'hémoglobine humaine dans les selles.

5. Procédé selon la revendication 4, **caractérisé en ce que** ledit au moins un autre marqueur est ou sont :
- L-FABP,
- CA19-9 et ACE,
- Béta-2 Microglobuline et ACE
- Béta-2 Microglobuline, CA19-9 et ACE,
- Béta-2 Microglobuline, L-FABP et ACE,
- L-FABP, CA19-9 et ACE,
- Béta-2 Microglobuline, CA19-9 et ACE,
- Béta-2 Microglobuline, CA19-9, L-FABP et ACE,
- Béta-2 Microglobuline, CA19-9, Galectine-3, L-FABP, MIF et ACE, ou
- Béta-2 Microglobuline, CA19-9, Galectine-3, L-FABP, MIF, Plastine I et ACE.

6. Utilisation du procédé selon l'une quelconque des revendications 1 à 5 dans le diagnostic précoce, le dépistage, le suivi thérapeutique, le pronostic et le diagnostic des rechutes dans le cadre du cancer colorectal.

7. Anticorps monoclonal anti-ProDéfensine-A6 reconnaissant spécifiquement l'épitope 1 de séquence SEQ ID N°6.

## Patentansprüche

1. In-vitro-Diagnoseverfahren für Kolorektalkrebs durch Bestimmung einer Erhöhung der Konzentration des Proteinmarkers ProDefensin-A6 in einer biologischen Probe von einem Patienten mit Verdacht auf Kolorektalkrebs im Vergleich zu für den gesunde Individuen bestimmten Referenzwerten, unter Verwendung eines für ProDefensin-A6 spezifischen monoklonalen Antikörpers, der Epitop 1 von SEQ ID NR. 6 erkennt.

2. Diagnoseverfahren nach Anspruch 1, wobei der Antikörper zum Einfangen von ProDefensin-A6 eingesetzt wird.

3. Verfahren nach einem der Ansprüche 1 bis 2, **dadurch gekennzeichnet, dass** es sich bei der biologischen Probe um eine vom Tumor beabstandete Probe handelt.

4. Verfahren nach einem der Ansprüche 1 bis 3, **dadurch gekennzeichnet, dass** es darin besteht, dass man auch das Vorhandensein von mindestens einem anderen Tumormarker, ausgewählt aus den folgenden Markern, in einer oder mehreren biologischen Proben von einem Patienten mit Verdacht auf Kolorektalkrebs bestimmt: Leukozytenelastaseinhibitor, Ezrin, Aminoacylase 1, Liver Fatty Acid-Binding Protein, Intestinal Fatty Acid-Binding Protein, Apolipoprotein AI, Apolipoprotein AII, Plastin-I, Beta-2-Mikroglobulin, Proteasom 20S, Galectin-3, L-Lactatdehydrogenase-Kette B, Calreticulin, Regenerating Islet-Derived Protein 3 Alpha, Tumor-Associated Calcium Signal Transducer 1, Keratine type II-Cytoskeletal 8, Keratine type I Cytoskeletal 18, Keratin type I Cytoskeletal 19, Epithelial-Cadherin, ACE, Villin, CA19-9, CA 242, CA 50, CA 72-2, Testosteron, TIMP-1, Cripto-1, Proteindisulfidisomerase, Intelectin-1, Cytokeratin 20, Translationally-Controlled Tumor Protein, (Pro)defensin-A5, MIF, Pyruvatkinase M2-PK, Calgranulin C, CD24, CCSA-3 (kolonkrebsspezifisches Antigen) und CCSA-4, der Nachweis von methylierter DNA im Blut, vorzugsweise von methylierter DNA des Gens AXL4 oder von methylierter DNA des Gens Septin-9, der Nachweis von spezifischen Veränderungen von DNA-Fragmenten im Stuhl wie von spezifischen Mutationen der DNA im Stuhl oder von spezifischen Veränderungen des Methylierungsprofils der DNA im Stuhl, der Nachweis von menschlichem Hämoglobin im Stuhl.

5. Verfahren nach Anspruch 4, **dadurch gekennzeichnet, dass** der mindestens eine andere Marker Folgendes ist oder sind:
- L-FABP,
- CA19-9 und ACE,
- Beta-2 Mikroglobulin und ACE,
- Beta-2 Mikroglobulin, CA19-9 und ACE,
- Beta-2 Mikroglobulin, L-FABP und ACE,
- L-FABP, CA19-9 und ACE,
- Beta-2 Mikroglobulin, CA19-9 und ACE,
- Beta-2 Mikroglobulin, CA19-9, L-FABP und ACE,
- Beta-2 Mikroglobulin, CA19-9, Galectin-3-, L-FABP, MIF und ACE oder
- Beta-2 Mikroglobulin, CA19-9, Galectin-3, L-FABP, MIF, Plastin I and ACE.

6. Verwendung des Verfahrens nach einem der Ansprüche 1 bis 5 in der Frühdiagnose, im Screening, in der therapeutischen Begleitung, in der Prognose und in der Diagnose von Rezidiven im Rahmen von Kolorektalkrebs.

7. Monoklonaler Antikörper anti-ProDefensin-A6, der spezifisch Epitop I der Sequenz SEQ ID NR. 6 erkennt.

## Claims

1. Method for the *in vitro* diagnosis of colorectal cancer by determining the increase of the concentration, relative to the reference values determined for healthy patients, of protein Prodefensin-A6 marker in a biological sample taken from a patient suspected of having colorectal cancer, using a monoclonal antibody specific for the Prodefensin-A6 recognizing the epitope 1 of SEQ ID N°6.

2. Method according to Claim 1, in which the said antibody is used for the capture of Prodefensin-A6.

3. Method according to any one of Claims 1 to 2, **characterized in that** the biological sample is a sample remote from the tumour.

4. Method according to any one of Claims 1 to 3, **characterized in that** it also consists in determining the presence, in one or more biological samples taken from a patient suspected of having colorectal cancer, of at least one other tumour marker chosen from the following markers: Leukocyte Elastase Inhibitor, Ezrin, Aminoacylase 1, Liver Fatty Acid-Binding Protein, Intestinal Fatty Acid-Binding Protein, Apolipoprotein AI, Apolipoprotein AII, I-Plastin, Beta2-Microglobulin, Proteasome 20S, Galectin-3, L-Lactate Dehydrogenase Chain B, Calreticulin, Regenerating Islet-Derived Protein 3 Alpha, Tumour-Associated Calcium Signal Transducer 1, Keratin Type II Cytoskeletal 8, Keratin Type I Cytoskeletal 18, Keratin Type I Cytoskeletal 19, Epithelial Cadherin, CEA, Villin, CA19-9, CA 242, CA 50, CA 72-2, Testosterone, TIMP-1, Cripto-1, Protein Disulphide Isomerase, Intelectin-1, Cytokeratin 20, Translationally-Controlled Tumour Protein, (Pro)Defensin-A5, MIF, Pyruvate Kinase M2-PK, Calgranulin C, CD24, CCSA-3 (colon cancer specific antigen) and CCSA-4, the detection of methylated DNA in the blood, preferably methylated DNA of the AXL4 gene or methylated DNA of the Septin-9 gene, the detection of specific alterations in faecal DNA fragments, such as specific mutations of faecal DNA or specific alterations of the methylation profile of faecal DNA, and the detection of faecal human haemoglobin.

5. Method according to Claim 4, **characterized in that** it comprises determining the presence of Prodefensin-A6 and of the following markers:
- L-FABP,
- CA19-9 and CEA,
- Beta2-Microglobulin and CEA,
- Beta2-Microglobulin, CA19-9 and CEA,
- Beta2-Microglobulin, L-FABP and CEA,
- L-FABP, CA19-9 and CEA,
- Beta2-Microglobulin, CA19-9 and CEA,
- Beta2-Microglobulin, CA19-9, L-FABP and CEA,
- Beta2-Microglobulin, CA19-9, Galectin-3, L-FABP, MIF And CEA, Or
- Beta2-Microglobulin, CA19-9, Galectin-3, L-FABP, MIF, I-Plastin And CEA.

6. Use of the method according to any one of Claims 1 to 5, in early diagnosis, screening, therapeutic follow-up, prognosis and relapse diagnosis in relation to colorectal cancer.

7. Monoclonal antibody anti-ProDefensin specifically recognizing the epitope 1 of SEQ ID N°6.
